# EUROPEAN PATENT SPECIFICATION

(11) **EP 3 615 026 B1**
(45) Date of publication and mention of the grant of the patent: **03.03.2021**
(21) Application number: 18721012.5
(22) Date of filing: 24.04.2018
(51) Int. Cl.: A61K 31/4184, A61K 31/519, A61K 31/517, A61K 31/55, A61K 31/166, A61K 31/473, A61P 17/02

(54) **A PARP INHIBITOR IN COMBINATION WITH A GLUCOCORTICOID AND/OR ASCORBIC ACID AND/OR A PROTEIN GROWTH FACTOR FOR THE TREATMENT OF IMPAIRED WOUND HEALING**
EIN PARP-HEMMER IN KOMBINATION MIT EINEM GLUCOCORTICOID UND/ODER ASCORBINSÄURE UND/ODER EINEM PROTEINWACHSTUMSFAKTOR ZUR BEHANDLUNG VON GESTÖRTER WUNDHEILUNG
UN INHIBITEUR DE PARP EN COMBINAISON AVEC UN GLUCOCORTICOÏDE ET/OU DE L'ACIDE ASCORBIQUE ET/OU UN FACTEUR DE CROISSANCE DE PROTÉINES POUR TRAITER DES TROUBLES DE LA CICATRISATION DE PLAIES

(30) Priority: 28.04.2017 EP 17000742
(43) Date of publication of application: 04.03.2020
(73) Proprietor: Akribes Biomedical GmbH, 1030 Wien (AT)
(72) Inventor: WOLFF-WINISKI, Barbara, 1230 Wien (AT); STÜTZ, Anton, 4813 Altmünster (AT); DÖRFLER, Petra, 2345 Brunn am Gebirge (AT)
(74) Representative: Pintsch, Tanja
(86) International application number: PCT/EP2018/060429
(87) International publication number: WO 2018/197461

(56) References cited:
- ZHOU XIN ET AL: "Poly-ADP-ribose polymerase inhibition enhances ischemic and diabetic wound healing by promoting angiogenesis", JOURNAL OF VASCULAR SURGERY, vol. 65, no. 4, 8 June 2016 (2016-06-08), pages 1161-1169, XP085097852, ISSN: 0741-5214, DOI: 10.1016/J.JVS.2016.03.407 cited in the application
- TAREK EL-HAMOLY ET AL: "3-aminobenzamide, a poly (ADP ribose) polymerase inhibitor, enhances wound healing in whole body gamma irradiated model : PARP inhibition & gamma irradiated wounds", WOUND REPAIR AND REGENERATION., vol. 23, no. 5, 2015, pages 672-684, XP055414446, ISSN: 1067-1927, DOI: 10.1111/wrr.12330 cited in the application
- HENGGE ET AL: "Adverse effects of topical glucocorticosteroids", JOURNAL OF THE AMERICAN ACADEMY OF DERMATOLOGY, vol. 54, no. 1, 2006, pages 1-15, XP005219461, ISSN: 0190-9622, DOI: 10.1016/J.JAAD.2005.01.010 cited in the application

## Description

The present invention relates to a PARP inhibitor in combination with a glucocorticoid and/or ascorbic acid and/or a protein growth factor for the treatment of impaired wound healing.

Chronic wounds are a major health issue worldwide with 5.7 million affected patients in the US alone and an expected increase due to the aging population and growing incidence of metabolic diseases.

Chronic wounds have a multifactorial etiology and are dependent on different variables: a) underlying disease, e.g. diabetes, arterial or venous insufficiency, b) pressure, c) age and nutritional status and d) microbial environment.

Chronic wounds are generally understood as those wounds that have not healed within 2 months. They are a major health issue worldwide. In developed countries, including the US and the EU, it has been estimated that 1 to 2% of the total population will experience a chronic wound during their lifetime [Gottrup F (2004) Am J Surg 187:38S-43S].

The major chronic wound indications are venous ulcers, pressure ulcers and diabetic foot ulcers. Venous ulcers are defects in pathologically altered tissue on the lower leg based on chronic venous insufficiency, often accompanied by deep venous thrombosis. Pressure ulcers are the results of severe tissue hypoxemia in immobilized patients. Diabetic foot ulceration can affect up to 25% of patients with diabetes throughout their lifetime and often results in lower limb amputation. The standard of care for all of these wounds, as recommended by the German Society for Dermatology [Dissemond J et al (2014) JDDG 1610-0379/2014/1207:541-554] includes wound dressings, surgical and biological (maggot) debridement, infection control and negative pressure therapy. Regranex® (PDGF: platelet-derived growth factor) was the only registered pharmacological treatment for a long time, but its therapeutic efficacy is minor, as is the success of cell-based therapies. Recombinant human EGF (rhEGF) is registered as Heberprot-P® in several countries for treating ulcerations in the diabetic foot ulcus syndrome. Moreover,

Trafermin (brand name: Fiblast®), also known as recombinant human basic fibroblast growth factor (rhbFGF), is a recombinant form of human basic fibroblast growth factor (bFGF) which is marketed in Japan as a topical spray for the treatment of skin ulcers.

Recurrence is a problem in one third of all chronic wounds, regardless of their treatment.

Even though they are anti-inflammatory in other settings, topical glucocorticoids cannot be used because one of their side effects is actually delayed wound healing [Hengge UR (2006) J Am Acad Dermatol 54:1-15]. Therefore, as a "dogma" in the prior art, topical glucocorticoids are described to impair wound healing. Further, non-steroidal anti-inflammatory drugs, e.g. ibuprofen, are only effective in ameliorating wound pain [Dissemond j et al (2014)].

There is therefore an ongoing and strong medical need for reliable and effective therapies for the treatment of impaired skin wound healing in patients.

It was surprisingly found in the present application, as shown in the examples and corresponding Figures, that the following combinations a) to d) exhibit an outstanding and synergistic fibroblast proliferation (2D) enhancing and fibroblast derived matrix formation (3D) enhancing effect, using wound exudates from chronic wound patients:
a) PARP inhibitors and glucocorticoids,
b) PARP inhibitors, glucocorticoids and Vitamin C,
c) PARP inhibitors and Vitamin C,
d) PARP inhibitors and protein growth factors.

The fibroblast proliferation assay (2D) and the fibroblast derived matrix formation assay (3D) are predictive assays for wound healing.

Therefore, the combinations of PARP inhibitors with Vitamin C and/or glucocorticoids and/or protein growth factors are surprisingly suitable for the treatment of chronic wounds. Particularly strong synergistic effects were observed in diabetes patients.

Moreover, PARP inhibitors are further surprisingly suitable for treating impaired skin wound healing in patients, who are already obtaining a therapy with a glucocorticoid and/or vitamin C and/or protein growth factors. In particular, patients receiving glucocorticoid therapy may be patients which suffer from co-morbidities, and/or may be patients to whom a graft was transplanted in the past. Such patients are treated with glucocorticoids in order to treat the co-morbidity and/or to achieve immunosuppression, such as to prevent GvHD or ameliorate an inflammatory disease. Patients receiving protein growth factors, in particular becaplermin (PDGF-BB) may receive such treatment as standard therapy of wound healing. Therefore, in one preferred embodiment, the subject is treated with at least protein growth factor for treating or preventing impaired wound healing.

Poly(ADP-ribose)polymerase (PARP) or poly(ADP-ribose)synthase (PARS) is a nuclear enzyme that has an essential role in recognizing DNA damage, facilitating DNA repair, controlling RNA transcription, mediating cell death, and regulating immune response. PARP activity is required for the repair of single-stranded DNA breaks through the base excision repair pathways. Cancer cells are often deficient in double-stranded DNA-repair capability, and are therefore more dependent on PARP directed single-stranded DNA-repair than are normal cells. Consequently, inhibition of PARP by specific PARP inhibitors has been described in the art to enhance the antitumor effects of DNA-damaging agents in cancer cells.

Further, there have been few reports on an enhancing effect of certain specific PARP inhibitors in very specific models in the broadest context of wound healing (Farkas B et al (2002) Reduction of acute photodamage in skin by topical application of a novel PARP inhibitor. Biochem Pharmacol 63:921-932; Zhou X et al (2016) Poly-ADP-ribose polymerase inhibition enhances ischemic and diabetic wound healing by promoting angiogenesis. J Vasc Surg, doi 10.1016/j.jvs.2016.03.407; Byun Y-S et al (2015) Poly(ADP-ribose) polymerase inhibition improves corneal epithelial innervation and wound healing in diabetic rats. Invest Ophthalmol Vis Sci 56:1948-1955; El-Hamoly T et al (2015) 3-aminobenzaminde, a poly (ADP ribose) polymerase inhibitor, enhances wound healing in whole body gamma irradiated model. Wound Rep Reg 23:672-684; El-Hamoly T et al (2014) Activation of poly (ADP ribose) polymerase-1 delays wound healing by regulating keratinocyte migration and production of inflammatory mediators. Mol Med 20:363-371; Sarras MP (2014) Inhibition of poly-APD ribose polymerase enzyme activitiy prevents hyperglycemia-induced impairment of angiogenesis during wound healing. Wound Rep Reg 22:666-670; Virag L & Szabo C (2002) The therapeutic potential of poly (ADP ribose) polymerase inhibitors. Pharmacol Rev 54:375-429; Akbar A et al (2017) The clinically used poly (ADP ribose) polymerase (PARP) inhibitor olaparib improves organ function, suppresses inflammatory responses and accelerates wound healing in a murine model of third-degree burn injury. Br J Pharmacol doi: 10.1111/bph.13735; Asmussen S et al (2011) The angiotensin-converting enzyme inhibitor captopril inhibits poly(ADP-ribose)polymerase activation and exerts beneficial effects in an ovine model of burn and smoke injury. Shock 3: 402-409; Thorsell AG (2016) Structural Basis for Potency and Promiscuity in Poly(ADP-ribose) Polymerase (PARP) and Tankyrase Inhibitors. J Med Chem 59:335-357; WO 01/42219 A2; Zhou X et al (2017) Poly-ADP-ribose polymerase inhibition enhances ischemic and diabetic wound healing by promoting angiogenesis. J Vasc Surg 65:1161-1169).

However, the predictability of these reports for clinical efficacy is very limited. Further, PARP inhibitors have not been investigated so far in clinical studies of wound healing or using patient tissues.

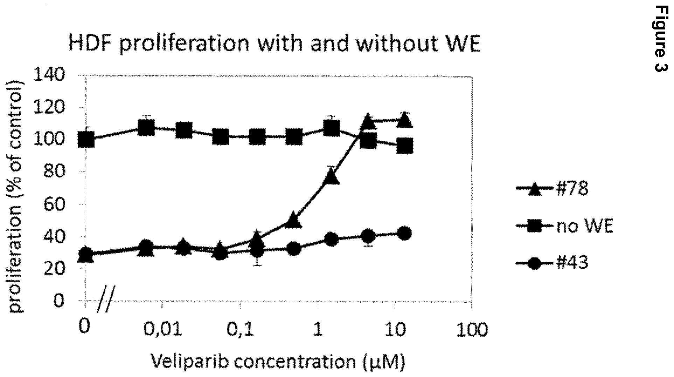

It was surprisingly found that the PARP inhibitor veliparib completely reversed inhibition of wound exudate (WE)-induced fibroblast proliferation for wound exudate from a diabetic patient in the proprietary and predictive assay system in Example 1 (Example 1.1, Figure 3). The effect of veliparib could be confirmed in a number of further wound exudate samples from other patients.

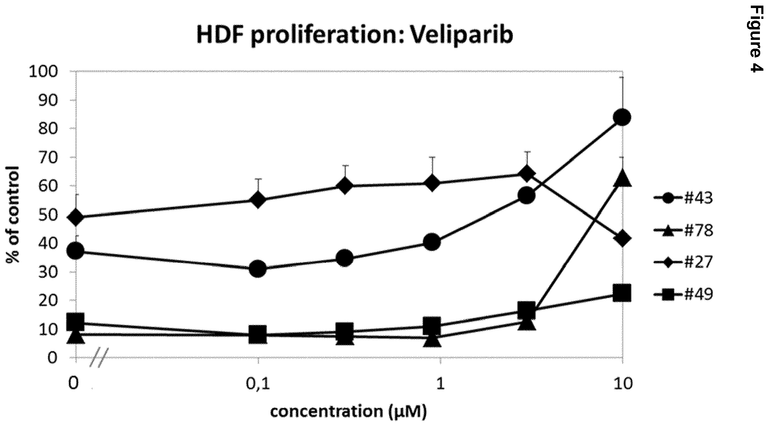

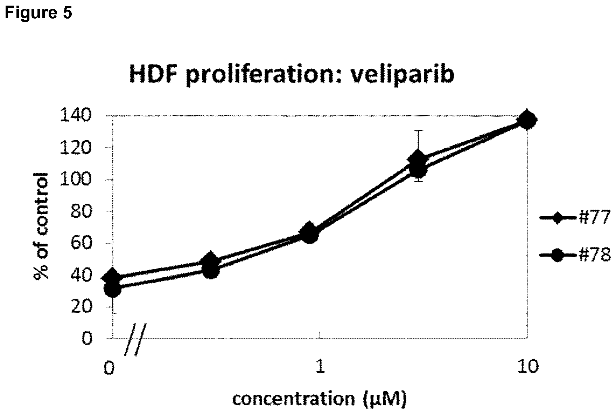

The effect of veliparib was further most prominent in patients with diabetes (Figure 4). Moreover, the effect of veliparib was reproducible in different samples of the same patient (Figure 5). Remarkably, said patient received a glucocorticoid, namely prednisolone, as co-medication.

Therefore, the experimental results suggest that a PARP inhibitor is in particular effective in the treatment of impaired wound healing for patients already obtaining glucocorticoids as therapy, such as a therapy of a co-morbidity.

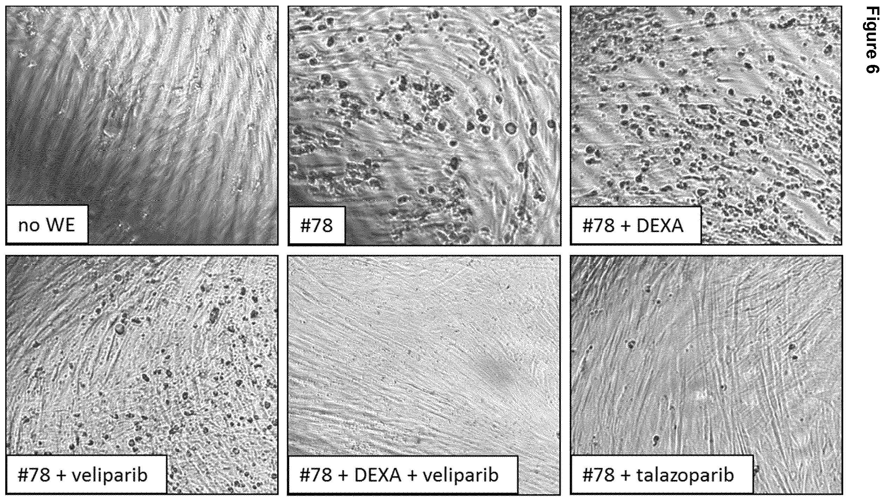

Further, the synergistic effect of a PARP inhibitor and a glucocorticoid could be further experimentally confirmed. As shown in Figure 6, PARP inhibitors talazoparib and veliparib both "cleaned up" WE-induced fibroblast matrix inhibition, and the combination of veliparib with dexamethasone was even superior to each substance alone, thereby showing a surprising synergistic effect.

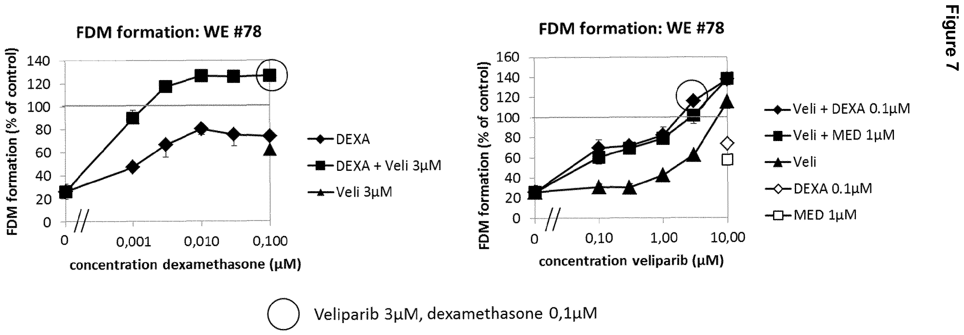

Moreover, the glucocorticoids dexamethasone and medroxyprogesterone were shown to enhance the effect of veliparib on rescuing fibroblast-derived matrix (FDM) formation after wound exudate treatment. Veliparib, in turn, enhances the effect of glucocorticoids in this patient (Figure 7). Said patient exhibited diabetes and immunosuppression after kidney transplantation as co-morbidities in addition to having a chronic wound (diabetic ulcer).

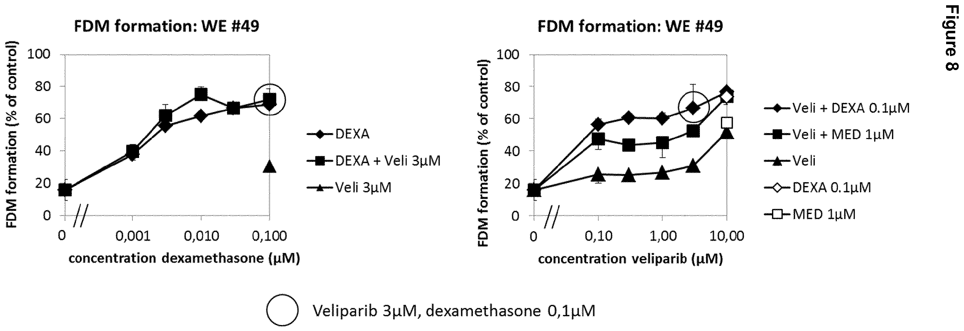

As shown in Figure 8, the effect of veliparib could be enhanced with glucocorticoids in another non-diabetic and non-immunosuppressed patient, but not vice versa.

Therefore, the data clearly show a synergistic effect of PARP inhibitors and glucocorticoids in treating and preventing impaired wound healing, and the effect is in particular prominent in patients suffering from diabetes, in immunosuppressed patients, as well as in patients receiving glucocorticoid therapy.

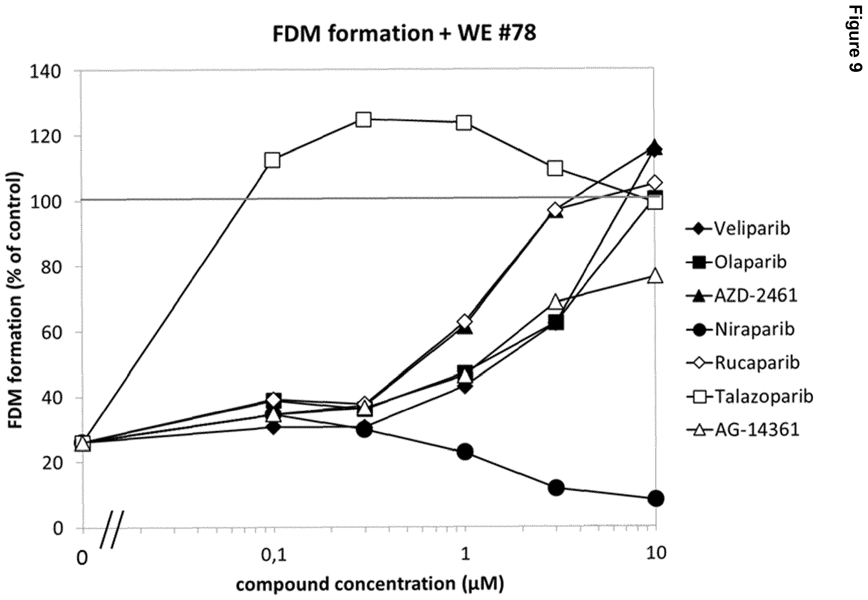

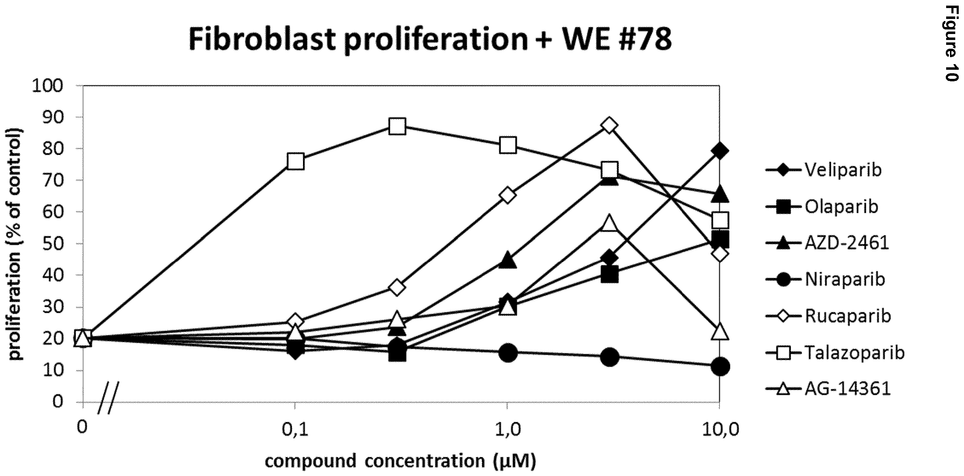

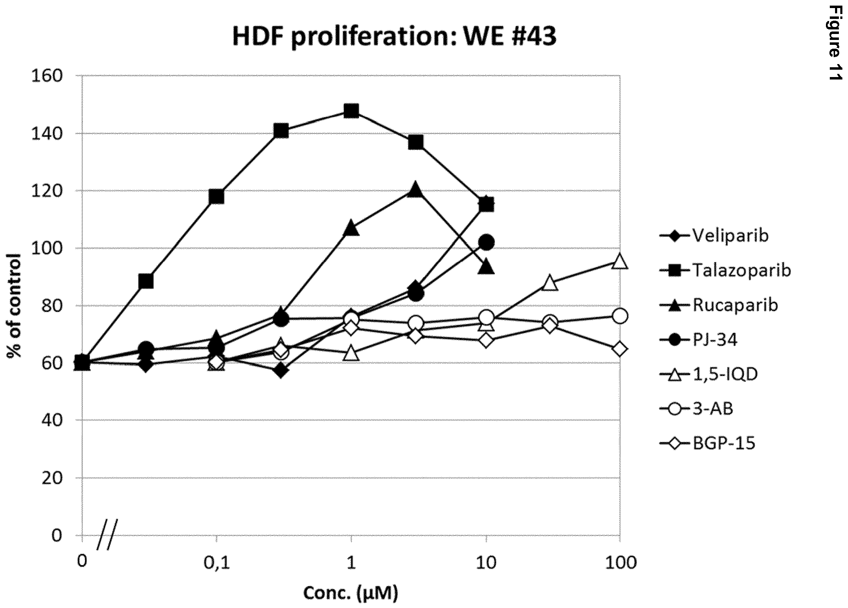

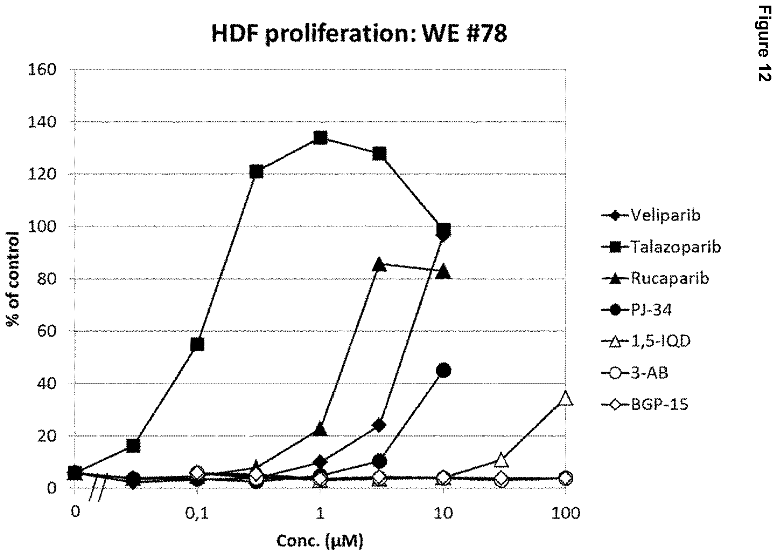

Further, several, structurally distinct PARP inhibitors exhibited a strong enhancing effect in the fibroblast-derived matrix (also called FDM or 3D FDM) formation assay (Figure 9). In particular, a strong positive effect could be shown for PARP inhibitors talazoparib, veliparib, olaparib, rucaparib, AZD-2461 and AG-14361. This enhancing effect could be confirmed in the 2D fibroblast proliferation assay (Figure 10).
Further, several of the experimental PARP inhibitors, for which some non-predictive, preliminary data in the broadest context of wound healing were described in the prior art as recited above, were found to be inactive or at least clearly less active in both the 2D fibroblast proliferation assay and the 3D fibroblast-derived matrix formation assay (Figures 11 and 12).

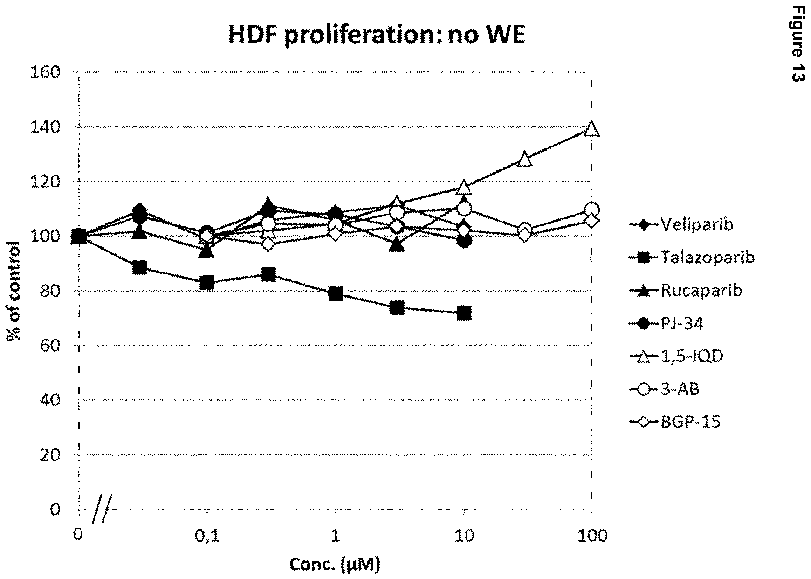

Remarkably, the PARP inhibitors have either no effect on fibroblast proliferation in the absence of wound exudate, or only a marginal effect (Figure 13).

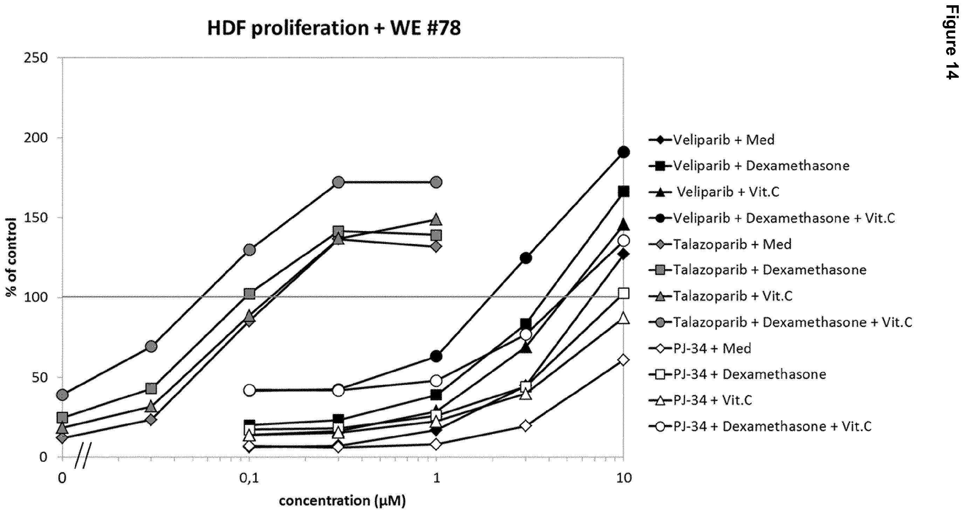

Moreover, it was surprisingly shown in the fibroblast proliferation assay and using titration of PARP inhibitors with fixed concentrations of dexamethasone (3 nM; suboptimal) and vitamin C (100 µg/ml), that both the glucocorticoid dexamethasone and vitamin C enhance the positive enhancing effect of the PARP antagonists, thereby showing a synergistic effect (Figure 14).

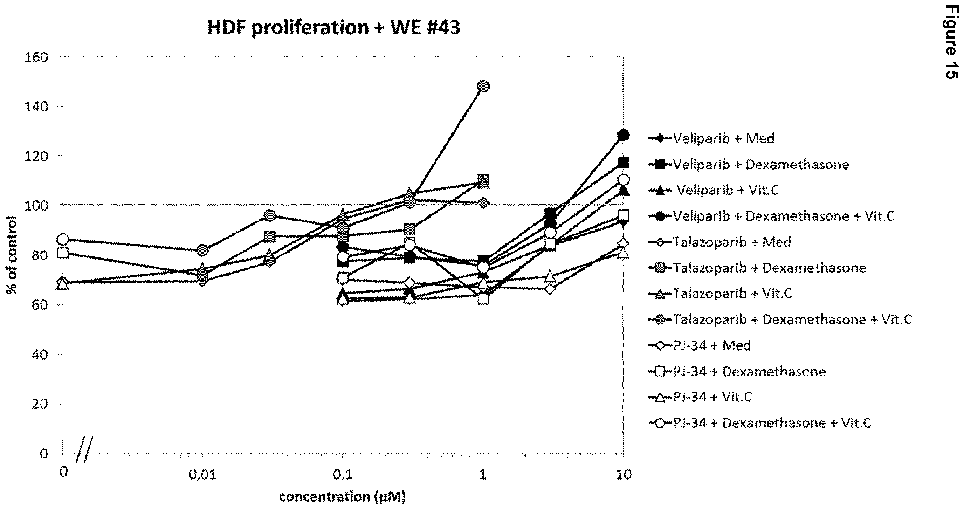

The strong and consistent synergistic effect could be confirmed both for the glucocorticoid and vitamin C for a different patient (Figure 15).

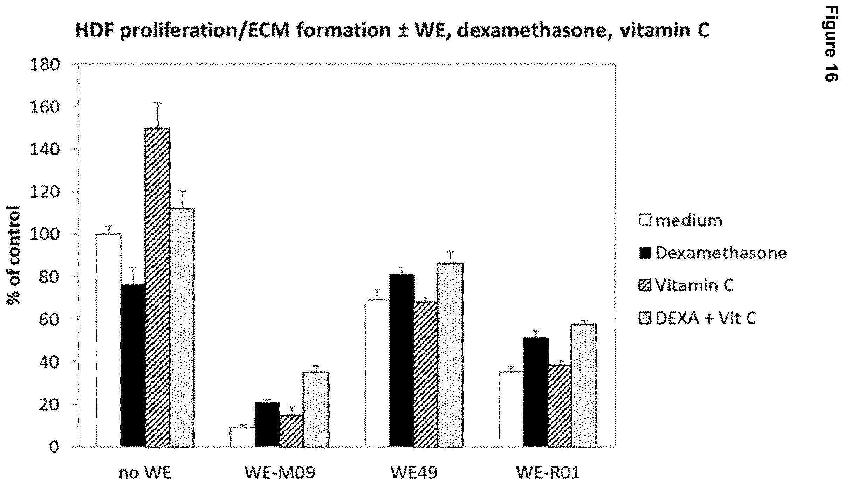

In summary, the data using the 2D fibroblast proliferation assay and the 3D fibroblast-derived matrix (FDM) formation assay show for glucocorticoids and vitamin C (Figure 16):
- In the absence of wound exudate, dexamethasone inhibits fibroblast proliferation and fibroblast-derived matrix (FDM) formation,
- In the presence of wound exudate, dexamethasone surprisingly enhances fibroblast proliferation and fibroblast-derived matrix (FDM) formation for specific wound exudates,
- In the absence of wound exudate, vitamin C enhances both fibroblast proliferation in the fibroblast proliferation assay (also called 2D assay) and fibroblast-derived matrix (FDM) formation in the fibroblast-derived matrix (FDM)) formation assay (also called 3D assay),
- In the presence of 2/3 of the wound exudates tested, vitamin C (ascorbic acid) has no effect on fibroblast proliferation in the 2D assay or fibroblast-derived matrix (FDM) formation in the 3D assay,
- When both dexamethasone and vitamin C (ascorbic acid) enhanced proliferation in the presence of wound exudate, the combination of the two was superior as compared to each compound alone, thereby surprisingly exerting a synergistic effect.

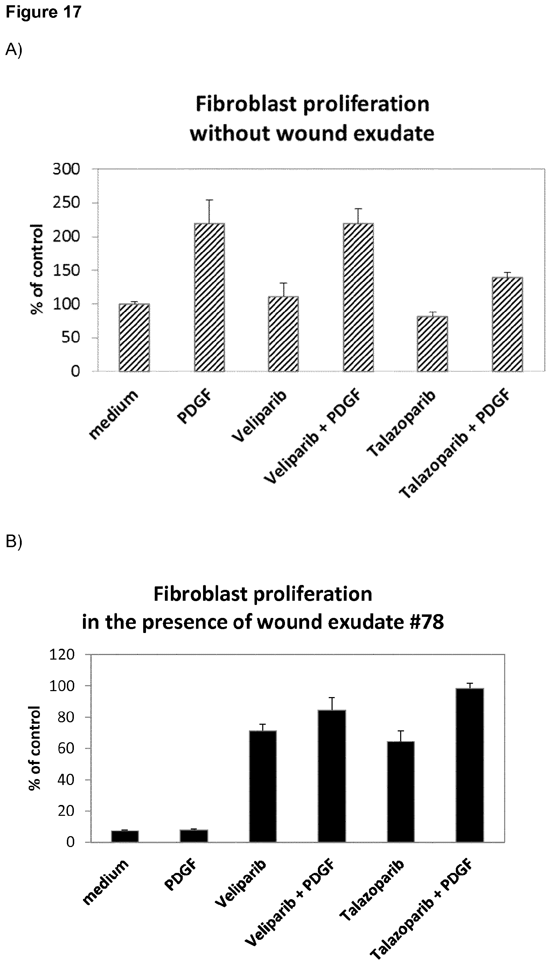

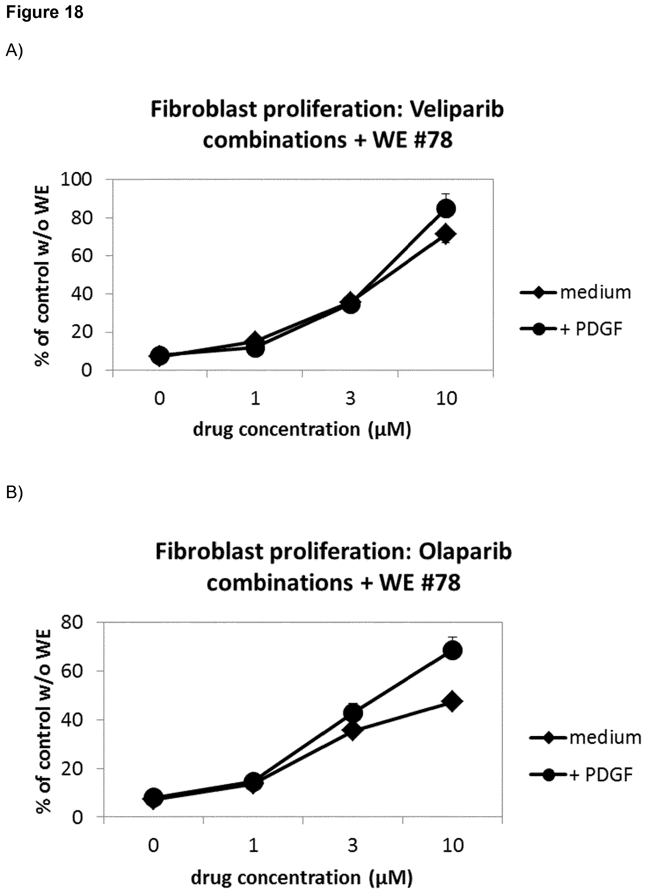

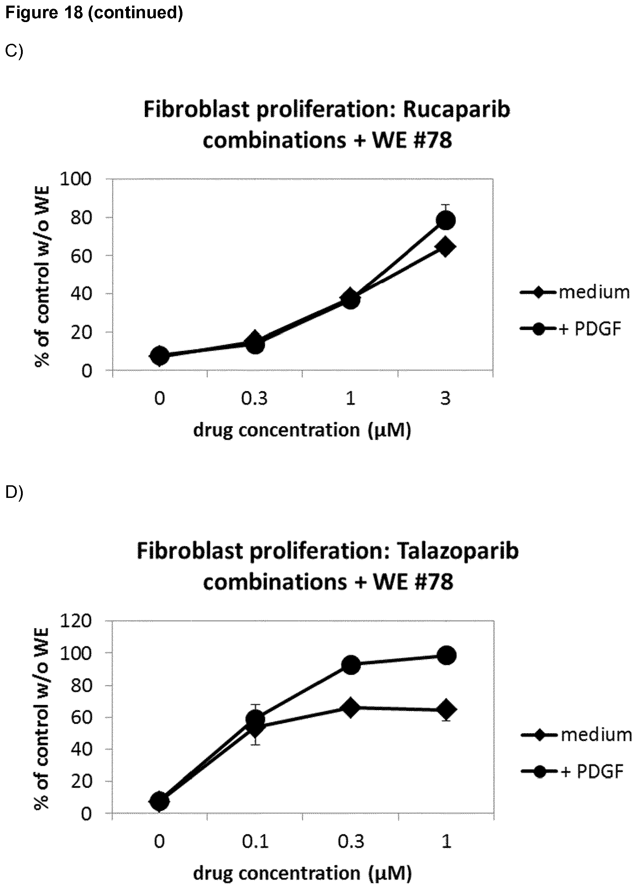

Moreover, it was surprisingly found that, in two separate experiments, the PARP inhibitors veliparib, olaparib, talazoparib and rucaparib showed an enhanced positive effect of recovery of fibroblast proliferation by PDGF in the presence of wound exudate #78 (Figure 18). In the absence of WE, PDGF induces HDF proliferation, whereas in the presence of WE #78, PDGF has no effect. Veliparib and talazoparib, at 10 µM and 1 µM, respectively, have no or an inhibitory effect on fibroblasts on their own in the absence of WE, but induce proliferation in the presence of WE #78 (Figure 17). This effect is additively or synergistically enhanced by PDGF (20 ng/ml). The results are summarized as follows: 1. In the presence of WE, veliparib and talazoparib surprisingly enhance HDF proliferation. 2. When PDGF was combined with either veliparib or talazoparib, surprisingly the combination with the protein growth factor was better than each compound alone. Moreover, veliparib, olaparib, rucaparib and talazoparib were tested ± PDGF in the 2D fibroblast culture assay on fibroblast proliferation. As shown in Figure 18, veliparib, olaparib, rucaparib and Talazoparib each show a dose-dependent increase of HDF proliferation, which, surprisingly, is even further enhanced by the addition of PDGF. This positive additive or synergistic effect of veliparib, olaparib, rucaparib and talazoparib in combination with the protein growth factor PDGF is surprising, as PDGF has no effect on the WE-induced inhibition of fibroblast proliferation in the presence of wound exudate #78. Notably, as indicated above, in the absence of WE, the PARP inhibitors either had no effect on PDGF-induced induction of proliferation or showed inhibition of the PDGF effect.

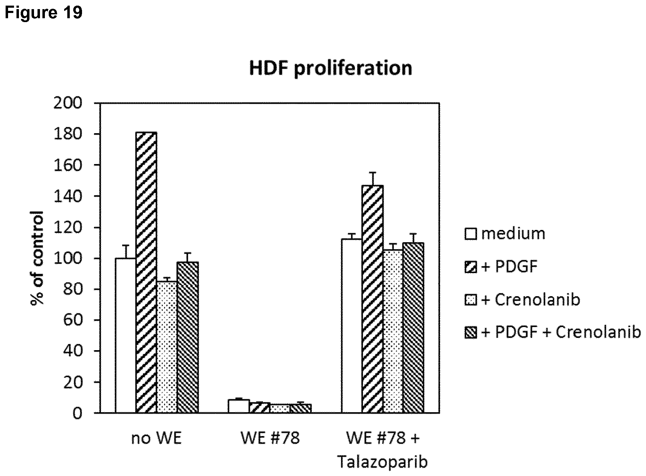

In addition, it could be shown in the 2D fibroblast assay that in the absence of wound exudate, PDGF enhances proliferation; an effect, which is completely abrogated by the PDGF receptor inhibitor crenolanib. In the presence of wound exudate alone, fibroblast proliferation is strongly inhibited, without any effect of PDGF and/or crenolanib. Talazoparib, in the presence of wound exudate, rescued the cells from the inhibitory effect of the wound exudate, and PDGF further enhanced this recovery. The PDGF effect in this system was completely abolished by crenolanib, indicating that talazoparib restores the responsiveness of the fibroblasts to PDGF in the presence of this wound exudate (Figure 19).

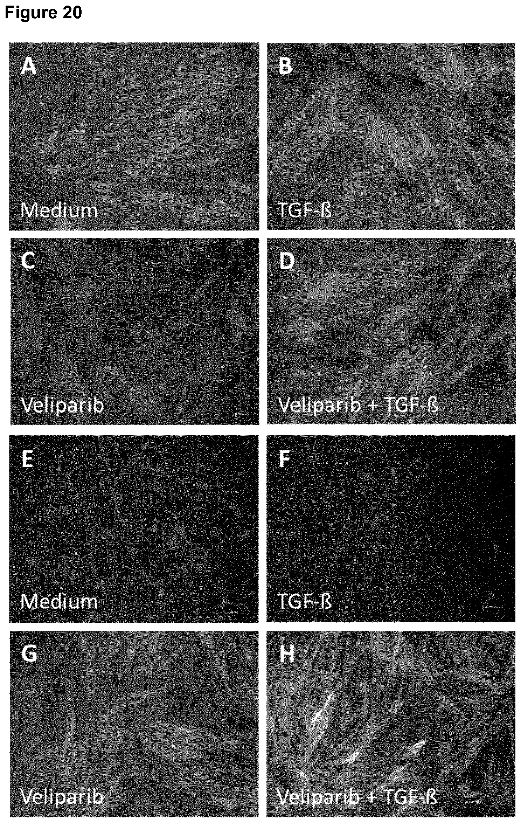

In addition, the 2D fibroblast assay was performed in the absence and presence of TGF-β to induce myofibroblast differentiation and veliparib (10 µM). In the absence of wound exudate, TGF-β increased the staining for the myofibroblast marker alpha-smooth muscle actin (a-SMA), while veliparib had no effect. In the presence of wound exudate, TGF-β did not induce α-SMA on its own, but in combination with veliparib showed more strongly stained cells than veliparib alone. Veliparib, in combination with TGF-β led to expression of α-SMA, an indicator of wound contractility (Figure 20).

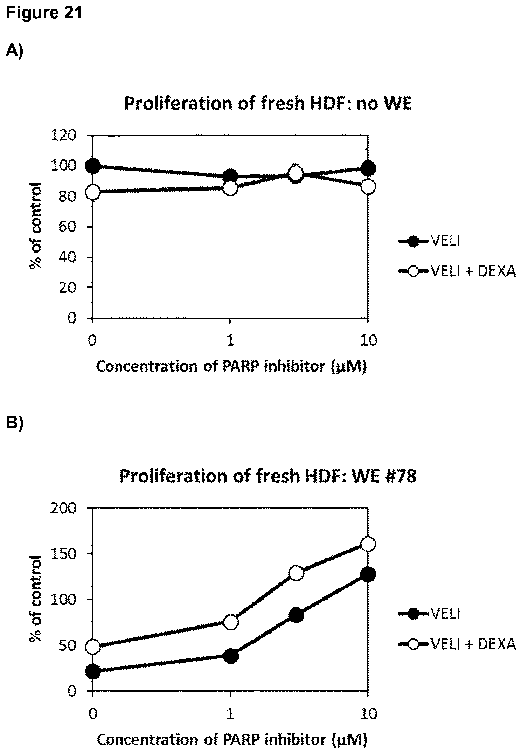

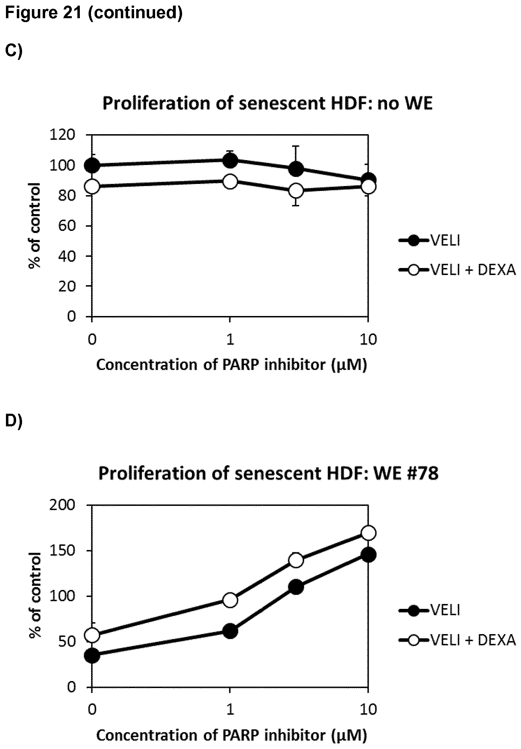

Moreover, proliferation of fresh (passage 9) and senescent (passage 21) human dermal fibroblast (HDF) was determined without and with wound exudate and the PARP inhibitor veliparib alone or in combination with dexamethasone. Veliparib was used at 1, 3 and 10 µM; dexamethasone was kept constant at a suboptimal dose (3 nM). In the absence of wound exudate, dexamethasone contributed to inhibition of HDF proliferation. In the presence of wound exudate, dexamethasone contributed to the enhancement of HDF proliferation by veliparib. This effect was observed both in fresh and senescent fibroblasts (Figure 21).

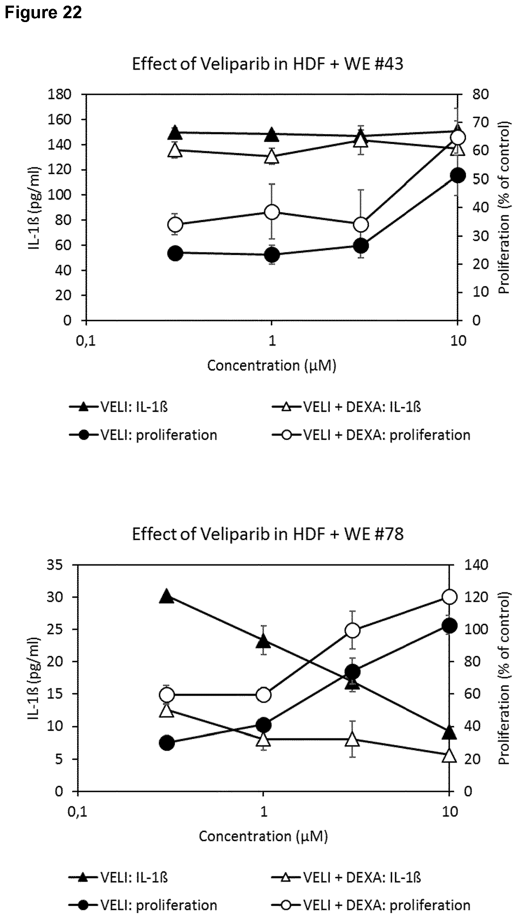

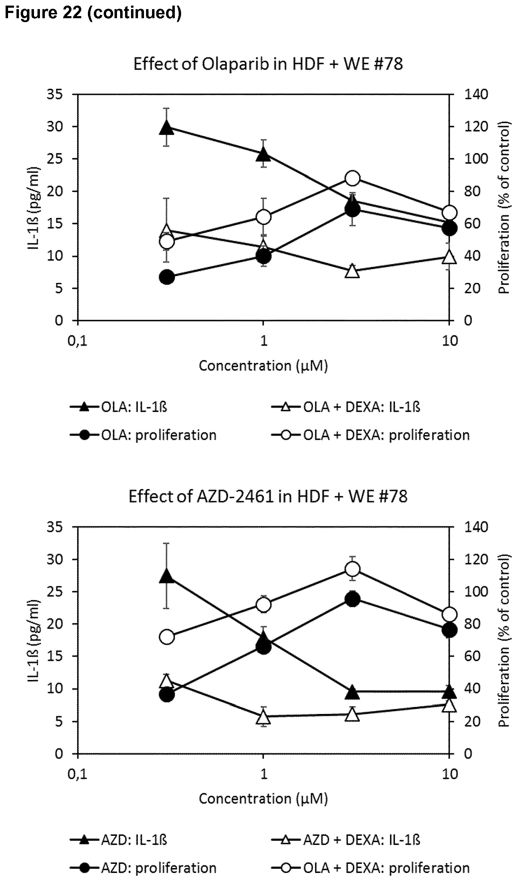

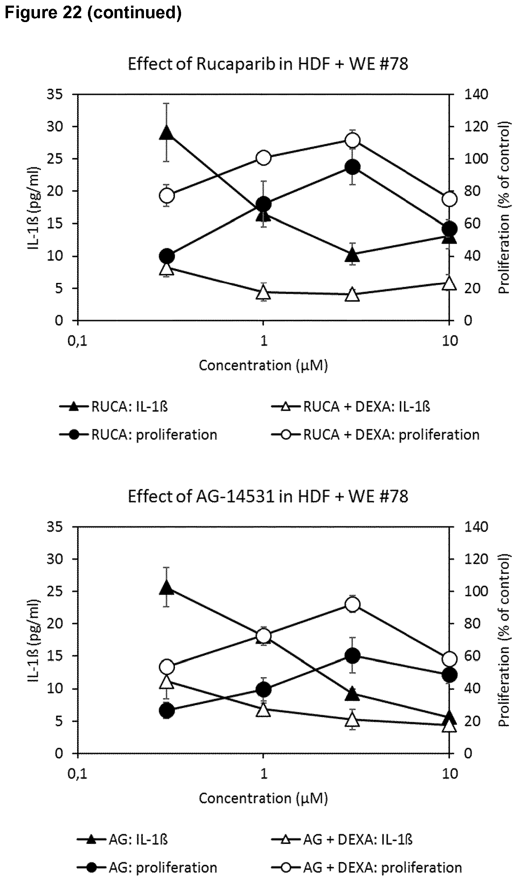

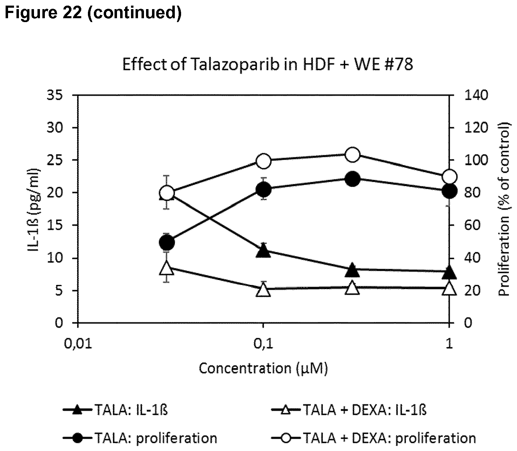

Further, the effects of different PARP inhibitors in the presence or absence of a suboptimal concentration of dexamethasone on wound exudate-induced inhibition of fibroblast proliferation and induction of IL-1β secretion was determined. Two different wound exudates (#43 and #78) were used with veliparib. The compounds veliparib, olaparib, AZD-2461, rucaparib, AG-14351 and talazoparib enhanced cell proliferation while at the same time reducing IL-1β secretion. These effects were enhanced by dexamethasone. (Figure 22).

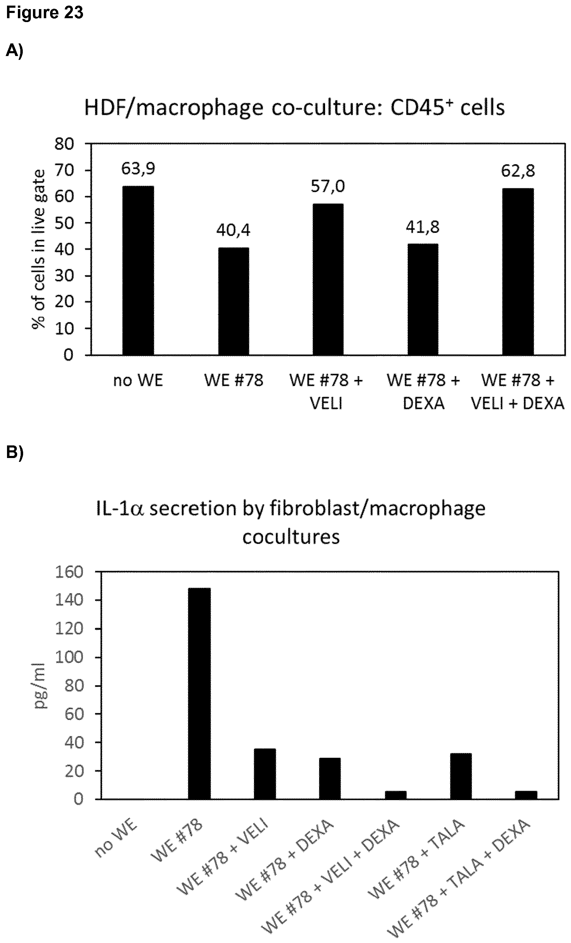

Figure 23 shows results of a fibroblast - macrophage coculture experiment with wound exudate and veliparib (10 µM) or talazoparib (0.1 µM) in the absence or presence of dexamethasone (10 nM). It was found that the percentage of live cells in the FACS CD45-gate (corresponding to macrophages), which was reduced upon incubation with wound exudate, was increased by veliparib and further increased by the combination of veliparib with dexamethasone, while dexamethasone alone had only a marginal effect. Moreover, it was found that the proinflammatory cytokine IL-1α, induced by wound exudate, was reduced by veliparib and talazoparib, and this effect was found to be enhanced by dexamethasone.

Therefore, in one aspect, the present invention relates to a poly-ADP-ribose polymerase (PARP) inhibitor for use in the prevention and/or treatment of impaired skin wound healing in a subject,
wherein the subject:
(i) is a subject treated with at least one glucocorticoid, and/or
(ii) is a subject to which a pharmaceutical, nutritional supplement or dietary supplement comprising ascorbic acid or a pharmaceutically acceptable salt thereof is administered, and/or
(iii) is a subject treated with at least one protein growth factor.

In one preferred embodiment, the present invention relates to a poly-ADP-ribose polymerase (PARP) inhibitor for use in the prevention and/or treatment of impaired skin wound healing in a subject,
wherein the subject is a subject treated with at least one glucocorticoid, and optionally further:
- is a subject to which a pharmaceutical, nutritional supplement or dietary supplement comprising ascorbic acid or a pharmaceutically acceptable salt thereof is administered, and/or
- is a subject treated with at least one protein growth factor.

In another preferred embodiment, the present invention relates to a poly-ADP-ribose polymerase (PARP) inhibitor for use in the prevention and/or treatment of impaired skin wound healing in a subject,
wherein the subject is a subject treated with at least one protein growth factor, and optionally further:
- is a subject treated with at least one glucocorticoid, and/or
- is a subject to which a pharmaceutical, nutritional supplement or dietary supplement comprising ascorbic acid or a pharmaceutically acceptable salt thereof is administered.

In another preferred embodiment, the present invention relates to a poly-ADP-ribose polymerase (PARP) inhibitor for use in the prevention and/or treatment of impaired skin wound healing in a subject,
wherein the subject is a subject treated with at least one protein growth factor, and optionally further:
- is a subject treated with at least one glucocorticoid, and/or
- is a subject to which a pharmaceutical, nutritional supplement or dietary supplement comprising ascorbic acid or a pharmaceutically acceptable salt thereof is administered.

In another preferred embodiment, the present invention relates to a poly-ADP-ribose polymerase (PARP) inhibitor for use in the prevention and/or treatment of impaired skin wound healing in a subject,
wherein the subject is a subject to which a pharmaceutical, nutritional supplement or dietary supplement comprising ascorbic acid or a pharmaceutically acceptable salt thereof is administered, and optionally further:
- is a subject treated with at least one glucocorticoid, and/or
- is a subject treated with at least one protein growth factor.

The experimental data summarized above show a beneficial effect for a PARP inhibitor in the context of a wound exudate from a patient who already receives a glucocorticoid therapy, e.g. as immunosuppressive therapy in the context of a prior organ transplantation. Further, a synergistic effect was observed in the examples for PARP inhibitors in combination with a glucocorticoid and/or vitamin C (ascorbic acid) and for PARP inhibitors in combination with protein growth factors. Accordingly, a PARP inhibitor is surprisingly found to be suitable to treat or prevent impaired skin wound healing in subjects, that already receive a glucocorticoid therapy or to which Vitamin C is already administered as a pharmaceutical, nutritional supplement or dietary supplement, or that already receive a protein growth factor therapy.

A subject treated with at least one glucocorticoid is a subject to which a glucocorticoid was administered at least once, preferably several times within at least 1 or 2 weeks or months prior to the administration of the PARP inhibitor. In a preferred embodiment, the glucocorticoid is administered to said patient repetitively, such as 1, 2, 3, 4, 5, 6, 7, 8, 9, 10 or more times, in particular over a time period of 1, 2, 3, 4, or 5 weeks or months or more. The glucocorticoid therapy may be a systemic therapy, such as an oral therapy, or a local therapy, such as a topical therapy. The subject is preferably treated with a therapeutically effective dose and regimen for treating the co-morbidity treatable with the respective glucocorticoid. Typically, for systemic applications, the glucocorticoid dose will be in the range of about 0,1 to 1000 mg/day, depending on the glucocorticoid and disease to be treated. Topical formulations of glucocorticoids are typically administered in a concentration of 0,01 to 10 % (w/v), 0, 001 to 6 % (w/v) or 0,001 to 1 % (w/v), such as 0,01 to 0,1% (w/v), such as a cream, gel, lotion, ointment or the like.

Co-morbidities that may be treated with a glucocorticoid are known in the art and include immunosuppression in the context of organ transplantation and Graft versus Host Disease (GvHD), allergic disorders, such as asthma, atopic dermatitis, contact dermatitis, drug hypersensitivity reactions, perennial or seasonal allergic rhinitis, and serum sickness, dermatologic diseases, such as bullous dermatitis herpetiformis, dermatitis, atopic dermatitis, eczema, itching, psoriasis, exfoliative erythroderma, mycosis fungoides, pemphigus, and severe erythema multiforme (Stevens-Johnson syndrome), endocrine disorders, such as primary or secondary adrenocortical insufficiency, congenital adrenal hyperplasia, hypercalcemia associated with cancer, and thyroiditis, gastrointestinal diseases, such as regional enteritis and ulcerative colitis, hematologic disorders, such as acquired (autoimmune) hemolytic anemia, congenital (erythroid) hypoplastic anemia (Diamond-Blackfan anemia), idiopathic thrombocytopenic purpura, pure red cell aplasia, and secondary thrombocytopenia; trichinosis with neurologic or myocardial involvement, tuberculous meningitis when used with appropriate antituberculous chemotherapy, for the palliative management of leukemias and lymphomas; diseases of the nervous system, such as acute exacerbations of multiple sclerosis, cerebral edema associated with primary or metastatic brain tumor, craniotomy, or head injury, ophthalmic diseases, such as sympathetic ophthalmia, temporal arteritis, uveitis, and ocular inflammatory conditions; renal diseases, such as idiopathic nephrotic syndrome or lupus erythematosus, respiratory diseases, such as berylliosis, fulminating or disseminated pulmonary tuberculosis, idiopathic eosinophilic pneumonias, symptomatic sarcoidosis; rheumatic disorders, such as acute gouty arthritis, acute rheumatic carditis, ankylosing spondylitis, psoriatic arthritis, rheumatoid arthritis, including juvenile rheumatoid arthritis and for the treatment of dermatomyositis, polymyositis, and systemic lupus erythematosus.

The glucocorticoid treatment is typically administered to patients to treat an underlying co-morbidity, such as an immunosuppressive therapy in the context of transplantation of a graft, or for locally treating a skin disorder such as atopic dermatitis or psoriasis.

A subject treated with a pharmaceutical, nutritional supplement or dietary supplement comprising ascorbic acid or a pharmaceutically acceptable salt thereof is a subject to which a pharmaceutical, nutritional supplement or dietary supplement comprising ascorbic acid or a pharmaceutically acceptable salt thereof was administered at least once, preferably several times within at least 1 or 2 weeks prior to the administration of the PARP inhibitor. In a preferred embodiment, the pharmaceutical, nutritional supplement or dietary supplement comprising ascorbic acid or a pharmaceutically acceptable salt thereof is administered to said patient repetitively, such as 1, 2, 3, 4, 5, 6, 7, 8, 9, 10 or more times, in particular over a time period of 1, 2, 3, 4, or 5 weeks or months or more. The administration of the pharmaceutical, nutritional supplement or dietary supplement comprising ascorbic acid or a pharmaceutically acceptable salt thereof may be a systemic administration, such as oral administration, or local administration, such as topical administration. For example, a pharmaceutical, nutritional supplement or dietary supplement comprising ascorbic acid or a pharmaceutically acceptable salt thereof for oral administration may contain 50 mg to 1 g per dose, such as tablets, pills or capsules.

A pharmaceutical, nutritional supplement or dietary supplement comprising ascorbic acid or a pharmaceutically acceptable salt thereof may be administered to a patient to treat or prevent a vitamin C deficiency such as scurvy, or to maintain general well-being.

A subject treated with at least one protein growth factor is a subject to which a protein growth factor was administered at least once, preferably several times within at least 1 or 2 weeks or months prior to the administration of the PARP inhibitor. In a preferred embodiment, the protein growth factor is administered to said patient repetitively, such as 1, 2, 3, 4, 5, 6, 7, 8, 9, 10 or more times, in particular over a time period of 1, 2, 3, 4, or 5 weeks or months or more. The protein growth factor therapy may be a systemic therapy, such as an oral therapy, or a local therapy, such as a topical therapy, preferably the therapy is a topical therapy. The subject is preferably treated with a therapeutically effective dose and regimen for treating or preventing impaired wound healing, or for treating an underlying co-morbidity, such as lung fibrosis in the case of TGF-β. Typically, for topical applications, the topical formulations of protein growth factors are typically administered in a concentration of 0,0001 to 10 % (w/v), 0,0001 to 6 % (w/v) or 0,0001 to 1 % (w/v), such as 0,001 to 0,1% (w/v), such as a cream, gel, lotion, ointment or the like. In particular, a gel containing 0.01% PDGF-BB (becaplermin) may be used, which is marketed as Regranex®. The protein growth factor is in a preferred embodiment a human protein growth factor and/or is selected from a platelet derived growth factor (PDGF), transforming growth factor beta (TGF-β), basic fibroblast growth factor (bFGF), keratinocyte growth factor (KGF), epidermal growth factor (EGF), Insulin-like growth factor 1 (IGF-1), vascular endothelial growth factor (VEGF) and (hepatocyte growth factor) HGF. In an even more preferred embodiment, the protein growth factor is selected from a platelet derived growth factor (PDGF), transforming growth factor beta (TGF-β), and basic fibroblast growth factor (bFGF), most preferably the protein growth factor is PDGF, in particular becaplermin.

Alternatively, a PARP inhibitor may be combined with a pharmaceutical composition comprising a glucocorticoid, and/or ascorbic acid or a pharmaceutically acceptable salt thereof and/or a pharmaceutical composition comprising a protein growth factor, for treating impaired skin wound healing in a subject. The PARP inhibitor may be administered to the subject simultaneously with a glucocorticoid, and/or ascorbic acid and/or a protein growth factor, or temporally separated therefrom and/or spatially separate or together, such as a pharmaceutical composition comprising a PARP inhibitor and one, two or three of a glucocorticoid, a protein growth factor and ascorbic acid.

Therefore, in another aspect, the present invention relates to a poly-ADP-ribose polymerase (PARP) inhibitor in combination with one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
for use in the treatment of impaired skin wound healing in a subject.

In one preferred embodiment, the present invention relates to a poly-ADP-ribose polymerase (PARP) inhibitor in combination with a pharmaceutical composition comprising a glucocorticoid, for use in the treatment of impaired skin wound healing in a subject, optionally further in combination with ascorbic acid or a pharmaceutically acceptable salt thereof, and/or a pharmaceutical composition comprising a protein growth factor.

In another preferred embodiment, the present invention relates to a poly-ADP-ribose polymerase (PARP) inhibitor in combination with a pharmaceutical composition comprising a protein growth factor, for use in the treatment of impaired skin wound healing in a subject, optionally further in combination with ascorbic acid or a pharmaceutically acceptable salt thereof, and/or a pharmaceutical composition comprising a glucocorticoid.

In another preferred embodiment, the present invention relates to a poly-ADP-ribose polymerase (PARP) inhibitor in combination with ascorbic acid or a pharmaceutically acceptable salt thereof, for use in the treatment of impaired skin wound healing in a subject, optionally further in combination with a pharmaceutical composition comprising a protein growth factor, and/or a pharmaceutical composition comprising a glucocorticoid.

Further, the PARP inhibitor may be provided as a pharmaceutical composition separate from the pharmaceutical composition comprising a glucocorticoid, and/or ascorbic acid or a pharmaceutically acceptable salt thereof, and/or a protein growth factor. Alternatively, a pharmaceutical composition comprising a PARP inhibitor and one, two or three, preferably one or two, even more preferably oneof the following (i) to (iii) may be provided for the use of the present invention: (i) a pharmaceutical composition comprising a glucocorticoid, (ii) ascorbic acid or a pharmaceutically acceptable salt thereof, (iii) a pharmaceutical composition comprising a protein growth factor.

Further, a pharmaceutical composition comprising a poly-ADP-ribose polymerase (PARP) inhibitor and one, two or three of the following (i) (iii):
(i) a pharmaceutical composition comprising a glucocorticoid,
(ii) ascorbic acid or a pharmaceutically acceptable salt thereof,
(iii) a pharmaceutical composition comprising a protein growth factor,
may be provided as kit-of-parts for use in the treatment of impaired skin wound healing in a subject.

The combination of a PARP inhibitor and a glucocorticoid is particularly preferred for the uses according to the present invention.

Therefore, in a preferred embodiment, the present invention relates to a poly-ADP-ribose polymerase (PARP) inhibitor in combination with a glucocorticoid, and optionally further in combination with ascorbic acid or a pharmaceutically acceptable salt thereof or a protein growth factor, for use in the treatment of impaired skin wound healing in a subject.

Further, the combination of a PARP inhibitor and a protein growth factor is particularly preferred for the uses according to the present invention.

Therefore, in another preferred embodiment, the present invention relates to a poly-ADP-ribose polymerase (PARP) inhibitor in combination with a protein growth factor and optionally further in combination with ascorbic acid or a pharmaceutically acceptable salt thereof or a glucocorticoid, for use in the treatment of impaired skin wound healing in a subject.

The terms "treat", "treating" and "treatment" refer to alleviating or abrogating a disease and/or its attendant symptoms. The term "prevention" or "prevent" refers to treatment that prevents the occurrence of a condition in a subject.

A "wound" is understood as damage to a tissue of a living individual, such as cuts, tears, burns, or breaks, preferably a wound is understood as open injury of a tissue of a living individual.

The present invention relates to compounds, and compositions comprising such compounds, as well as combinations of such compounds and compositions, for the prevention and/or treatment of impaired skin wound healing in a subject.

Accordingly, a "skin wound" is understood as a damage to a skin of a living individual, such as cuts, tears, burns, or breaks. Preferably, a skin wound is understood as open injury of the skin of a living individual. The skin may be located at any area of an individual, such as for example the head, the arms, the legs, the chest, or the back. Further, the individual may have one, two, three, four or more skin wounds. Further, the area of a skin wound may differ. In a preferred embodiment, the skin wound forms wound exudate. In another preferred embodiment, the skin wound forms a wound biofilm.

"Impaired skin wound healing" refers to a skin wound which does not heal at an expected rate. In a preferred embodiment, the impaired skin wound healing is a non-healing skin wound or chronic skin wound. A non-healing skin wound is preferably understood as a skin wound which does not close within 2 months under standard therapy, preferably within 3 or more months under standard therapy. Preferably, a non-healing skin wound is characterized by a lack of wound closure, an increase of the area and/or depth of the wound, necrosis and/or infections of the skin wound, and/or lack of granulation.

As used herein, a "healing skin wound" is understood as a skin wound which heals at an expected rate, in particular, as a skin wound which closes within 2 months under standard therapy. Preferably, a healing skin wound is characterized by ongoing wound closure, granulation, absence of necrosis and/or absence of infections.

The subject or individual may be an otherwise healthy individual or may exhibit further diseases and/or co-morbidities, and/or is treated with medication(s) for further diseases and/or co-morbidities. In a preferred embodiment, the subject or individual, in addition to impaired skin wound healing, exhibits further diseases, and/or co-morbidities, and/or is treated with medication(s) for further diseases and/or co-morbidities.

In a preferred embodiment of any of the above aspects of the invention of a poly-ADP-ribose polymerase (PARP) inhibitor for use, the subject suffers from at least one comorbidity associated with impaired skin wound healing, and/or the subject is treated with at least one glucocorticoid for treating and/or preventing at least one comorbidity associated with impaired skin wound healing.

In one preferred embodiment the subject suffers from at least one co-morbidity associated with impaired skin wound healing. Such co-comorbidities are for example diabetes, suppressed immune system following transplantation of a graft and GvHD. Further co-morbidities include adipositas, increased blood pressure, venous stasis or peripheral arterial occlusion. Further co-morbidities are diseases treatable with glucocorticoids as recited above.

A co-morbidity is understood as the presence of one or more additional diseases or disorders co-occurring with a given disease.

It was surprisingly found that a) the treatment of a subject with a PARP inhibitor in combination with a glucocorticoid and/or ascorbic acid and/or a protein growth factor, as well as b) the treatment of a subject with a PARP inhibitor wherein the subject receives glucocorticoid treatment and/or ascorbic acid treatment and/or protein growth factor treatment,
is in particular effective in those subjects suffering from at least one comorbidity associated with impaired skin wound healing such as diabetes, suppressed immune system following transplantation of a graft and GvHD.

In a preferred embodiment of the present invention, the subject has undergone transplantation of a graft, and/or obtains immunosuppressive therapy, and/or is treated with at least one immunosuppressive drug.

Therefore, in a yet another preferred embodiment of any of the above aspects of the invention, the subject:
(i) has undergone transplantation of a graft, and/or
(ii) obtains immunesuppressive therapy, and/or
(iii) is treated with at least one immunosuppressive drug, such as a glucocorticoid or a calcineurin inhibitor.

In a yet further preferred embodiment, the subject further suffers from diabetes.

Therefore, in one preferred embodiment, immunosuppressive therapy is by administering a glucocorticoid and/or a calcineurin inhibitor. In another preferred embodiment, the immunosuppressive drug is selected from a glucocorticoid and a calcineurin inhibitor. Suitable calcineurin inhibitors are known in the art and include tacrolimus, pimecrolimus and cyclosporin A. Suitable glucocorticoids are described below in more detail.

The skin wound of the subject may already receive a treatment such as a standard therapy for treating wound healing or may be untreated regarding the skin wound.

"Standard therapy" is understood as a treatment recommended in general by physicians for skin wounds, in particular one or more selected from wound dressings, surgical and biological (maggot) debridement, infection control, negative pressure therapy, and therapy with a biological or cell treatment.

Therefore, in one preferred embodiment the skin wound of the subject may be untreated or treated with standard therapy for treating wound healing or with one or more of the following for treating wound healing: compression, wound dressings, surgical debridement, biological debridement, infection control, antibiotic therapy, negative pressure therapy, proteins, in particular protein growth factors, antibodies, peptides, sugars, cells or cell constituents, artificial skin, human blood-derived products, gene therapy or genetically engineered wound bed modifications, drugs, herbal medicines, or plant extracts. In one preferred embodiment, the skin wound of the subject may be untreated or treated with standard therapy for treating wound healing wherein the standard therapy does not include treatment with protein growth factors. In another preferred embodiment, the skin wound of the subject may be untreated or treated with standard therapy for treating wound healing wherein the standard therapy includes treatment with protein growth factors.

The invention may be used to treat or prevent different types of skin wounds exhibiting impaired skin wound healing. Different types of skin wounds exhibiting impaired skin wound healing which can be treated in accordance with the present invention include a wound of a diabetic patient, a skin wound which is infected by at least one microorganism, an ischemic wound, a wound in a patient suffering from deficient blood supply or venous stasis, an ulcer, such as a diabetic ulcer, venous ulcer, arterial ulcer, such as ulcus cruris arteriosum, mixed ulcer, or pressure ulcer, a neuropathic wound, ulcus cruris, surgical wound, burn, dehiscence, neoplastic ulcer, a bullous skin disease, such as epidermolysis bullosa, and rare ulcer. Microorganisms infecting skin wounds are known in the art and include bacteria and fungi, such as corynebacteria, staphylococci, streptococci, and yeasts such as candida species.

In yet another preferred embodiment of any of the above aspects of the invention, the skin wound is selected from a wound of a diabetic patient, a skin wound which is infected by at least one microorganism, an ischemic wound, a wound in a patient suffering from deficient blood supply or venous stasis, an ulcer, such as a diabetic ulcer, venous ulcer, arterial ulcer, such as ulcus cruris arteriosum, mixed ulcer, or pressure ulcer, a neuropathic wound, ulcus cruris, surgical wound, burn, dehiscence, neoplastic ulcer, a bullous skin disease, such as epidermolysis bullosa, and rare ulcer.

For example, the PARP inhibitors were shown to be useful in the medical uses of the present invention for the treatment of a plurality of skin wounds, including, in particular, a wound of a diabetic patient and diabetic ulcers.

Therefore, in yet another preferred embodiment of any of the above aspects of the invention, the skin wound is selected from a wound of a diabetic patient and/or a diabetic ulcer.

An ulcer is understood as a sore on the skin, accompanied by the disintegration of tissue. Ulcers can result in complete loss of the epidermis and often portions of the dermis and even subcutaneous fat.

The "subject" or "individual" is an animal, preferably the individual is a vertebrate, in particular a mammal, more preferably a human.

Moreover, it was surprisingly found that the administration of a PARP inhibitor is particularly effective in case of a patient who already receives a glucocorticoid therapy, namely a treatment with prednisolone, for treating an underlying co-morbidity.

The treatment with at least one glucocorticoid of a patient already receiving a glucocorticoid therapy may occur by various routes of administration, depending on the co-morbidity treated by the glucocorticoid, and may in particular be systemic or cutaneous administration. For example, the co-morbidity may be a skin disease such as eczema, dermatitis, atopic dermatitis or psoriasis. In this case, the subject may be treated by topical, in particular cutaneous, administration, e.g. with a glucocorticoid-containing cream, lotion, gel or the like, or by systemic administration, in particular oral administration, such as a glucocorticoid-containing tablet or pill. For example, the co-morbidity may be transplantation of a graft and/or GvHD. In this case, the subject may be treated by systemic administration, in particular oral administration, such as a glucocorticoid-containing tablet or pill.

Therefore, in yet another preferred embodiment of any of the above aspects of the invention, the subject is treated with at least one glucocorticoid by systemic or cutaneous administration.

Further, the administration of a PARP inhibitor is particularly effective in case of a patient who already receives a protein growth factor therapy, in particular selected from a platelet derived growth factor (PDGF), transforming growth factor beta (TGF-β), basic fibroblast growth factor (bFGF), keratinocyte growth factor (KGF), epidermal growth factor (EGF), Insulin-like growth factor 1 (IGF-1), vascular endothelial growth factor (VEGF) and hepatocyte growth factor (HGF) therapy, for treating an underlying co-morbidity or from treating or preventing impaired wound healing.

The treatment with at least one protein growth factor of a patient already receiving a protein growth factor therapy may occur by various routes of administration, depending on the co-morbidity treated by the protein growth factor, and may in particular be systemic or cutaneous administration. In case of preventing or treating a skin disease such as wound healing, the protein growth factor is preferably administered topically or cutaneously. In this case, the subject may be treated by topical, in particular cutaneous, administration, e.g. with a protein growth factor-containing cream, lotion, gel or the like. For example, the patient already receives becaplermin (PDGF-BB) for treating or preventing impaired wound healing. In this case, the subject may be treated by topical administration, e.g. with a PDGF-BB-containing gel (Regranex®). In another example, the co-morbidity may be lung fibrosis, such as for a patient treated with TGF-β. In another example, the co-morbidity may be cancer or side-effects from cancer chemotherapy, such as oral mucositis, such as for a patient treated with human KGF (palifermin; recombinant KGF). In these cases, the subject may be treated by systemic administration, in particular oral administration, such as a protein growth factor-containing tablet or pill or by injection, such as intravenous injection. For example, palifermin may be administered by bolus injection of a buffered solution of palifermin, e.g. at a dose of 50 to 300 µg/kg bw, such as 180 µg/kg bw.

Therefore, in yet another preferred embodiment of any of the above aspects of the invention, the subject is treated with at least one protein growth factor by systemic or topical administration, more preferably by topical, in particular cutaneous administration.

The examples surprisingly show a synergistic effect of a PARP inhibitor both with a glucocorticoid and/or vitamin C. Therefore, the combination of a PARP inhibitor with one or both of a glucocorticoid and/or vitamin C is for the first time shown to be particularly useful in the context of a medical treatment. The examples further surprisingly show a synergistic effect of a PARP inhibitor with a protein growth factor. Therefore, the combination of a PARP inhibitor with a protein growth factor is for the first time shown to be particularly useful in the context of a medical treatment.

Therefore, in another aspect, the present invention relates to a poly-ADP-ribose polymerase (PARP) inhibitor in combination with one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
for use as a medicament.

In one preferred embodiment, the present invention relates to a poly-ADP-ribose polymerase (PARP) inhibitor in combination with one or both of the following (i) and (ii):
(i) a pharmaceutical composition comprising a glucocorticoid,
(ii) ascorbic acid or a pharmaceutically acceptable salt thereof,
for use as a medicament.

In another preferred embodiment, the present invention relates to a poly-ADP-ribose polymerase (PARP) inhibitor in combination with a pharmaceutical composition comprising a protein growth factor, for use as a medicament.

It is understood that the preferred embodiments described in the context of other embodiments of the present invention also apply this embodiment of the invention.

For example, in one preferred embodiment, a PARP inhibitor may be combined with a pharmaceutical composition comprising a glucocorticoid, and/or ascorbic acid or a pharmaceutically acceptable salt thereof, for treating a disease or disorder in a subject. In another preferred embodiment, a PARP inhibitor may be combined with a pharmaceutical composition comprising a protein growth factor for treating a disease or disorder in a subject. In yet another, a PARP inhibitor may be combined with a pharmaceutical composition comprising a glucocorticoid and a protein growth factor for treating a disease or disorder in a subject. The PARP inhibitor may be administered to the subject simultaneously with a glucocorticoid, and/or ascorbic acid and/or a protein growth factor, or temporally separated therefrom and/or spatially separate or together, such as a pharmaceutical composition comprising a PARP inhibitor and one, two or three of a glucocorticoid, a protein growth factor and ascorbic acid.

In another aspect, the present invention relates to a kit, or kit-of-parts, comprising:
(a) a pharmaceutical composition comprising a poly-ADP-ribose polymerase (PARP) inhibitor,
   and one, two or three of the following (b) to (d):
(b) a pharmaceutical composition comprising a glucocorticoid,
(c) ascorbic acid or a pharmaceutically acceptable salt thereof,
(d) a pharmaceutical composition comprising a protein growth factor.

In preferred embodiments, the kit or kit-of-parts comprise:
- (a) and (b), and optionally (c) and/or (d), or
- (a) and (d), and optionally (c) and/or (b), or
- (a) and (b) and (c), and optionally (d), or
- (a) and (c) and optionally (b) and/or (d), or
- (a) and (c) and (d) and optionally (d),
- (a) and (b) and (c) and (d).

Ascorbic acid or a pharmaceutically acceptable salt thereof may be provided in any suitable form, such as a pharmaceutical, nutritional supplement or dietary supplement comprising ascorbic acid or a pharmaceutically acceptable salt thereof.

The kit or kit-of-parts may for example comprise a pharmaceutical composition comprising a poly-ADP-ribose polymerase (PARP) inhibitor, and a pharmaceutical composition comprising a glucocorticoid, and optionally further ascorbic acid or a pharmaceutically acceptable salt thereof and/or a pharmaceutical composition comprising a protein growth factor. Ascorbic acid or pharmaceutically acceptable salt thereof and/or the protein growth factor may be contained in the pharmaceutical composition comprising a poly-ADP-ribose polymerase (PARP) inhibitor, and/or a pharmaceutical composition comprising a glucocorticoid, or as (a) separate composition(s).

The kit or kit-of-parts may for example comprise a pharmaceutical composition comprising a poly-ADP-ribose polymerase (PARP) inhibitor, and a pharmaceutical composition comprising a protein growth factor, and optionally further a pharmaceutical composition comprising a glucocorticoid and/or ascorbic acid or a pharmaceutically acceptable salt thereof. Ascorbic acid or pharmaceutically acceptable salt thereof and/or the glucocorticoid may be contained in the pharmaceutical composition comprising a poly-ADP-ribose polymerase (PARP) inhibitor, and/or a pharmaceutical composition comprising a protein growth factor, or as (a) separate composition(s).

In one preferred embodiment, the kit comprises a pharmaceutical composition comprising a poly-ADP-ribose polymerase (PARP) inhibitor,
and one, two or three of the following (b) to (d):
(b) a pharmaceutical composition comprising a glucocorticoid,
(c) ascorbic acid or a pharmaceutically acceptable salt thereof,
(d) a pharmaceutical composition comprising a protein growth factor,
wherein the PARP inhibitor and one, two or three of the glucocorticoid, protein growth factor and ascorbic acid or pharmaceutically acceptable salt thereof, are comprised in the same pharmaceutical composition.

In one further preferred embodiment, the kit comprises a pharmaceutical composition comprising a poly-ADP-ribose polymerase (PARP) inhibitor, and a pharmaceutical composition comprising a protein growth factor,
and optionally further one or both of the following (b) and (c):
(b) a pharmaceutical composition comprising a glucocorticoid,
(c) ascorbic acid or a pharmaceutically acceptable salt thereof,
wherein the PARP inhibitor and the protein growth factor and optionally one or both of the glucocorticoid, and ascorbic acid or pharmaceutically acceptable salt thereof, are comprised in the same pharmaceutical composition.

In one further preferred embodiment, the kit comprises a pharmaceutical composition comprising a poly-ADP-ribose polymerase (PARP) inhibitor,
and one or both of the following (b) and (c):
(b) a pharmaceutical composition comprising a glucocorticoid,
(c) ascorbic acid or a pharmaceutically acceptable salt thereof,
wherein the PARP inhibitor and one or both of the glucocorticoid, and ascorbic acid or pharmaceutically acceptable salt thereof, are comprised in the same pharmaceutical composition.

In another preferred embodiment, the kit or kit-of-parts may be provided as a package containing separately packed compositions comprising a poly-ADP-ribose polymerase (PARP) inhibitor and compositions comprising the other active agent(s). The compositions may be provided in containers, vials, syringes, ampules or the like.

The PARP inhibitors, glucocorticoids, protein growth factors and ascorbic acid as used in the present invention may be independently formulated for the same or different administration routes.

The pharmaceutical compositions contain the respective active agent(s), and optionally one or more pharmaceutically acceptable excipients and/or pharmaceutically acceptable excipients. The active agent(s) is the PARP inhibitor, glucocorticoid, protein growth factor or ascorbic acid, respectively.

A "pharmaceutically acceptable carrier" means a carrier or diluent that does not cause significant irritation to an organism and does not abrogate the biological activity and properties of the administered active agent. The carrier employed can be, for example, a solid, liquid, or gas. Examples of solid carriers include lactose, terra alba, sucrose, talc, gelatin, agar, pectin, acacia, magnesium stearate, and stearic acid. Examples of liquid carriers are sugar syrup, peanut oil, olive oil, and water. Examples of gaseous carriers include carbon dioxide and nitrogen.

A "pharmaceutically acceptable excipient" means an inert substance added to a pharmaceutical composition to further facilitate administration of a compound. Examples, without limitation, of excipients include calcium carbonate, calcium phosphate, various sugars and types of starch, cellulose derivatives, gelatin, vegetable oils and polyethylene glycols.

In a preferred embodiment of any of the above aspects of the invention, the (PARP) inhibitor is formulated for systemic, preferably oral or intravenous administration, or the (PARP) inhibitor is formulated for local administration, in particular for topical, mucosal or subcutaneous administration. For example, formulations for oral or intravenous administration of PARP inhibitors are known in the art. Moreover, the skilled person is aware of techniques for providing formulations for local administration, in particular for topical, mucosal or subcutaneous administration. For example, the PARP inhibitor may be formulated as being incorporated into a wound dressing or bandage, or as gel, semi-solid gel, cream, lotion, ointment, spray, dispersion, salve, liposomal or nanoparticulate formulation or for application by microneedles.

In another preferred embodiment of any of the above aspects of the invention, the glucocorticoid is formulated for systemic, preferably oral or intravenous administration, or the glucocorticoid is formulated for local administration, in particular for topical, mucosal or subcutaneous administration. Formulations for topical, oral and/or intravenous administration of glucocorticoids are known in the art and in medical practice. For example, the glucocorticoid may be formulated as being incorporated into a wound dressing or bandage, or as gel, semi-solid gel, cream, lotion, ointment, spray, dispersion, salve, liposomal or nanoparticulate formulation or for application by microneedles. For example, topical formulations containing glucocorticoids, e.g. cortisone, hydrocortisone or betamethasone, are available and have marketing approval and may be used according to the invention.

In another preferred embodiment of any of the above aspects of the invention, the protein growth factor is formulated for systemic, preferably oral or intravenous administration, or the protein growth factor is formulated for local administration, in particular for perilesional and/or intralesional, topical, mucosal or subcutaneous administration or topical, mucosal or subcutaneous administration. Formulations for perilesional and/or intralesional, topical, oral and/or intravenous administration of protein growth factors are known in the art. For example, the protein growth factor may be formulated as being incorporated into a wound dressing or bandage, or as gel, semi-solid gel, cream, lotion, ointment, spray, dispersion, salve, liposomal or nanoparticulate formulation or for application by microneedles. For example, topical formulations containing human PDGF are available and have marketing approval and may be used according to the invention. Further, oral formulations containing human TGF-β are available and have marketing approval and may be used according to the invention. Further, formulations containing human KGF for injections are described in the art and may be used according to the invention. For example, formulations containing human EGF are known as Heberprot-P® for perilesional and/or intralesional administration. For example, spray formulations containing human basic fibroblast growth factor are known as Trafermin or Fiblast® for topical administration.

In another preferred embodiment of any of the above aspects of the invention, ascorbic acid or a pharmaceutically acceptable salt thereof is formulated for systemic, preferably oral or intravenous administration, or ascorbic acid or a pharmaceutically acceptable salt thereof is formulated for local administration, in particular for topical, mucosal or subcutaneous administration. Pharmaceuticals, nutritional supplements or dietary supplements comprising ascorbic acid or a pharmaceutically acceptable salt thereof for oral administration are well known in the art and include powders, capsules, tablets, granulates, caplets, pills and the like. Moreover, the skilled person is aware of techniques for providing formulations comprising ascorbic acid or a pharmaceutically acceptable salt thereof for local administration, in particular for topical, mucosal or subcutaneous administration. For example, the ascorbic acid or pharmaceutically acceptable salt thereof may be formulated as being incorporated into a wound dressing or bandage, or as gel, semi-solid gel, cream, lotion, ointment, spray, dispersion, or salve, or for application by microneedles.

"Ascorbic acid" according to the present invention refers to L-(+) ascorbic acid or 1,2-Dihydroxyethyl]-3,4-dihydroxy-5-hydrofurane-2-one, and derivatives thereof which are metabolized to L-(+) ascorbic acid in the human body, such as dehydroascorbic acid (DHA) or a pharmaceutically acceptable salt thereof. A preferred ascorbic acid or pharmaceutically acceptable salt thereof according the present invention is selected from L-(+) ascorbic acid or a pharmaceutically acceptable salt thereof and dehydroascorbic acid or a pharmaceutically acceptable salt thereof, more preferably L-(+) ascorbic acid or a pharmaceutically acceptable salt thereof. Preferred pharmaceutically acceptable salts of ascorbic acid include the sodium and calcium salt.

In one preferred embodiment, ascorbic acid or pharmaceutically acceptable salt thereof may be comprised in a pharmaceutical composition comprising a PARP inhibitor, and/or may be comprised in a pharmaceutical composition comprising a glucocorticoid and/or may be comprised in a pharmaceutical composition comprising a protein growth factor.

The term "PARP inhibitor" as used herein refers to an inhibitor or antagonist of Poly(ADP-ribose) polymerases (PARP 1 and/or PARP2) activity. In a preferred embodiment, a PARP inhibitor inhibits PARP1 and optionally further inhibits PARP2. A PARP inhibitor or antagonist is a compound that selectively inhibits the activity of PARP and refers to a compound that, when administered to a subject, reduces the PARP activity within the subject.

The PARP inhibitor may be any type of compound. For example, the compound may be a small organic molecule or a biological compound such as an antibody or an enzyme. Examples of PARP inhibitors are described in Penning, Current Opinion In Drug Discovery & Development, 2010, 13 (5): 577-586. A person skilled in the art can easily determine whether a compound is capable of inhibiting PARP activity. Assays for evaluating PARP activity are for example, described in Andrabi SA et al. (2006, Proc Natl Acad Sci USA.;103(48):18308-13). PARP inhibition may be determined using conventional methods, including for example dot blots (Affar EB et al., Anal Biochem. 1998; 259(2):280-3), and BER assays that measure the direct activity of PARP to form poly ADP-ribose chains for example by using radioactive assays with tritiated substrate NAD or specific antibodies to the polymer chains formed by PARP activity (K.J. Dillon et al, Journal of Biomolecular Screening, 8(3): 347-352 (2003)).

Examples of compounds which are known PARP inhibitors and which may be used include compounds and derivatives thereof from the class of nicotinamides, benzamides, isoquinolinones, dihydroisoquinolinones, benzimidazoles, indoles, phthalazin-1(2H)-ones, quinazolinones, isoindolinones, phenanthridines, phenanthhdinones, benzopyrones, unsaturated hydroximic acid derivatives and pyridazines.

Examples of compounds which are known PARP inhibitors and which may be used in accordance with the invention include:
Nicotinamides, such as 5-methyl nicotinamide and O-(2-hydroxy-3-piperidino-propyl)-3-carboxylic acid amidoxime, and analogues and derivatives thereof.

Benzamides, including 3-substituted benzamides, such as 3-aminobenzamide, 3-hydroxybenzamide, 3-nitrosobenzamide, 3-methoxybenzamide and 3-chloroprocainamide, and 4-aminobenzamide, 1,5-di[(3- carbamoylphenyl) aminocarbonyloxy] pentane, and analogues and derivatives thereof.

Isoquinolinones and dihydroisoquinolinones, including 2H-isoquinolin-1-ones, 3H-quinazolin-4-ones, 5-substituted dihydroisoquinolinones such as 5-hydroxy dihydroisoquinolinone, 5-methyl dihydroisoquinolinone, and 5-hydroxy isoquinolinone, 5-amino isoquinolin-1-one, 5-dihydroxyisoquinolinone, 3,4 dihydroisoquinolin-1 (2H)-ones such as 3,4 dihydro-5-methoxy-isoquinolin-1(2H)-one and 3,4 dihydro-5-methyl-1 (2H)isoquinolinone, isoquinolin-1 (2H)-ones, 1,6,-naphthyridine-5(6H)-ones, 1,8-naphthalimides such as 4-amino-1,8-naphthalimide, isoquinolinone, 2,3-dihydrobenzo[de]isoquinolin-1-one, and tetracyclic lactams, including benzpyranoisoquinolinones such as benzopyrano [4,3,2-de] isoquinolinone, and analogues and derivatives thereof.

Benzimidazoles and indoles, including benzoxazole-4-carboxamides, benzimidazole-4-carboxamides, such as 2-substituted benzoxazole, 4-carboxamides, and 2-substituted benzimidazole, 4-carboxamides such as 2-aryl benzimidazole 4-carboxamides and 2-cycloalkylbenzimidazole-4-carboxamides, including 2-(4-hydroxphenyl) benzimidazole, 4-carboxamide, quinoxalinecarboxamides, imidazopyridinecarboxamides, 2-phenylindoles, 2-substituted benzoxazoles, such as 2-phenyl benzoxazole and 2-(3-methoxyphenyl) benzoxazole, 2-substituted benzimidazoles, such as 2-phenyl benzimidazole and 2-(3-methoxyphenyl) benzimidazole, azepinoindoles and azepinoindolones such as dihydrodiazapinoindolinone, 3-substituted dihydrodiazapinoindolinones, such as tetrahydrodiazapinoindolinone and 2,3, dihydro-isoindol-1-one, and analogues and derivatives thereof.

Phthalazin-1 (2H)-ones and quinazolinones, such as 4-hydroxyquinazoline, phthalazinone, 5-methoxy-4-methyl-1 phthalazinones, 4-substituted phthalazinones, 4-(1-piperazinyl)-1 (2H)-phthalazinone, and 2-substituted quinazolines, such as 8-hydroxy-2-methylquinazolin-4-(3H) one, tricyclic phthalazinones and 2-aminophthalhydrazide, and analogues and derivatives thereof.

Isoindolinones and analogues and derivatives thereof.

Phenanthridines and phenanthridinones, such as 5[H]phenanthridin-6-one, substituted 5[H] phenanthridin-6-ones, especially 2-,3-substituted 5[H] phenantridin-6-ones and sulfonamide/carbannide derivatives of 6(5H)phenanthridinones, and N-(6-oxo-5,6-dihydrophenanthridin-2-yl]-2-(N,N-dimethylannino}acetannide, and analogues and derivatives thereof.

Benzopyrones such as 1,2-benzopyrone, 6-nitrosobenzopyrone, 6-nitroso 1,2-benzopyrone, and 5-iodo-6-aminobenzopyrone, and analogues and derivatives thereof.

Unsaturated hydroximic acid derivatives such as O-(3-piperidino-2-hydroxy-1-propyl)nicotinic amidoxime, and analogues and derivatives thereof.

Pyridazines, including fused pyridazines and analogues and derivatives thereof.

Additional PARP inhibitors are described for example in WO2009/093032, WO2009/004356, WO2006/078503, WO2006/078711, WO2006/42638, WO2006/024545, WO2006/003150, WO2006/003148, WO2006/003147, WO2006/003146, WO2004/043959, WO2005/123687, WO2005/097750, WO2005/058843, WO2005/054210, WO2005/054209, WO2005/054201, US2005/054631 WO2005/012305, WO2004/108723, WO2004/105700, US2004/229895, WO2004/096793, WO2004/096779, WO2004/087713, WO2004/048339, WO2004/024694, WO2004/014873, US6,635,642, US5,587,384, WO2003/080581, WO2003/070707, WO2003/055865, WO2003/057145, WO2003/051879, US6,514,983, WO2003/007959, US6,426,415, WO2003/007959, WO2002/036599, WO2002/094790, WO2002/068407, US6,476,048, WO2001/090077, WO2001/085687, WO2001/085686, WO2001/079184, WO2001/057038, WO2001/023390, WO2001/021615, WO2001/016136, WO2001/012199, WO95/24379, Banasik et al. J. Biol. Chem., 267:3, 1569-75 (1992), Banasik et al. Molec. Cell. Biochem. 138:185-97 (1994)), Cosi (2002) Expert Opin. Ther. Patents 12 (7), and Southan & Szabo (2003) Curr Med Chem 10:321 -340.

In a preferred embodiment, the PARP inhibitor inhibits PARP1 and optionally PARP2.

In another preferred embodiment of any of the above aspects of the invention, the the PARP inhibitor is selected from veliparib, talazoparib, olaparib (AZD-2281), rucaparib, AZD-2461, iniparib, and PJ-34, or a pharmaceutically acceptable salt thereof.

Veliparib, also called ABT-888, is a PARP inhibitor which is known in the art and which is 2-((2R)-2-methylpyrrolidin-2-yl)-1H-benzimidazole-4-carboxamide. Veliparib has the following formula (I):

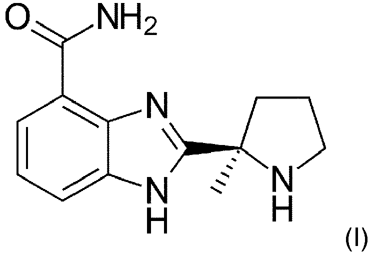

Talazoparib is a PARP inhibitor which is known in the art and which has the following formula (II): 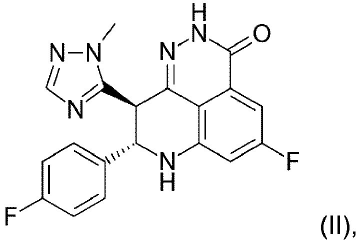 or a pharmaceutically acceptable salt thereof.

Olaparib, also called AZD-2281, is a PARP inhibitor which is known in the art, and which has the following formula (III): 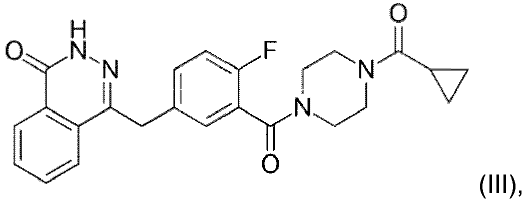 or a pharmaceutically acceptable salt thereof.

Rucaparib is a PARP inhibitor targeting PARP-1 which is known in the art and which has the following formula (IV): 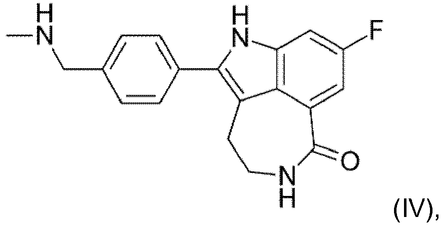 or a pharmaceutically acceptable salt thereof.

AZD-2461 is a PARP inhibitor which is known in the art and which has the following formula (V): 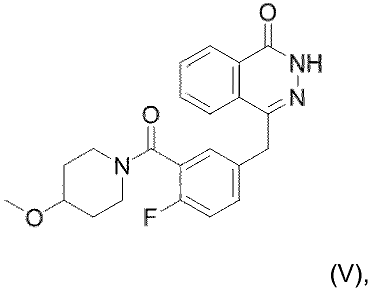 or a pharmaceutically acceptable salt thereof.

Iniparib is a PARP inhibitor which is known in the art and which is 4-lodo-3-nitrobenzamide. It has the following formula (VI): 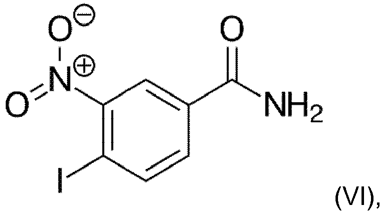 or a pharmaceutically acceptable salt thereof.

PJ-34 is a PARP inhibitor which is known in the art and which is N-(6-Oxo-5,6-dihydro-phenanthridin-2-yl)-N,N-dimethylacetamide.HCI. It has the following formula (VII): 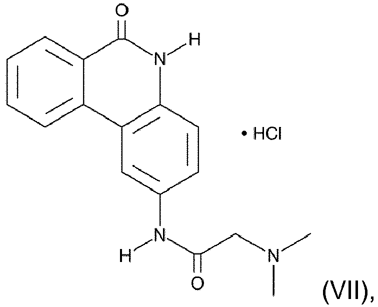 or a pharmaceutically acceptable salt thereof.

The term "pharmaceutically acceptable" is used to mean that the modified noun is appropriate for use as a pharmaceutical product or as a part of a pharmaceutical product. Pharmaceutically acceptable salts include salts commonly used to form alkali metal salts and to form addition salts of free acids or free bases. In general, these salts typically may be prepared by conventional means by reacting, for example, the appropriate acid or base with a compound used in the invention.

Pharmaceutically acceptable acid addition salts can be prepared from an inorganic or organic acid. Examples of often suitable inorganic acids include hydrochloric, hydrobromic, hydroiodic, nitric, carbonic, sulfuric, and phosphoric acid. Suitable organic acids generally include, for example, aliphatic, cycloaliphatic, aromatic, araliphatic, heterocyclic, carboxylic, and sulfonic classes of organic acids. Specific examples of often suitable organic acids include acetate, trifluoroacetate, formate, propionate, succinate, glycolate, gluconate, digluconate, lactate, malate, tartaric acid, citrate, ascorbate, glucuronate, maleate, fumarate, pyruvate, aspartate, glutamate, benzoate, anthranilic acid, mesylate, stearate, salicylate, p-hydroxybenzoate, phenylacetate, mandelate, embonate (pamoate), ethanesulfonate, benzenesulfonate, pantothenate, 2-hydroxyethanesulfonate, sulfanilate, cyclohexylaminosulfonate, algenic acid, beta-hydroxybutyric acid, galactarate, galacturonate, adipate, alginate, bisulfate, butyrate, camphorate, camphorsulfonate, cyclopentanepropionate, dodecylsulfate, glycoheptanoate, glycerophosphate, heptanoate, hexanoate, nicotinate, oxalate, palmoate, pectinate, 2-naphthalenesulfonate, 3-phenylpropionate, picrate, pivalate, thiocyanate, tosylate, and undecanoate.

Pharmaceutically acceptable base addition salts include, for example, metallic salts and organic salts. Preferred metallic salts include alkali metal (group la) salts, alkaline earth metal (group IIa) salts, and other physiologically acceptable metal salts. Such salts may be made from aluminum, calcium, lithium, magnesium, potassium, sodium, and zinc. Preferred organic salts can be made from amines, such as tromethamine, diethylamine, N,N'-dibenzylethylenediamine, chloroprocaine, choline, diethanolamine, ethylenediamine, meglumine (N-methylglucamine), and procaine. Basic nitrogen-containing groups can be quatemized with agents such as lower alkyl (C]-C6) halides (e.g., methyl, ethyl, propyl, and butyl chlorides, bromides, and iodides), dialkyl sulfates (e.g., dimethyl, diethyl, dibutyl, and diamyl sulfates), long chain halides (e.g., decyl, lauryl, myristyl, and stearyl chlorides, bromides, and iodides), arylalkyl halides (e.g., benzyl and phenethyl bromides), and others.

The PARP inhibitors are administered to a subject in a therapeutically effective amount. For systemic applications, the PARP inhibitor dose will be in the range of about 10 to 1000 mg/day, depending on the PARP inhibitor. Topical formulations of PARP inhibitors may be administered in a concentration of about 0,00001 to 10 % (w/v), about 0,00001 to 6 % (w/v) or about 0,00001 to 1 % (w/v), such as 0,0001 to 0,1% (w/v), such as a cream, gel, lotion, ointment, liposomal or nanoparticulate formulation or the like 0.001 to 1% (w/v).

Glucocorticoids (GCs) are a class of corticosteroids. Glucocorticoids are corticosteroids that bind to the glucocorticoid receptor (GR). Glucocorticoids are well known in the art and in medical practice. Numerous glucocorticoids have marketing approval for the treatment of diseases and are widely used in medical practice. Suitable glucocorticoids include cortisol, cortisone acetate, prednisone, prednisolone, methylprednisolone, chloroprednisone, cloprednol, difluprednate, fludrocortisone acetate, fluocinolone, fluperolone, fluprednisolone, loteprednol, prednicarbate, tixocortol, triamcinolone, triamcinolone acetonide, dexamethasone, betamethasone, beclometasone, deoxycorticosterone acetate, alclometasone, clobetasol, clobetasone, clocortolone, desoximetasone, diflorasone, difluocortolone, fluclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, budesonide, ciclesonide, deflazacort, desonide, formocortal, fluclorolone acetonide, fludroxycortide, flunisolide, fluocinolone acetonide, fluocinonide, halcinonide, hydroxymethylprogesterone, and medroxyprogesterone and pharmaceutically acceptable salts thereof.

Therefore, in yet another preferred embodiment of any of the above aspects of the invention, the glucocorticoid is selected from the group consisting of cortisol, cortisone acetate, prednisone, prednisolone, methylprednisolone, chloroprednisone, cloprednol, difluprednate, fludrocortisone acetate, fluocinolone, fluperolone, fluprednisolone, loteprednol, prednicarbate, tixocortol, triamcinolone, triamcinolone acetonide, dexamethasone, betamethasone, beclometasone, deoxycorticosterone acetate, alclometasone, clobetasol, clobetasone, clocortolone, desoximetasone, diflorasone, difluocortolone, fluclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, budesonide, ciclesonide, deflazacort, desonide, formocortal, fluclorolone acetonide, fludroxycortide, flunisolide, fluocinolone acetonide, fluocinonide, halcinonide, hydroxymethylprogesterone, and medroxyprogesterone or a pharmaceutically acceptable salt thereof.

Typically, for systemic applications, the glucocorticoid dose will be in the range of about 0,1 to 1000 mg/day, depending on the glucocorticoid. Topical formulations of glucocorticoids are typically administered in a concentration of 0,001 to 10 % (w/v), about 0,001 to 6 % (w/v) or about 0,001 to 1 % (w/v), such as 0,01 to 0,1% (w/v), such as a cream, gel, lotion, ointment, liposomal or nanoparticulate formulation or the like.

A protein growth factor is a protein which exhibits an enhancing and/or stimulatory effect on the proliferation of at least one cell type present in the skin of an animal. In a preferred embodiment, the protein growth factor does not cause cancer when administered to a subject, in particular when administered topically to a subject and/or the protein growth factor is suitable for administration to a subject for therapeutic and/or preventive purposes. In a preferred embodiment, the protein growth factor is a human protein growth factor. In yet another preferred embodiment, the protein growth factor is a recombinant protein growth factor, in particular recombinant protein growth factor. In a more preferred embodiment, the protein growth factor is selected from a platelet derived growth factor (PDGF), transforming growth factor beta (TGF-β), basic fibroblast growth factor (bFGF), keratinocyte growth factor (KGF), epidermal growth factor (EGF), Insulin-like growth factor 1 (IGF-1), vascular endothelial growth factor (VEGF) and (hepatocyte growth factor) HGF. In an even more preferred embodiment, the protein growth factor is selected from a platelet derived growth factor (PDGF), transforming growth factor beta (TGF-β), and basic fibroblast growth factor (bFGF), most preferably the protein growth factor is PDGF, in particular becaplermin. A PDGF is preferably selected from a PDGF containing a homodimer or heterodimer of the group selected from PDGF-A, PDGF-B, PDGF-C and PDGF-D, more preferably selected from PDGF-A and PDGF-B. In an even more preferred embodiment, the PDGF is the homodimer of the B chain of platelet-derived growth factor, designated PDGF-BB or becaplermin. In a preferred embodiment, KGF is KGF-2, in particular human KGF-2. In another preferred embodiment, the protein growth factor is human epidermal growth factor (EGF), in particular recombinant human EGF (rhEGF).

Further, it was surprisingly found that the assays based on fibroblast proliferation as described in Example 1.1 and fibroblast-derived matrix formation as described in Example 1.2 surprisingly allow for the identification of subjects suffering from impaired skin wound healing which are responsive to a treatment and/or prevention with a combination of a PARP inhibitor and one, two or three of a glucocorticoid, ascorbic acid or a pharmaceutically acceptable salt thereof and a protein growth factor.

Moreover, it was surprisingly found that the assays based on fibroblast proliferation as described in Example 1.1 and fibroblast-derived matrix formation as described in Example 1.2 surprisingly allow for the identification of subjects suffering from impaired skin wound healing which are responsive to a treatment and/or prevention with a PARP inhibitor wherein the subject: (i) is a subject treated with at least one glucocorticoid, and/or (ii) is a subject to which a pharmaceutical, nutritional supplement or dietary supplement comprising ascorbic acid or a pharmaceutically acceptable salt thereof is administered, and/or (iii) is a subject treated with at least one protein growth factor.

The assays may be used for successful stratification and identification of subjects suffering from impaired skin wound healing.

Therefore, in a preferred embodiment of any of the above aspects of the invention, the subject is identified to be responsive to the treatment of impaired skin wound healing by performing steps i) and/or ii):
i) measuring the proliferation of primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid
      (ii) ascorbic acid or a pharmaceutically acceptable salt thereof
      (iii) a protein growth factor.

In one preferred embodiment of the present invention, the sample is a wound exudate sample. In another preferred embodiment, the sample is a wound biofilm sample. In a more preferred embodiment, the sample is a wound exudate sample.

In a more preferred embodiment, the subject is identified to be responsive to the treatment of impaired skin wound healing, in case the value of proliferation of primary fibroblast cells measured in step i) and/or the value of the fibroblast-derived matrix formation by primary fibroblast cells measured in step ii) is at least 20% above a control value established in the absence of the compounds of (2).

In one preferred embodiment, the compounds of (2) are a PARP inhibitor and a glucocorticoid. In such embodiment, the subject is identified to be responsive to the treatment of impaired skin wound healing with a PARP inhibitor and a glucocorticoid in case the value of proliferation of primary fibroblast cells measured in step i) and/or the value of the fibroblast-derived matrix formation by primary fibroblast cells measured in step ii) in the presence of the PARP inhibitor and glucocorticoid is at least 20% above a control value established in the absence of the compounds.

The glucocorticoid to be administered to the subject in case the subject is identified to be responsive may be the same glucocorticoid or a different glucocorticoid, preferably the same glucocorticoid.

In another preferred embodiment, the compounds of (2) are a PARP inhibitor and ascorbic acid or a pharmaceutically acceptable salt thereof. In such embodiment, the subject is identified to be responsive to the treatment of impaired skin wound healing with a PARP inhibitor and ascorbic acid or a pharmaceutically acceptable salt thereof, in case the value of proliferation of primary fibroblast cells measured in step i) and/or the value of the fibroblast-derived matrix formation by primary fibroblast cells measured in step ii) in the presence of the PARP inhibitor and ascorbic acid or a pharmaceutically acceptable salt thereof is at least 20% above a control value established in the absence of the compounds.

In one preferred embodiment, the compounds of (2) are a PARP inhibitor and a protein growth factor. In such embodiment, the subject is identified to be responsive to the treatment of impaired skin wound healing with a PARP inhibitor and a protein growth factor, in case the value of proliferation of primary fibroblast cells measured in step i) and/or the value of the fibroblast-derived matrix formation by primary fibroblast cells measured in step ii) in the presence of the PARP inhibitor and protein growth factor is at least 20% above a control value established in the absence of the compounds.

The protein growth factor to be administered to the subject in case the subject is identified to be responsive may be the same protein growth factor or a different protein growth factor, preferably the same protein growth factor.

In yet another preferred embodiment, the compounds of (2) are a PARP inhibitor and two or three of a glucocorticoid, protein growth factor and ascorbic acid or a pharmaceutically acceptable salt thereof. In such embodiment, the subject is identified to be responsive to the treatment of impaired skin wound healing with a PARP inhibitor and two or three of ascorbic acid or a pharmaceutically acceptable salt thereof, a protein growth factor and a glucocorticoid, in case the value of proliferation of primary fibroblast cells measured in step i) and/or the value of the fibroblast-derived matrix formation by primary fibroblast cells measured in step ii) in the presence of the PARP inhibitor and two or three of ascorbic acid or a pharmaceutically acceptable salt thereof, a protein growth and a glucocorticoid is at least 20% above a control value established in the absence of the compounds.

The glucocorticoid to be administered to the subject in case the subject is identified to be responsive may be the same glucocorticoid or a different glucocorticoid, preferably the same glucocorticoid.

The protein growth factor to be administered to the subject in case the subject is identified to be responsive may be the same protein growth factor or a different protein growth factor, preferably the same protein growth factor.

Measuring the proliferation of primary fibroblast cells in the presence of a wound exudate sample, or wound biofilm sample, obtained from said skin wound and the compounds of (2) may be performed as shown in the examples, in particular in Example 1.1. The assay is also referred to as "HDF proliferation", "human dermal fibroblast proliferation", "fibroblast proliferation" or "2D fibroblast proliferation" assay in the present application. For the assay, primary fibroblast cells are used, which may be primary mammal dermal fibroblasts, preferably primary human dermal fibroblasts. Methods for culturing primary human dermal fibroblast cells are known in the art and are for example described in the examples. For example, the cells may be cultured using DMEM medium containing FCS. In a further preferred embodiment, the cells are incubated on a solid support, thereby allowing the cells to adhere to the support, as for example described in the Examples, where multiwell plates were used. Further, the cells are contacted with the wound exudate sample, or wound biofilm sample, which is optionally diluted, e.g. diluted with medium or a saline aqueous liquid, and the compounds of (2). The contacting may be performed before or after adherence of the cells occurs. For example, the contacting may be achieved by adding the optionally diluted, liquid wound exudate sample, or wound biofilm sample, and the compounds of (2) to the cells either prior to adherence, for example at the seeding of the cells, or after adherence. The contacting may be achieved e.g. by pipetting, and optionally gentle mixing. The cells are incubated for an appropriate time, such as for 6 hours to 300 hours, more preferably 12 hours to 200 hours, even more preferably 24 hours to 120 hours. In the examples, 72 hours were successfully used. For negative control samples, a corresponding liquid in the absence of the compounds of (2) may be added in addition to wound exudate, or wound biofilm, or only wound exudate, or only wound biofilm, is added. Subsequently, the amount, preferably the cell number, including the formation of extracellular matrix, of the primary fibroblast cells is determined, such as by fixing cells and determining total protein content. The cells may for example be fixed using paraformaldehyde. Further, a suitable dye, such as sulforhodamine B may be used for determining the amount, preferably the cell number, including the formation of extracellular matrix, of the primary fibroblast cells. The stained cells including the extracellular matrix formed may then be quantified e.g. by determining absorbance or fluorescence at a suitable wavelength, depending on the dye. Preferably, the steps are performed in 2D cell culture, which allows for culturing the cells adherently on a solid support. Preferably, the sample is a wound exudate sample.

Preferably, the method step includes the following steps:
(i) culturing primary human dermal fibroblast cells,
(ii) incubating the cells on a solid support, thereby allowing the cells to adhere to the support,
(iii) contacting the cells with (1) the wound exudate sample or wound biofilm sample, which is optionally diluted, and the compounds of (2), wherein the contacting may be performed before or after adherence of the cells occurs, and wherein the contacting of (1) and (2) may be performed simultaneously or sequentially, and
(iv) determining the amount, preferably the cell number, including the formation of extracellular matrix, of the primary fibroblast cells, such as by fixing cells and determining total protein content,
preferably wherein the method is performed in 2D cell culture.

In one preferred embodiment of the present invention, the sample is a wound exudate sample. In another preferred embodiment, the sample is a wound biofilm sample. In a more preferred embodiment, the sample is a wound exudate sample.

The culturing of cells is preferably performed at about 20°C to 40°C, more preferably 25°C to 38°C, even more preferably at about 37°C.

Measuring the fibroblast-derived matrix formation by primary fibroblast cells in the presence of a wound exudate sample or wound biofilm sample obtained from a skin wound may be performed as shown in the examples, in particular in Example 1.2. The assay is also referred to as "ECM formation", "fibroblast-derived matrix", or "3D fibroblast derived matrix" assay in the present application. For the assay, primary fibroblast cells are used, which may be primary mammal dermal fibroblasts, preferably primary human dermal fibroblasts. In the examples, primary human dermal fibroblast cells are seeded on a support, which is preferably pre-coated with an adhesion enhancing agent, such as gelatin. For example, the coating may be achieved by incubating the support with a solution or suspension containing the adhesion enhancing agent, such as gelatin. In the examples, a 0,2% gelatin solution was successfully used. Preferably, the cells are cultured until confluence is reached. Subsequently, the cells are contacted with (i) a matrix promoting supplement, (ii) the wound exudate sample, or wound biofilm sample, which is optionally diluted, and (iii) the compounds of (2), wherein (i), (ii) and (ii) may be contacted simultaneously or sequentially. For example, the matrix promoting supplement, which is preferably selected from a solution comprising Vitamin C or a physiologically acceptable salt thereof, such the sodium salt, or 2-phospho-L-ascorbic acid or a physiologically acceptable salt thereof, and a combination of EGF and insulin, is added to the cells, e.g. by pipetting, and optionally gentle mixing. The wound exudate sample, or wound biofilm sample, which is optionally diluted, may be contacted simultaneously or sequentially and the compounds of (2) are added simultaneously or sequentially. For example, the optionally diluted wound exudate sample or wound biofilm sample may be mixed with the matrix promoting supplement, and the mixture may be added to the cells, and the compounds of (2) are added subsequently. Alternatively, the optionally diluted wound exudate sample or wound biofilm sample may be added separately, but simultaneously, or separately, but subsequent to or prior to the matrix promoting supplement and/or the compounds of (2). In case of subsequent non-simultaneous contacting, the components (i), (ii) and (iii) are preferably contacted within 1 hour. The cells are subsequently incubated, preferably for 12 hours to 20 days, wherein the medium is optionally replaced at least one time with fresh medium supplemented with optionally diluted wound exudate, or wound biofilm, and matrix promoting supplement. In the example, the medium was replaced once after 4 days of incubation, and the total incubation was 8 days. As a 3-dimensional fibroblast-derived matrix is formed, the solid support preferably contains at least one cavity which allows for filling of the space and therefore allows for a 3D cell culture. Subsequently, the amount of the fibroblast-derived matrix is determined, such as by fixing cells and determining total protein content. The cells may for example be fixed using paraformaldehyde. Further, a suitable dye, such as sulforhodamine B may be used for determining the amount, preferably the cell number, including the formation of extracellular matrix, of the primary fibroblast cells. The stained cells including the formation of extracellular matrix may then be quantified e.g. by determining absorbance or fluorescence at a suitable wavelength, depending on the dye. For negative control samples, a corresponding liquid in the absence of the compounds of (2) may be added in addition to wound exudate, or wound biofilm, or only wound exudate or wound biofilm is added.

Accordingly, the method step preferably includes the following steps:
(i) seeding primary human dermal fibroblast cells on a support, which is preferably pre-coated with an adhesion enhancing agent, such as gelatin,
(ii) culturing the cells on the support, preferably until confluence is reached,
(iii) contacting the cells with (i) a matrix promoting supplement, (ii) the wound exudate sample, or wound biofilm sample, which is optionally diluted, and (iii) the compounds of (2), wherein (i) and (ii) may be contacted simultaneously or sequentially,
(iv) determining the amount of the fibroblast-derived matrix, such as by fixing cells and determining total protein content,
preferably wherein the method is performed in 3D cell culture.

In one preferred embodiment of the present invention, the sample is a wound exudate sample. In another preferred embodiment, the sample is a wound biofilm sample. In a more preferred embodiment, the sample is a wound exudate sample.

The "fibroblast-derived matrix" or "FDM" is understood as the extracellular matrix (ECM) formed by living fibroblast cells in an environment conducive for matrix formation, e.g. in the presence of a matrix promoting supplement. FDM is obtainable as described in the examples. In particular, FDM is obtainable by (i) seeding primary human dermal fibroblast cells on a support, which is pre-coated with an adhesion enhancing agent, such as gelatin, (ii) culturing the cells on the support, preferably until confluence is reached and (iii) contacting the cells with a matrix promoting supplement, such as Vitamin C or a physiologically acceptable salt thereof, or 2-phospho-L-ascorbic acid or a physiologically acceptable salt thereof, or a combination of EGF and insulin.

A "matrix promoting supplement" is understood as a compound or composition which promotes the formation of fibroblast-derived matrix by living fibroblast cells in an *in vitro* cell culture. Suitable matrix promoting supplements are Vitamin C or a physiologically acceptable salt thereof, such the sodium salt, or 2-phospho-L-ascorbic acid or a physiologically acceptable salt thereof, and a combination of EGF and insulin, as well as compositions comprising the compounds, such as solutions or suspensions. A combination of EGF and insulin may be provided to the cell culture separately, e.g. as separate solutions comprising EGF or insulin respectively, or together, e.g. as solution comprising EGF and insulin.

An "adhesion enhancing agent" is an agent which enhances adhesion of cells to a solid support, such as a plastic support, but which does not substantially interfere with the viability of the cells. In a preferred embodiment, the adhesion enhancing agent is gelatin or fibronectin, more preferably gelatin.

"2D cell culture" is understood as a cell culture wherein the cells are cultured in a planar or substantially planar surface. In a preferred embodiment, the 2D cell culture is culturing of adherent cells.

"3D cell culture" is understood as a cell culture wherein the cells are cultured on a non-planar or substantially non-planar surface. In a preferred embodiment, the 3D cell culture is culturing of adherent cells and/or culturing of cells within a matrix, such as ECM, in particular FDM.

A "support" or "solid support" is preferably selected from a chip, array, such as a microarray or nanoarray, a plate, such as a multiwell plate, or a dish. For cell culture applications, the solid support is preferably suitable for culturing cells, for example the support may be a plastic support.

"Wound exudate" is understood as the extracellular fluid located within and above a skin wound. The wound exudate is also referred to a "liquid biopsy".

"Wound biofilm" is understood as substance, resulting from an infection of a skin wound by micro-organisms that are capable of forming colonies. Typically, the wound biofilm is a gummy or gum-like substance. A wound biofilm comprises microbial species selected from bacteria, fungi, yeasts, algae and other micro-organisms, and cellular debris. A wound biofilm is formed when certain types of micro-organisms attach themselves to the surface of skin wounds by secreting a gummy or gum-like substance. For example, a wound biofilm sample may be obtained by surgical sharp debridement of the wound surface or by wiping of the wound surface with a swab, such as a cotton swab or nylon-flocked swab, or wound dressing material.

A "wound exudate sample" or "WE" is understood as a sample of wound exudate obtained from a skin wound of an individual. Methods for obtaining a wound exudate sample are known in the art. For example, a wound exudate sample may be obtained by a physical or chemical method, in particular by applying negative pressure to the skin wound, such as by using a negative pressure drainage device, a method using capillary forces, collecting wound exudate in a film dressing or membrane, collecting wound exudate in a syringe, applying an absorptive material, such as absorptive beads, or a filter, or by using a swab, such as a cotton swab or nylon-flocked swab, in particular wherein the film dressing or membrane is a cellulose layer and/or wherein the absorptive material is a cellulose layer. Preferred suitable cellulose layers are nanocellulose layers, such as nanocellulose layers. The volume of wound exudate sample may vary and may be in the range of 1 nl to 1 I, 10 nl to 10 1 I, or 100 nl to 1 I, such as 1 µl to 1 l, 1 ml to 1 l or 10 ml to 1 l. For example, wound exudate samples investigated in the examples had a volume of up to 400 ml and typically had a volume of 0,1 to 100 ml, in particular 10 to 50 ml. The wound exudate sample may be used the methods of the invention directly after obtaining the sample or may be stored, in particular stored at <4°C, <0°C or <10°C, such as about -20°C, before usage in the methods of the invention.

The nanocellulose layer which can be used according to the invention may be a nanocellulose membrane or dressing, which is optionally covered, and which may have e.g. a disc-like form. Accordingly, the cellulose layer or nanocellulose layer is in one preferred embodiment a cellulose disc or nanocellulose disc. Typically, the nanocellulose surface area brought into contact with wound exudate is in the range of about 1 cm² to about 100 cm².

A "wound biofilm sample" or "WB" is understood as a sample of wound biofilm obtained from a skin wound of an individual. Methods for obtaining a wound biofilm sample are known in the art. For example, a wound biofilm sample may be obtained by surgical sharp debridement or by wiping of the wound surface with a swab, such as a cotton swab or nylon-flocked swab, or wound dressing material. The volume of wound biofilm sample may vary and may be in the range of 1 nl to 1 l, 10 nl to 1 l, or 100 nl to 1 I, such as 1 µl to 1 l, 1 ml to 1 I or 10 ml to 1 I. The wet weight of wound biofilm may vary and may be in the range of 10 µg to 10 g, 100 µg to 10 g, such as 1 mg to 10 g, 10 mg to 10 g, 100 mg to 10 g, or 1 g to 10 g. The wound biofilm sample may be used the methods of the invention directly after obtaining the sample or may be stored, in particular stored at <4°C, <0°C or <10°C before usage in the methods of the invention. The wound biofilm sample can be extracted with a suitable liquid, such as cell culture medium or buffer, in particular with liquid of 5 to 10 times of the weight of the sample.

It was surprisingly found that the above assays relating to measuring the proliferation of primary fibroblast cells and the fibroblast-derived matrix formation by primary fibroblast cells can reliably identify subjects responsive to a treatment and/or prevention of impaired skin wound healing of any of the above embodiments of the invention.

Moreover, it was found that the accuracy of the identification of responsive subjects is improved in case both measuring the proliferation of primary fibroblast cells and the fibroblast-derived matrix formation by primary fibroblast cells is performed. Accordingly, in a more preferred embodiment, the subject is identified to be responsive to the treatment of impaired skin wound healing in case the value of proliferation of primary fibroblast cells measured in step i) and the value of the fibroblast-derived matrix formation by primary fibroblast cells measured in step ii) is at least 20% above a control value established in the absence of the compounds of (2).

Further, the accuracy of the identification of responsive subjects is improved in case the measured values are clearly increased vis-à-vis the respective control value established in the absence of the compounds of (2).

Accordingly, in a yet further preferred embodiment, the subject is identified to be responsive to the treatment of impaired skin wound healing in case the value of proliferation of primary fibroblast cells measured in step i) and/or the value of the fibroblast-derived matrix formation by primary fibroblast cells measured in step ii) is at least 30%, 40%, 50%, 60%, 70%, 80%, 100% or more above a control value established in the absence of the compounds of (2).

The control value(s) may be determined in parallel or may be established independently, preferably in parallel.

In a further aspect, the present invention relates to an *in vitro* method of identifying a subject suffering from impaired skin wound healing to be responsive to the treatment with a poly-ADP-ribose polymerase (PARP) inhibitor in combination with one, two or three of (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
comprising performing steps i) and/or ii):
i) measuring the proliferation of primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor;
wherein the subject is identified to be responsive to the treatment of impaired skin wound healing with the combination in case the value of proliferation of primary fibroblast cells measured in step i) and/or the value of the fibroblast-derived matrix formation by primary fibroblast cells measured in step ii) is at least 20% above a control value established in the absence of the compounds of (2).

In one preferred embodiment of the present invention, the sample is a wound exudate sample. In another preferred embodiment, the sample is a wound biofilm sample. In a more preferred embodiment, the sample is a wound exudate sample.

For the in vitro method, the same preferred embodiments apply as described for the preferred embodiments wherein the subject is identified to be responsive to the treatment of impaired skin wound healing by performing steps i) and/or ii).

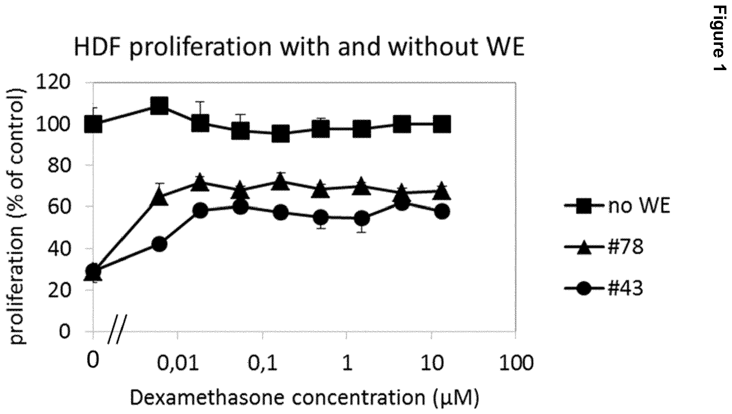

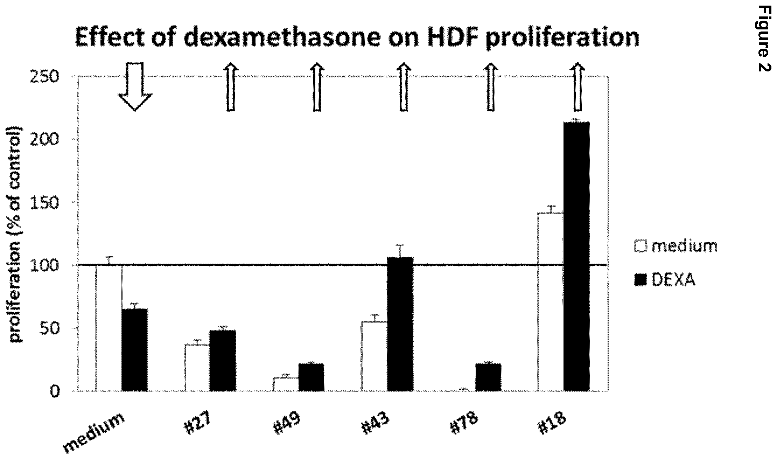

Moreover, it was surprisingly found in the present application that the glucocorticoid dexamethasone dose-dependently relieved wound exudate (WE)-induced inhibition of HDF (human dermal fibroblast) proliferation (see Example 1.1 and Figures 1, 2). Qualitatively identical results were obtained with 8 other glucocorticoid receptor agonists, namely hydrocortisone, prednisolone, medroxyprogesterone, beclomethasone, loteprednol, fluticasone, halomethasone, prelone (data not shown). Moreover, the effects of the glucocorticoids were reversible by the antagonist mifepristone, indicating a glucocorticoid-specific effect. Glucocorticoids are described in the prior art to impair wound healing. In accordance with this prior art knowledge, dexamethasone reduces fibroblast proliferation in normal medium (Figure 16). It was now surprisingly found that, in the presence of WE of specific patients, dexamethasone as well as other glucocorticoids enhance proliferation, indicating a wound healing promoting effect.

Therefore, the present examples show that dexamethasone inhibits both HDF proliferation and extracellular matrix (ECM) formation, as determined pursuant to Examples 1.1 and 1.2, in the absence of WE, whereas in the presence of WE, dexamethasone, as well as medroxyprogesterone, surprisingly enhances HDF proliferation and ECM formation.

Therefore, it was surprisingly found that, while glucocorticoids do exert a negative effect on wound healing in most patients as described in the prior art as "dogma", the present assays of determining HDF proliferation and FDM formation as described in Examples 1.1 and 1.2 allow for the stratification for those patients who are responsive to a treatment of impaired skin wound healing with a glucocorticoid. In particular, a patient can be identified to be responsive to a treatment with a glucocorticoid, in case a glucocorticoid exerts a positive, enhancing effect in the HDF proliferation assay and/or ECM formation assay. Optionally, a PARP inhibitor and/or Vitamin C, and/or a protein growth factor may further be included in the test.

Therefore, the present disclosure relates to an *in vitro* method of identifying a subject suffering from impaired skin wound healing to be responsive to the treatment with a glucocorticoid, optionally in combination with one, two or three of (i) to (iii):
(i) a pharmaceutical composition comprising a poly-ADP-ribose polymerase (PARP) inhibitor,
(ii) ascorbic acid or a pharmaceutically acceptable salt thereof,
(iii) a pharmaceutical composition comprising a protein growth factor;
comprising performing steps i) and/or ii):
i) measuring the proliferation of primary fibroblast cells in the presence of:
   (1) a wound exudate sample or wound biofilm sample obtained from the skin wound of said subject, and
   (2) the following compound(s): a glucocorticoid and optionally one, two, or three of (i) to (iii):
      (i) a PARP inhibitor,
      (ii) ascorbic acid or a pharmaceutically acceptable salt thereof,
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compound(s): a glucocorticoid and optionally one, two or three of (i) to (iii):
      (i) a PARP inhibitor,
      (ii) ascorbic acid or a pharmaceutically acceptable salt thereof,
      (iii) a protein growth factor;
wherein the subject is identified to be responsive to the treatment of impaired skin wound healing in case the value of proliferation of primary fibroblast cells measured in step i) and/or the value of the fibroblast-derived matrix formation by primary fibroblast cells measured in step ii) is at least 20% above a control value established in the absence of the compound(s) of (2).

In one preferred disclosure, the sample is a wound exudate sample. In another preferred disclosure , the sample is a wound biofilm sample. In a more preferred disclosure, the sample is a wound exudate sample.

In a preferred disclosure, the compounds of (2) are a glucocorticoid and optionally one or both of (i) and (ii):
(i) a PARP inhibitor,
(ii) ascorbic acid or a pharmaceutically acceptable salt thereof.

In a further aspect, the present disclosure relates to a glucocorticoid or a pharmaceutically acceptable salt thereof for use of in the treatment of impaired skin wound healing in a subject, wherein the subject is identified to be responsive to the treatment of impaired skin wound healing by performing steps i) and/or ii):
i) measuring the proliferation of primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof;
ii) measuring the fibroblast-derived matrix formation by primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof,
wherein the subject is identified to be responsive to the treatment with the glucocorticoid or a pharmaceutically acceptable salt thereof, in case the value of proliferation of primary fibroblast cells measured in step i) and/or the value of the fibroblast-derived matrix formation by primary fibroblast cells measured in step ii) is at least 20% above a control value established in the absence of the glucocorticoid or pharmaceutically acceptable salt thereof.

In one preferred disclosure, the sample is a wound exudate sample. In another preferred disclosure, the sample is a wound biofilm sample. In a more preferred embodiment, the sample is a wound exudate sample.

For the *in vitro* method of identifying a subject suffering from impaired skin wound healing to be responsive to the treatment with a glucocorticoid, and the glucocorticoid or a pharmaceutically acceptable salt thereof for use of in the treatment of impaired skin wound healing in a subject, the same preferred disclosure applies as described for the other aspects and embodiments of the invention, to the extent these embodiments can be reasonably applied. In particular, the preferred disclosure described above for the fibroblast proliferation assay and fibroblast-derived matrix formation assay, as well as for the glucocorticoids and their administration are understood to also apply these aspects of the present disclosure.

The present disclosure further relates to a method of preventing or treating impaired skin wound healing in a subject, comprising administering to a subject in need thereof a therapeutically effective amount of a glucocorticoid or a pharmaceutically acceptable salt thereof, wherein the subject is identified to be responsive to the treatment of impaired skin wound healing by performing steps i) and/or ii):
i) measuring the proliferation of primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof;
ii) measuring the fibroblast-derived matrix formation by primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof,
and identifying the subject to be responsive to the treatment with the glucocorticoid or a pharmaceutically acceptable salt thereof, in case the value of proliferation of primary fibroblast cells measured in step i) and/or the value of the fibroblast-derived matrix formation by primary fibroblast cells measured in step ii) is at least 20% above a control value established in the absence of the glucocorticoid or pharmaceutically acceptable salt thereof.

In one preferred disclosure, the sample is a wound exudate sample. In another preferred disclosure, the sample is a wound biofilm sample. In a more preferred disclosure, the sample is a wound exudate sample.

The present disclosure relates to a method of preventing or treating impaired skin wound healing in a subject, comprising administering to a subject in need thereof a therapeutically effective amount of a poly-ADP-ribose polymerase (PARP) inhibitor and one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor.

The present disclosure further relates to a method of preventing or treating impaired skin wound healing in a subject, comprising administering to a subject in need thereof a therapeutically effective amount of a poly-ADP-ribose polymerase (PARP) inhibitor, wherein the subject:
(i) is a subject treated with at least one glucocorticoid, and/or
(ii) is a subject to which a pharmaceutical, nutritional supplement or dietary supplement comprising ascorbic acid or a pharmaceutically acceptable salt thereof is administered, and/or
(iii) is a subject treated with at least one protein growth factor.

The present disclosure further relates a method of preventing or treating impaired skin wound healing in a subject, comprising administering to a subject in need thereof a therapeutically effective amount of a poly-ADP-ribose polymerase (PARP) inhibitor and one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor;
wherein the subject is identified to be responsive to said PARP inhibitor and one, two or three of (i) to (iii) by performing steps i) and/or ii):
i) measuring the proliferation of primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid
      (ii) ascorbic acid or a pharmaceutically acceptable salt thereof,
      (iii) a protein growth factor;
and identifying the subject to be responsive to the treatment of impaired skin wound healing in case the value of proliferation of primary fibroblast cells measured in step i) and/or the value of the fibroblast-derived matrix formation by primary fibroblast cells measured in step ii) is at least 20% above a control value established in the absence of the compounds of (2).

In one preferred disclosure, the sample is a wound exudate sample. In another preferred disclosure, the sample is a wound biofilm sample. In a more preferred disclosure, the sample is a wound exudate sample.

"Effective amount" refers to the amount sufficient to induce a desired biological, pharmacological, or therapeutic outcome in a subject. A therapeutically effective amount of a compound can be employed as a zwitterion or as a pharmaceutically acceptable salt. A therapeutically effective amount means a sufficient amount of the compound to treat or prevent impaired skin wound healing at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed, the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than those required to achieve the desired therapeutic effect and to gradually increase the dosage until the desired effect is achieved.

The accuracy and reliability of the *in vitro* methods of the present invention as well as the uses of the present invention wherein responsiveness is determined based on the cellular assays described above can be further increased by including a further assay in the method, which measures the proliferation of keratinocyte cells, such as primary keratinocyte cells or HaCaT cells. The use of HaCaT cells is to be more preferred as compared to primary keratinocytes and allows for reliable prediction, in combination with the fibroblast-based assays described above.

Therefore, in another preferred embodiment of an embodiment of any of the above aspects of the invention, the subject is identified to be responsive to the treatment of impaired skin wound healing with a combination of the invention or a PARP inhibitor as described above, by performing steps i) and/or ii) and/or iiia):
i) measuring the proliferation of primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor,
iiia) measuring the proliferation of keratinocyte cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor.

In one preferred embodiment of the present invention, the sample is a wound exudate sample. In another preferred embodiment, the sample is a wound biofilm sample. In a more preferred embodiment, the sample is a wound exudate sample.

In a more preferred embodiment, the subject is identified to be responsive to the treatment of impaired skin wound healing with a combination of the invention or a PARP inhibitor as described above, in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compounds of (2).

In a further aspect, the present invention relates to an *in vitro* method of identifying a subject suffering from impaired skin wound healing to be responsive to the treatment with a poly-ADP-ribose polymerase (PARP) inhibitor in combination with one, two or three of (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
comprising performing steps i) and/or ii) and/or iiia):
i) measuring the proliferation of primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid,
      (ii) ascorbic acid or a pharmaceutically acceptable salt thereof,
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compounds: a PARP inhibitor and one, two or three of (i) to (iii):
      (i) a glucocorticoid
      (ii) ascorbic acid or a pharmaceutically acceptable salt thereof,
      (iii) a protein growth factor;
iiia) measuring the proliferation of keratinocyte cells in the presence of:
   (1) a wound exudate sample or wound biofilm sample obtained from the skin wound of said subject, and
   (2) the following compounds: a PARP inhibitor and one, two or three of (i) (iii):
      (i) a glucocorticoid
      (ii) ascorbic acid or a pharmaceutically acceptable salt thereof
      (iii) a protein growth factor;
wherein the subject is identified to be responsive to the treatment of impaired skin wound healing in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compounds of (2).

In one preferred embodiment of the present invention, the sample is a wound exudate sample. In another preferred embodiment, the sample is a wound biofilm sample. In a more preferred embodiment, the sample is a wound exudate sample.

In step iiia), the proliferation of keratinocyte cells is measured in the presence of a wound exudate sample or wound biofilm sample, obtained from said skin wound and the compounds of (2). The keratinocyte proliferation assay preferably includes culturing human primary keratinocyte cells, or HaCaT cells, which is a standard keratinocyte cell line, under standard conditions, such as by using DMEM containing FCS as medium, as for example described in the Examples. The cells are subsequently incubated on a solid support, thereby allowing the cells to adhere to the support. Further, the cells are contacted with the wound exudate sample, or wound biofilm sample, which is optionally diluted, and the compounds of (2), wherein the contacting may be performed before or after adherence of the cells occurs. For example, the optionally diluted wound exudate sample or wound biofilm sample and the compounds of (2) may be independently added to the adherent cells, for example by pipetting or otherwise adding the liquid, or the optionally diluted wound exudate sample or wound biofilm sample may be added to the non-adherent cells, for example by pipetting or otherwise adding the liquid to the cells, followed by allowing the keratinocyte cells to adhere. The cells are subsequently incubated, preferably for 6 hours to 200 hours, preferably 24 hours to 100 hours. In the examples, the cells are incubated for 72 hours. Subsequently, the amount, preferably the cell number, of the keratinocyte cells, is determined, such as by fixing cells and determining total protein content. The cells may for example be fixed using paraformaldehyde. Further, a suitable dye, such as sulforhodamine B may be used for determining the amount, preferably the cell number, of the keratinocyte cells. The stained cells may then be quantified e.g. by determining absorbance or fluorescence at a suitable wavelength, depending on the dye. Preferably, the method is performed in 2D cell culture, which allows for culturing the cells adherently on a solid support. Preferably, the sample is a wound exudate sample.

A keratinocyte cell may be a primary keratinocyte cell or a keratinocyte cell line, in particular a human primary keratinocyte cell or a human keratinocyte cell line. In one preferred embodiment, the keratinocyte cells used in the present invention are selected from HaCaT cells and primary keratinocyte cells. HaCaT cells represent an established and widely used human keratinocyte cell line.

In a more preferred embodiment, the keratinocyte cells used in the present invention are HaCaT cells.

Therefore, in a preferred embodiment, measuring the proliferation of keratinocyte cells in the presence of a wound exudate sample or wound biofilm sample obtained from a skin wound and the compounds of (2) includes the following steps:
(i) culturing keratinocyte cells,
(ii) incubating the cells on a solid support, thereby allowing the cells to adhere to the support,
(iii) contacting the cells with the wound exudate sample or wound biofilm sample, which is optionally diluted, and the compounds of (2), wherein the contacting may be performed before or after adherence of the cells occurs, and wherein the contacting of (1) and (2) may be performed simultaneously or sequentially, and
(iv) determining the amount, preferably the cell number, of the keratinocyte cells, such as by fixing cells and determining total protein content,
preferably wherein the method is performed in 2D cell culture.

In a further aspect, the present disclosure relates to an *in vitro* method of identifying a subject suffering from impaired skin wound healing to be responsive to the treatment with a glucocorticoid, optionally in combination with one, two or three of (i) to (iii):
(i) a pharmaceutical composition comprising a poly-ADP-ribose polymerase (PARP) inhibitor,
(ii) ascorbic acid or a pharmaceutically acceptable salt thereof,
(iii) a pharmaceutical composition comprising a protein growth factor,
comprising performing steps i) and/or ii) and/or iiia):
i) measuring the proliferation of primary fibroblast cells in the presence of:
   (1) a wound exudate sample or wound biofilm sample obtained from the skin wound of said subject, and
   (2) the following compound(s): a glucocorticoid and optionally one, two or three of (i) to (iii):
      (i) a PARP inhibitor,
      (ii) ascorbic acid or a pharmaceutically acceptable salt thereof
      (iii) a protein growth factor;
ii) measuring the fibroblast-derived matrix formation by primary fibroblast cells in the presence of:
   (1) a wound exudate sample or wound biofilm sample obtained from the skin wound of said subject, and
   (2) the following compound(s): a glucocorticoid and optionally one, two or three of (i) to (iii):
      (i) a PARP inhibitor,
      (ii) ascorbic acid or a pharmaceutically acceptable salt thereof
      (iii) a protein growth factor;
iiia) measuring the proliferation of keratinocyte cells in the presence of:
   (1) a wound exudate sample or wound biofilm sample obtained from the skin wound of said subject, and
   (2) the following compound(s): a glucocorticoid and optionally one, two or three of (i) to (iii):
      (i) a PARP inhibitor,
      (ii) ascorbic acid or a pharmaceutically acceptable salt thereof
      (iii) a protein growth factor,
wherein the subject is identified to be responsive to the treatment of impaired skin wound healing in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compound(s) of (2).

In one preferred disclosure, the sample is a wound exudate sample. In another preferred disclosure, the sample is a wound biofilm sample. In a more preferred disclosure, the sample is a wound exudate sample.

In a preferred disclosure, the compounds of (2) are a glucocorticoid and optionally one or both of (i) and (ii):
(i) a PARP inhibitor,
(ii) ascorbic acid or a pharmaceutically acceptable salt thereof.

The present disclosure relates to a glucocorticoid or a pharmaceutically acceptable salt thereof for use of in the treatment of impaired skin wound healing in a subject,
wherein the subject is identified to be responsive to the treatment of impaired skin wound healing by performing steps i) and/or ii) and/or iiia):
i) measuring the proliferation of primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof;
ii) measuring the fibroblast-derived matrix formation by primary fibroblast cells in the presence of:
   (1) a wound exudate sample or wound biofilm sample obtained from the skin wound of said subject, and
   (2) the following compound:
      the glucocorticoid or a pharmaceutically acceptable salt thereof,
iiia) measuring the proliferation of keratinocyte cells in the presence of:
   (1) a wound exudate sample or wound biofilm sample obtained from the skin wound of said subject, and
   (2) the following compound:
      the glucocorticoid or a pharmaceutically acceptable salt thereof,
wherein the subject is identified to be responsive to the treatment with the glucocorticoid or a pharmaceutically acceptable salt thereof, in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the glucocorticoid or pharmaceutically acceptable salt thereof.

In one preferred disclosure, the sample is a wound exudate sample. In another preferred disclosure, the sample is a wound biofilm sample. In a more preferred disclosure, the sample is a wound exudate sample.

Moreover, the accuracy and reliability can be further increased by including one or more additional assays which determine macrophage M1 and M2 markers and/or cytokine markers IL1alpha, IL1beta and/or TNFalpha in the context of wound exudate or wound biofilm obtained from the respective subject. These M1 and M2 markers may be cell surface protein markers, protein markers in the supernatant of macrophages or marker mRNAs in macrophages.

Macrophages are tissue-resident professional phagocytes and antigen-presenting cells (APC), which differentiate from circulating peripheral blood monocytes. Activated macrophages of different phenotypes are classified by skilled persons into M1-macrophages and M2 macrophages. M1-macrophages are activated macrophages which comprise immune effector cells with an acute inflammatory phenotype. These are highly aggressive against bacteria and produce large amounts of lymphokines. The M2-macrophages are alternatively activated and anti-inflammatory.

A "M2 marker" is understood as a protein marker which is specific for M2 macrophages. Preferably, the marker is secreted by the macrophages. Suitable M2 markers are known in the art and are preferably selected from CCL22 and CCL18. The markers may be determined by methods known in the art, e.g. by using an immunological assay, even more preferably by using an ELISA assay.

A "M1 marker" is understood as a protein marker which is specific for M1 macrophages. Preferably, the marker is secreted by the macrophages. Suitable M1 markers are known in the art and are preferably selected from CXCL10 and IL-23p19. The markers may be determined by methods known in the art, e.g. by using an immunological assay, even more preferably by using an ELISA assay.

A "M1 cell surface marker" is understood as a protein marker which is expressed at the surface of macrophages, and which is specific for M1 macrophages. Suitable M1 cell surface markers are known in the art and are preferably selected from CD38, CD64 and CD197. The amount(s) and/or frequency distribution(s) of the cell surface markers may be determined by an immunological assay and/or a fluorescence assay, in particular by FACS analysis, whereby typically a frequency distribution is determined.

A "M2 cell surface marker" is understood as a protein marker which is expressed at the surface of macrophages, and which is specific for M2 macrophages. Suitable M2 cell surface markers are known in the art and are preferably selected from CD200 receptor (CD200R), CD206 and CD209. The amount(s) and/or frequency distribution(s) of the cell surface markers may be determined by an immunological assay and/or a fluorescence assay, in particular by FACS analysis, whereby typically a frequency distribution is determined.

A "M2 marker mRNA" is understood as an mRNA which is expressed by macrophages, and which is specific for M2 macrophages. Suitable M2 marker mRNAs are known in the art and are preferably selected CD200 receptor (CD200R), CD206, CD209, CCL22 and CCL18. The marker mRNAs may be determined by methods known in the art. Preferably, the amount may be determined by contacting a probe which specifically binds to a marker mRNA, wherein the probe is optionally labelled, with the macrophage RNA under conditions which are conducive to hybridization, and detecting the hybridized probe. For example, the mRNA may be reversely transcribed into cDNA prior to detection.

A "M1 marker mRNA" is understood as an mRNA which is expressed by macrophages, and which is specific for M1 macrophages. Suitable M1 marker mRNAs are known in the art and are preferably selected from CD38, CD64, CD197, CXCL10 and IL-23p19. Preferably, the amount may be determined by contacting a probe which specifically binds to a marker mRNA, wherein the probe is optionally labelled, with the macrophage RNA under conditions which are conducive to hybridization, and detecting the hybridized probe. For example, the mRNA may be reversely transcribed into cDNA prior to detection.

The ratio of M1/M2 markers is indicative of a responsive subject, in combination with one or more cellular assays described above relating to measuring the proliferation of primary fibroblast cells, measuring the fibroblast-derived matrix (FDM) formation by primary fibroblast cells and measuring the proliferation of keratinocyte cells. In particular, an elevated ratio of M1/M2 markers, M1/M2 cell surface markers or M1/M2 marker mRNAs is indicative of a non-responsive subject, whereas a low ratio of M1/M2 markers, M1/M2 cell surface markers or M1/M2 marker mRNAs is indicative of a responsive subject.

Moreover, the amounts of the pro-inflammatory cytokines IL1alpha, IL1beta and TNF-alpha secreted by macrophages in a macrophage/fibroblast co-culture were found to be particularly predictive for identifying healing skin wounds or non-healing skin wounds as well as for monitoring wound healing. In particular, higher amounts of these cytokines were found to be secreted in the presence of WE from non-healing wounds as compared to WE from healing wounds. Cytokines IL1alpha, IL1beta and TNF-alpha are proteins, preferably human proteins, which are well-known to a skilled person. IL1alpha (also known as Interleukin-1α or IL-1α), IL1beta (also known as Interleukin-1β or IL-1β) and TNF-alpha (also known as Tumor Necrosis Factor α or TNF-α) may be determined by methods known in the art, e.g. by using an immunological assay, even more preferably by using an ELISA assay, as described in the Examples. IL1alpha, IL1beta and TNF-alpha are known to be pro-inflammatory cytokines.

Therefore, in a further preferred embodiment of the present invention, one, two or three of the following assays iiib) to iiid) may be included in the uses and methods of the invention:
iiib) measuring the amount(s) of one or more M1 marker(s) and one or more M2 marker(s) in the supernatant of macrophages incubated with
   (1) a wound exudate sample or wound biofilm sample obtained from the skin wound of said subject, and
   (2) the compound(s) of (2) described for any of above embodiments of the invention,
   wherein the macrophages are in co-culture with fibroblasts,
iiic) measuring the amount(s) and/or frequency distribution(s) of one or more M1 cell surface marker(s) and one or more M2 cell surface marker(s) on macrophages incubated with
   (1) a wound exudate sample or wound biofilm sample obtained from the skin wound of said subject, and
   (2) the compound(s) of (2) described for any of above embodiments of the invention,
   wherein the macrophages are in co-culture with fibroblasts,
iiid) measuring the expression level(s) of one or more M1 marker mRNA(s) and one or more M2 marker mRNA(s) in macrophages incubated with
   (1) a wound exudate sample or wound biofilm sample obtained from the skin wound of said subject, and
   (2) the compound(s) of (2) described for any of above embodiments of the invention, wherein the macrophages are in co-culture with fibroblasts.

In one preferred embodiment of the present invention, the sample is a wound exudate sample. In another preferred embodiment, the sample is a wound biofilm sample. In a more preferred embodiment, the sample is a wound exudate sample.

Therefore, in a further preferred embodiment of the present invention, one, two or three of the following assays iiib) to iiid) or one, two, three or four of the following steps iiib) to iiie) may be included in the uses and methods of the invention:
iiib) measuring the amount(s) of one or more M1 marker(s) and one or more M2 marker(s) in the supernatant of macrophages incubated with
   (1) a wound exudate sample or wound biofilm sample obtained from said skin wound, and
   (2) the compound(s) of (2) described for any of above embodiments of the invention,
   wherein the macrophages are in co-culture with fibroblasts, and
   wherein the one or more M1 markers are selected from CXCL10 and IL-23p19, and the one or more M2 markers are selected from CCL22 and CCL18,
iiic) measuring the amount(s) and/or frequency distribution(s) of one or more M1 cell surface marker(s) and one or more M2 cell surface marker(s) on macrophages incubated with
   (1) a wound exudate sample or wound biofilm sample obtained from said skin wound, and
   (2) the compound(s) of (2) described for any of above embodiments of the invention,
   wherein the macrophages are in co-culture with fibroblasts, and
   wherein the one or more M1 cell surface markers are selected from CD38, CD64 and CD197, and wherein the one or more M2 cell surface markers are selected from CD200 receptor, CD206 and CD209,
iiid) measuring the expression level(s) of one or more M1 marker mRNA(s) and one or more M2 marker mRNA(s) in macrophages incubated with
   (1) a wound exudate sample or wound biofilm sample obtained from said skin wound, and
   (2) the compound(s) of (2) described for any of above embodiments of the invention,
   wherein the macrophages are in co-culture with fibroblasts, and
   wherein the one or more M1 marker mRNA(s) are selected from CD38, CD64, CD197, CXCL10 and IL-23p19, and the one or more M2 marker mRNA(s) are selected from CD200 receptor (CD200R), CD206, CD209, CCL22 and CCL18,
iiie) measuring the amount(s) of one or more cytokine markers in the supernatant of macrophages incubated
   (1) with a wound exudate sample or wound biofilm sample obtained from said skin wound, and
   (2) the compound(s) of (2) described for any of above embodiments of the invention,
   wherein the macrophages are in co-culture with fibroblasts, and
   wherein the one or more cytokine markers are selected from IL-1alpha, IL-1beta and TNF-alpha.

Preferably, the subject is identified to be responsive to the treatment with the compound(s) of (2), in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compound(s) of (2), and/or in case one or more of the following applies:
- the ratio of amount(s) of one or more M1 marker(s) to the amount(s) of one or more M2 marker(s) obtained in iiib) is/are below a control value established in the absence of the compound(s) of (2),
- the ratio of amount(s) and/or frequency distribution(s) of one or more M1 cell surface marker(s) to the amount(s) and/or frequency distribution(s) of one or more M2 cell surface marker(s) obtained in iiic) is/are below a control value established in the absence of the compound(s) of (2),
- the ratio of expression level(s) of one or more M1 marker mRNA(s) to the expression level(s) of one or more M2 marker mRNA(s) obtained in iiid) is/are below a control value established in the absence of the compound(s) of (2).

Preferably, the subject is identified to be responsive to the treatment with the compound(s) of (2), in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compound(s) of (2), and/or in case one or more of the following applies:
- the ratio of amount(s) of one or more M1 marker(s) to the amount(s) of one or more M2 marker(s) obtained in iiib) is/are below a control value established in the absence of the compound(s) of (2),
- the ratio of amount(s) and/or frequency distribution(s) of one or more M1 cell surface marker(s) to the amount(s) and/or frequency distribution(s) of one or more M2 cell surface marker(s) obtained in iiic) is/are below a control value established in the absence of the compound(s) of (2), in particular wherein the ratio is selected from a CD38/CD209 ratio, a CD197/CD209 ratio and a CD197/CD206 ratio,
- the ratio of expression level(s) of one or more M1 marker mRNA(s) to the expression level(s) of one or more M2 marker mRNA(s) obtained in iiid) is/are below a control value established in the absence of the compound(s) of (2),
- the value obtained in iiie) is below a control value established in the absence of the compound(s) of (2).

It was found that the following M1 cell surface marker / M2 cell surface marker ratios are also predictive for responsiveness: a CD38/CD209 ratio, a CD197/CD209 ratio or a CD197/CD206 ratio below a control value established in the absence of the compound(s) of (2) is identifying a patient to be responsive to the treatment with the compound(s).

Therefore, in another preferred embodiment, the ratio of amount(s) and/or frequency distribution(s) is selected from a CD38/CD209 ratio, a CD197/CD209 ratio and a CD197/CD206 ratio.

The frequency distribution may be determined by determining the %age of cells which are positive for a given marker within a population, which is the most commonly used readout in FACS analysis. Alternatively, the amount may be determined by determining the quantity of cell surface expression, as a surrogate for the number of labelled molecules on the cell surface per individual cell when using labelled binding agents for the markers, as for example measured by the mean fluorescence intensity.

In a preferred embodiment, measuring the amount(s) of one or more M1 marker(s) and one or more M2 marker(s) in the supernatant of macrophages incubated with a wound exudate sample or wound biofilm sample obtained from a skin wound includes the following steps:
(i) co-culturing primary human monocyte cells with (a) human dermal fibroblast cells in 2D cell culture or (b) fibroblast-derived matrices,
(ii) incubating the cells until macrophage differentiation is reached, optionally wherein CD163 is used as a cell surface marker of macrophage differentiation,
(iii) contacting the cells with a wound exudate sample or wound biofilm sample, which is optionally diluted, and the compounds of (2), wherein the contacting may be performed before or after adherence of the cells occurs, and wherein the contacting of (1) and (2) may be performed simultaneously or sequentially, and
(iv) determining the amount of one or more M1 markers and one or more M2 markers in the cell culture supernatant,
preferably wherein the one or more M1 markers are selected from CXCL10 and IL-23p19, and/or the one or more M2 markers are selected from CCL22 and CCL18, more preferably wherein the markers are determined by using an immunological assay, even more preferably by using an ELISA assay.

In one preferred embodiment of the present invention, the sample is a wound exudate sample. In another preferred embodiment, the sample is a wound biofilm sample. In a more preferred embodiment, the sample is a wound exudate sample.

For example, primary human monocyte cells may be co-cultured with human dermal fibroblast cells in 2D cell culture, or with fibroblast-derived matrices. Methods for generating fibroblast-derived matrices are described above, as well as in the examples. Subsequently, the cells are incubated until macrophage differentiation is reached. For example, CD163 can be used as a cell surface marker of macrophage differentiation. Further, the cells are contacted with a wound exudate sample, or wound biofilm sample, which is optionally diluted, and the compounds of (2), for example by pipetting the sample to the cells, and optionally gentle mixing. The compounds are added after macrophages have differentiated; e.g. after 4 to 7 days. Further, the cells are incubated, preferably for 1 hour 100 hours, e.g. 4 hours to 100 hours. Subsequently, the amount of one or more M1 markers and one or more M2 markers in the cell culture supernatant is determined. The supernatant is typically harvested for such purpose and the markers are determined using a suitable assay, such as immunological assay. For example, an ELISA may be used.

In another preferred embodiment, measuring the amount(s) and/or frequency distribution(s) of one or more M1 cell surface marker(s) and one or more M2 cell surface marker(s) on macrophages incubated with a wound exudate sample or wound biofilm sample obtained from a skin wound includes the following steps:
(i) co-culturing primary human monocyte cells with (a) human dermal fibroblast cells in 2D cell culture or (b) fibroblast-derived matrices,
(ii) incubating the cells until macrophage differentiation is reached, optionally wherein CD163 is used as a cell surface marker of macrophage differentiation,
(iii) contacting the cells with a wound exudate sample or wound biofilm sample, which is optionally diluted, and the compounds of (2)
(iv) determining the amount(s) and/or frequency distribution(s) of one or more M1 cell surface marker(s) and one or more M2 cell surface marker(s) on the cell surface of macrophages.

In one preferred embodiment of the present invention, the sample is a wound exudate sample. In another preferred embodiment, the sample is a wound biofilm sample. In a more preferred embodiment, the sample is a wound exudate sample.

For example, primary human monocyte cells may be co-cultured with human dermal fibroblast cells in 2D cell culture, or with fibroblast-derived matrices. Methods for generating fibroblast-derived matrices are described above, as well as in the examples. Subsequently, the cells are incubated until macrophage differentiation is reached. For example, CD163 can be used as a cell surface marker of macrophage differentiation. Further, the cells are contacted with a wound exudate sample, or wound biofilm sample, which is optionally diluted, and the compounds of (2) for example by pipetting the sample to the cells, and optionally gentle mixing. The compounds are added after macrophages have differentiated; e.g. after 4 to 7 days. Further, the cells are incubated, preferably for 1 hour 100 hours, e.g. 4 hours to 100 hours. Subsequently, the amount(s) and/or frequency distribution(s) of one or more M1 cell surface marker(s) and one or more M2 cell surface marker(s) on the cell surface of macrophages is determined. For example, the cells may be harvested and subjected to FACS analysis, gating on the monocyte/macrophage population. Geometric means of mean fluorescence intensities can be used to quantify surface marker expression.

Preferably, the one or more M1 cell surface markers are selected from CD38, CD64 and CD197, and/or the one or more M2 cell surface markers are selected from CD200 receptor (CD200R), CD206 and CD209, more preferably wherein the amount(s) and/or frequency distribution(s) of the cell surface markers are determined by an immunological assay and/or a fluorescence assay, in particular by FACS analysis.

It was found that the following M1 cell surface marker / M2 cell surface marker ratios are also predictive for determining responsiveness: a CD38/CD209 ratio, a CD197/CD209 ratio and a CD197/CD206 ratio. A CD38/CD209 ratio, a CD197/CD209 ratio or a CD197/CD206 ratio below a control value established in the absence of the compound(s) of (2) is identifying a patient to be responsive to the treatment with the compound(s).

Therefore, in another preferred embodiment, the ratio of amount(s) and/or frequency distribution(s) is selected from a CD38/CD209 ratio, a CD197/CD209 ratio and a CD197/CD206 ratio.

Accordingly, in a preferred embodiment, the one or more M1 cell surface marker is selected from CD38 and the one or more M2 cell surface marker is selected from CD209, or the one or more M1 cell surface marker is selected from CD197 and the one or more M2 cell surface marker is selected from CD209 and CD206.

In one preferred embodiment, step (iv) comprises contacting the macrophages with binding agents, preferably antibodies, which specifically recognize one or more M1 surface marker(s) and one or more M2 surface marker(s), wherein the binding agents are optionally labelled, in particular labelled with a fluorescent label, and determining the amount of binding molecules bound to the macrophages, in particular by determining mean fluorescence intensity, thereby determining the amount(s) of the cell surface markers. For example, antibodies specifically recognizing the surface markers and which contain a fluorescent label may be used.

In another preferred embodiment, step (iv) comprises contacting the macrophages with binding agents, preferably antibodies, which specifically recognize one or more M1 surface marker(s) and one or more M2 surface marker(s), wherein the binding agents are optionally labelled, in particular labelled with a fluorescent label, and determining the percentages of cells which are positive for the one or more M1 surface marker(s) and the one or more M2 surface marker(s), respectively, within a cell population, in particular wherein FACS analysis is performed, thereby determining the frequency distribution(s) of the cell surface markers. For example, antibodies specifically binding to the surface markers and which contain a fluorescent label may be used.

Determination of proteins as binding agents of a marker protein can be performed using any of a number of known methods for identifying and obtaining proteins that specifically interact with proteins or polypeptides, for example, a yeast two-hybrid screening system such as that described in U.S. Pat. No. 5,283,173 and U.S. Pat. No. 5,468,614, or the equivalent. A binding agent which specifically recognizes a marker has preferably at least an affinity of 10⁷l/mol for its corresponding target molecule. The binding agent which specifically recognizes a marker preferably has an affinity of 10⁸ l/mol or even more preferred of 10⁹ l/mol for its target marker molecule. As the skilled person will appreciate, the term specific is used to indicate that other biomolecules present in the sample do not significantly bind to the binding agent which specifically recognizes the marker. Preferably, the level of binding to a biomolecule other than the target marker molecule results in a binding affinity which is only 10% or less, more preferably only 5% or less of the affinity to the target marker molecule, respectively. A preferred specific binding agent will fulfill both the above minimum criteria for affinity as well as for specificity.

A binding agent which specifically recognizes a marker preferably is an antibody reactive with the marker. The term antibody refers to a polyclonal antibody, a monoclonal antibody, antigen binding fragments of such antibodies, single chain antibodies as well as to genetic constructs comprising the binding domain of an antibody. The term "antibodies" includes polyclonal antibodies, monoclonal antibodies, fragments thereof such as F(ab')2, and Fab fragments, as well as any naturally occurring or recombinantly produced binding partners, which are molecules that specifically bind to a marker protein. Any antibody fragment retaining the above criteria of a specific binding agent can be used.

For measurement, the sample obtained from an individual is incubated with the binding agent that specifically recognizes the marker in question under conditions appropriate for formation of a binding agent marker-complex. Such conditions need not be specified, since the skilled artisan without any inventive effort can easily identify such appropriate incubation conditions. The amount of binding agent marker-complex is measured and used in the methods and uses of the invention. As the skilled artisan will appreciate there are numerous methods to measure the amount of the specific binding agent marker-complex all described in detail in relevant textbooks (cf., e.g., Tijssen P., *supra,* or Diamandis, E.P. and Christopoulos, T.K. (eds.), Immunoassay, Academic Press, Boston (1996)).

Particularly, monoclonal antibodies to the marker(s) are used in a quantitative (amount or concentration of the marker(s) is determined) immunoassay.

For example, the marker may be detected in a sandwich type assay format. In such assay a first specific binding agent is used to capture the marker in question on the one side and a second specific binding agent (e.g. a second antibody), which is labeled to be directly or indirectly detectable, is used on the other side. The second specific binding agent may contain a detectable reporter moiety or label such as an enzyme, dye, radionuclide, luminescent group, fluorescent group or biotin, or the like. Any reporter moiety or label could be used with the methods disclosed herein so long as the signal of such is directly related or proportional to the quantity of binding agent remaining on the support after wash. The amount of the second binding agent that remains bound to the solid support is then determined using a method appropriate for the specific detectable reporter moiety or label. For radioactive groups, scintillation counting or autoradiographic methods are generally appropriate. Antibody-enzyme conjugates can be prepared using a variety of coupling techniques (for review see, e.g., Scouten, W. H., Methods in Enzymology 135:30-65, 1987). Spectroscopic methods can be used to detect dyes (including, for example, colorimetric products of enzyme reactions), luminescent groups and fluorescent groups. Biotin can be detected using avidin or streptavidin, coupled to a different reporter group (commonly a radioactive or fluorescent group or an enzyme). Enzyme reporter groups can generally be detected by the addition of substrate (generally for a specific period of time), followed by spectroscopic, spectrophotometric or other analysis of the reaction products. Standards and standard additions can be used to determine the level of antigen in a sample, using well known techniques.

Immunoassays for measuring marker proteins of the invention include for example ELISA, enzyme immunoassay (EIA) and electro-chemiluminescence immunoassay (ECLIA) for the quantitative determination of a marker protein described herein.

In another preferred embodiment, measuring the expression level(s) of one or more M1 marker mRNA(s) and one or more M2 marker mRNA(s) in macrophages incubated with a wound exudate sample or wound biofilm sample obtained from a skin wound includes the following steps:
(i) co-culturing primary human monocyte cells with (a) human dermal fibroblast cells in 2D cell culture or (b) fibroblast-derived matrices,
(ii) incubating the cells until macrophage differentiation is reached, optionally wherein CD163 is used as a cell surface marker of macrophage differentiation,
(iii) contacting the cells with a wound exudate sample or wound biofilm sample, which is optionally diluted, and the compounds of (2), wherein the contacting may be performed before or after adherence of the cells occurs, and wherein the contacting of (1) and (2) may be performed simultaneously or sequentially, and
(iv) determining the expression level(s) of one or more M1 marker mRNA(s) and one or more M2 marker mRNA(s) in the macrophages.

In one preferred embodiment of the present invention, the sample is a wound exudate sample. In another preferred embodiment, the sample is a wound biofilm sample. In a more preferred embodiment, the sample is a wound exudate sample.

Preferably, the one or more M1 marker mRNA(s) are selected from CD38, CD64, CD197, CXCL10 and IL-23p19, and/or the one or more M2 marker mRNA(s) are selected from CD200 receptor (CD200R), CD206, CD209, CCL22 and CCL18, more preferably the method comprises contacting a probe which specifically binds to a marker mRNA, wherein the probe is optionally labelled, with the macrophage RNA under conditions which are conducive to hybridization, and detecting the hybridized probe.

For example, primary human monocyte cells may be co-cultured with human dermal fibroblast cells in 2D cell culture, or with fibroblast-derived matrices. Methods for generating fibroblast-derived matrices are described above, as well as in the examples. Subsequently, the cells are incubated until macrophage differentiation is reached. For example, CD163 can be used as a cell surface marker of macrophage differentiation. Further, the cells are contacted with a wound exudate sample, or wound biofilm sample, which is optionally diluted, and the compounds of (2), for example by pipetting the sample to the cells, and optionally gentle mixing. Further, the cells are incubated, preferably for 1 hour 100 hours, e.g. 4 hours to 100 hours. Subsequently, the expression level(s) of one or more M1 marker mRNA(s) and one or more M2 marker mRNA(s) in the macrophages is determined. For example, the cells may be harvested and mRNA expression level(s) may be determined using suitable probes. For example, the expression level of a housekeeping gene such as actin or GAPDH may be determined and the expression level(s) of M1 or M2 marker RNA(s) may be determined as expression level relative to a housekeeping gene.

In another preferred embodiment, measuring the amount(s) of one or more cytokine markers selected from IL-1alpha, IL-1 beta and TNF-alpha in the supernatant of macrophages incubated with a wound exudate sample or wound biofilm sample obtained from a skin wound includes the following steps:
(i) co-culturing primary human monocyte cells with (a) human dermal fibroblast cells in 2D cell culture or (b) fibroblast-derived matrices,
(ii) incubating the cells until macrophage differentiation is reached, optionally wherein CD163 is used as a cell surface marker of macrophage differentiation,
(iii) contacting the cells with a wound exudate sample or wound biofilm sample, which is optionally diluted, and the compounds of (2), wherein the contacting of (1) and (2) may be performed simultaneously or sequentially, and
(iv) determining the amount of one or more cytokine markers selected from IL-1alpha, IL-1 beta and TNF-alpha in the cell culture supernatant,
preferably wherein the cytokine markers are determined by using an immunological assay, more preferably by using an ELISA assay.

In one preferred embodiment of the present invention, the sample is a wound exudate sample. In another preferred embodiment, the sample is a wound biofilm sample. In a more preferred embodiment, the sample is a wound exudate sample.

For example, primary human monocyte cells may be co-cultured with human dermal fibroblast cells in 2D cell culture, or with fibroblast-derived matrices. Methods for generating fibroblast-derived matrices are described above, as well as in the examples. Subsequently, the cells are incubated until macrophage differentiation is reached. For example, CD163 can be used as a cell surface marker of macrophage differentiation. Further, the cells are contacted with a wound exudate sample or wound biofilm sample, which is optionally diluted, and the compounds of (2), wherein the contacting of the sample and the compounds of (2) may be performed simultaneously or sequentially, for example by pipetting the sample to the cells, and optionally gentle mixing. Further, the cells are incubated, preferably for 1 hour to 100 hours, e.g. 4 hours to 100 hours. Subsequently, the amount of one or more of IL-1alpha, IL-1beta and TNF-alpha in the cell culture supernatant is determined. The supernatant is typically harvested for such purpose and the cytokine markers are determined using a suitable assay, such as immunological assay. For example, an ELISA may be used. In a preferred embodiment, the sample is a wound exudate sample.

The amounts of IL-1alpha, IL-1 beta and TNF-alpha in the supernatant of macrophages are indicative for a patient responsive to the treatment with the compound(s) of (2). Accordingly, a patient is identified to be responsive to the treatment with the compound(s) of (2) in case the value obtained for the amounts of IL-1alpha, IL-1 beta and TNF-alpha is below a control value established in the absence of the compound(s) of (2).

### Figure legend

- Figure 1:: shows profiling of compound dexamethasone in the human dermal fibroblast proliferation assay (2D) with or without wound exudate. Squares: no WE added; circles: WE added from patient #43; triangles: WE added from patient #78. X-axis shows concentration of dexamethasone.
- Figure 2:: shows that the effect of dexamethasone on fibroblast proliferation differs in the presence and absence of different wound exudates. The effect of dexamethasone on fibroblast proliferation was determined for wound exudates from patients #27, #49, #43, #78 and #18. Left columns: no wound exudate added, only medium added; right columns: wound exudate added. In normal medium, dexamethasone reduces fibroblast proliferation. Paradoxically, in the presence of WE, dexamethasone enhances proliferation. This effect was confirmed for further glucocorticoids, namely hydrocortisone, prednisolone and medroxyprogesterone (data not shown). Therefore, it could surprisingly be shown that glucocorticoids are beneficial for chronic wound healing of specific patients, as opposed to the "dogma" in the prior art describing glucocorticoids to impair wound healing. Patients responsive to the treatment of impaired skin wound healing can be identified by applying an assay as described herein, in particular by determining human dermal fibroblast proliferation and/or fibroblast-derived matrix formation in the presence of wound exudate obtained from the respective patient.
- Figure 3:: shows profiling of compound veliparib in the human dermal fibroblast proliferation assay (2D) with or without wound exudate from patients #78 and #43. Squares: no WE added; circles: WE added from patient #43; triangles: WE added from patient #78. X-axis shows concentration of veliparib. Veliparib completely reversed inhibition of wound exudate (WE)-induced fibroblast proliferation with wound exudate #78 (diabetic patient).
- Figure 4:: shows profiling of compound veliparib in the human dermal fibroblast proliferation assay (2D) with or without wound exudate from a plurality of patients. Squares: WE added from patient #49; circles: WE added from patient #43; triangles: WE added from patient #78; diamonds: WE added from patient #27. X-axis shows concentration of veliparib. The effect of veliparib was most prominent in the two patients with diabetes.
- Figure 5:: shows the reproducibility of the effect of veliparib in fibroblast 2D culture with different samples of one patient. The different samples from one patient are denoted #77 and #78. The patient has the following co-morbidities: diabetes, adipositas, kidney transplantation. circles: WE added from sample #78; diamonds: WE added from sample #77. X-axis shows concentration of veliparib. The effect of veliparib was reproducible in different samples of the same patient (day 1 and day 8). This patient received a glucocorticoid (prednisolone) as co-medication. This suggests that veliparib is in particular suitable for treating impaired skin wound healing in patients already receiving a glucocorticoid as therapy as well as for treating impaired skin wound healing in combination with a glucocorticoid.
- Figure 6:: shows the effect of glucocorticoid dexamethasone and PARP inhibitors in the 3D fibroblast culture regarding the formation of fibroblast-derived matrix as optically evaluated by microscopy. DEXA: dexamethasone. The two PARP inhibitors compounds talazoparib and veliparib, especially talazoparib, "cleaned up" WE-induced fibroblast matrix inhibition. The combination of veliparib with dexamethasone was superior to each substance alone.
- Figure 7:: shows the effect of dexamethasone and PARP inhibitors in 3D fibroblast culture regarding the formation of fibroblast-derived matrix in patient #78. DEXA: dexamethasone; veli: veliparib; MED: medroxyprogesterone. Glucocorticoids DEXA (= dexamethasone) and MED (= medroxyprogesterone) enhance the effect of veliparib on rescuing FDM formation after WE treatment. Veliparib, in turn, enhances the effect of glucocorticoids in this patient (co-morbidities: diabetes, immunosuppression after kidney transplantation).
- Figure 8:: shows the effect of dexamethasone and PARP inhibitors in 3D fibroblast culture regarding the formation of fibroblast-derived matrix in patient #49 (patient is non-diabetic and non-immunosuppressed). DEXA: dexamethasone; veli: veliparib; MED: medroxyprogesterone. In this patient (non-diabetic, non-immunosuppressed), the effect of veliparib could be enhanced with glucocorticoids, but not vice versa.
- Figure 9:: shows the effect of a plurality of PARP inhibitors in 3D fibroblast culture with WE from patient #78. Talazoparib was the most active of the PARP inhibitors tested in 3D culture. This is in line with its higher potency in tumor models *in vitro* and *in vivo.* Filled diamonds: veliparib; filled squares: olaparib; filled triangles: AZD-2461; filled circles: niraparib; open diamonds: rucaparib; open squares: talazoparib; open triangles: AG-14361. A strong positive effect could be shown for PARP inhibitors talazoparib, veliparib, olaparib, rucaparib, and AZD-2461.
- Figure 10:: shows PARP inhibitor compound profiling: Mechanistically related PARP inhibitor compounds in 2D fibroblast culture with WE from patient #78. Filled diamonds: veliparib; filled squares: olaparib; filled triangles: AZD-2461; filled circles: niraparib; open diamonds: rucaparib; open squares: talazoparib; open triangles: AG-14361. The effect from 3D fibroblast culture relating to FDM formation could be reproduced in the 2D assay on human dermal fibroblast proliferation.
- Figure 11:: shows additional PARP inhibitor compound profiling data: Mechanistically related PARP inhibitor compounds in 2D fibroblast culture with WE from patient #43. Filled diamonds: veliparib; filled squares: talazoparib; filled triangles: rucaparib; filled circles: PJ-34; open triangles: 1,5 IQD; open circles: 3-AB; open diamonds: BGP-15. The literature PARP inhibitor compounds discussed in the context of skin disorders (PJ-34, 1,5-IQD; 3-AB and BGP-15) are less active or inactive.
- Figure 12:: shows additional PARP inhibitor compound profiling data: Mechanistically related PARP inhibitor compounds in 2D fibroblast culture with WE from patient #78. Filled diamonds: veliparib; filled squares: talazoparib; filled triangles: rucaparib; filled circles: PJ-34; open triangles: 1,5 IQD; open circles: 3-AB; open diamonds: BGP-15. The literature PARP inhibitor compounds discussed in the context of skin disorder (PJ-34, 1,5-IQD; 3-AB and BGP-15) are less active or inactive.
- Figure 13:: shows additional PARP inhibitor compound profiling; mechanistically related compounds in 2D fibroblast culture without WE (control). Filled diamonds: veliparib; filled squares: talazoparib; filled triangles: rucaparib; filled circles: PJ-34; open triangles: 1,5 IQD; open circles: 3-AB; open diamonds: BGP-15. Most compounds have no effect on fibroblast proliferation in the absence of WE.
- Figure 14:: shows 2D fibroblast culture results with wound exudate from patient #78: 3 different PARP inhibitors (veliparib, talazoparib and PJ-34) ± dexamethasone ± Vit. C. Both dexamethasone and vitamin C synergistically enhance the PARP inhibitor compound effects. Veliparib and talazoparib surprisingly show strongly beneficial effects.
- Figure 15:: shows 2D fibroblast culture results with wound exudate from patient #43: 3 different PARP inhibitors (veliparib, talazoparib and PJ-34) ± dexamethasone ± Vit. C. Both dexamethasone and vitamin C synergistically enhance the PARP inhibitor compound effects. Veliparib and talazoparib surprisingly show strongly beneficial effects.
- Figure 16:: shows 2D fibroblast culture results without wound exudate as compared to with wound exudate: 3 different PARP inhibitors (veliparib, talazoparib and PJ-34) ± dexamethasone ± Vit. C. In the absence of WE, dexamethasone inhibits HDF proliferation/extracellular matrix (ECM) formation. The results are summarized as follows: 1. In the presence of WE, dexamethasone surprisingly enhances HDF proliferation/ECM formation. 2. In the absence of WE, vitamin C enhances HDF proliferation/ECM formation. 3. In the presence of 2/3 WE, vitamin C has no effect on HDF proliferation/ECM formation. 4. When both DEXA and Vit C synergistically enhanced proliferation, the combination of the two was better than each compound alone.
- Figure 17:: shows 2D fibroblast culture (human dermal fibroblast (HDF) proliferation assay) results without wound exudate (A) as compared to with wound exudate #78 (B): 2 different PARP inhibitors (veliparib and talazoparib) ± PDGF. In the absence of WE, PDGF induces HDF proliferation, whereas in the presence of WE #78, PDGF has no effect. Veliparib (10 µM) and talazoparib (1 µM) have no or even an inhibitory effect on fibroblasts on their own in the absence of WE, but induce proliferation in the presence of WE #78. Surprisingly, this effect is additively or synergistically enhanced by PDGF (20 ng/ml). The results are summarized as follows: 1. In the presence of WE, veliparib and talazoparib surprisingly enhance HDF proliferation. 2. When PDGF was combined with either veliparib or talazoparib, surprisingly the combination with the protein growth factor was better than each compound alone.
- Figure 18:: shows 2D fibroblast (human dermal fibroblast (HDF) proliferation) culture results with wound exudate #78: 4 different PARP inhibitors (Veliparib (A), Olaparib (B), Rucaparib (C) and Talazoparib (D)) ± PDGF. In the presence of wound exudate #78, the four PARP inhibitor compounds veliparib, olaparib, rucaparib and talazoparib show a dose-dependent increase of HDF proliferation, which is even further enhanced by the addition of PDGF, which, on its own, is inactive in the presence of WE #78. (Diamonds: PARP inhibitor compound in medium + WE #78; circles: PARP inhibitor compound + PDGF, 20 ng/ml, in medium + WE #78).
- Figure 19:: shows 2D fibroblast (human dermal fibroblast (HDF) proliferation) culture results without and with wound exudate #78. In the absence of wound exudate, PDGF (20ng/ml) enhances proliferation, an effect, which is completely abrogated by the PDGF receptor inhibitor crenolanib (1 µM). In the presence of WE #78 alone, fibroblast proliferation is strongly inhibited, without any effect of PDGF and/or crenolanib. Talazoparib (0.3µM) in the presence of WE #78 rescues the cells from the inhibitory effect of the WE, and PDGF further enhances this recovery. The PDGF effect in this system is completely abolished by crenolanib, indicating that talazoparib restores the responsiveness of the fibroblasts to PDGF in the presence of this wound exudate.
- Figure 20:: shows 2D fibroblast (human dermal fibroblast (HDF) proliferation) culture results without and with wound exudate #78 in the absence and presence of TGF-β (20 ng/ml) to induce myofibroblast differentiation and veliparib (10 µM). In the absence of wound exudate (A - D), TGF-β increases the staining for the myofibroblast marker alpha-smooth muscle actin (α-SMA), while veliparib has no effect. In the presence of WE #78 (E - H), TGF-β does not induce α-SMA on its own, but in combination with veliparib shows more strongly stained cells than veliparib alone. Veliparib, in combination with TGF-β leads to expression of α-SMA, an indicator of wound contractility.
- Figure 21:: shows proliferation of fresh (A: passage 9) and senescent (B: passage 21) human dermal fibroblast (HDF) without and with wound exudate #78 and the PARP inhibitor veliparib (VELI) alone or in combination with dexamethasone (DEXA). Veliparib was used at 1, 3 and 10 µM; dexamethasone was kept constant at a suboptimal dose (3 nM). In the absence of wound exudate, dexamethasone contributes to inhibition of HDF proliferation. In the presence of wound exudate, dexamethasone contributes to the enhancement of HDF proliferation by veliparib. This effect is observed both in fresh (A) and senescent fibroblasts (B).
- Figure 22:: shows the effects of different PARP inhibitors in the presence or absence of a suboptimal concentration of dexamethasone on wound exudate-induced inhibition of fibroblast proliferation and induction of IL-1β secretion. PARP inhibitors were used at the concentrations indicated on the x-axis; dexamethasone was used at 3 nM. Two different wound exudates (#43 and #78) were used with veliparib. The compounds veliparib (VELI), olaparib (OLA), AZD-2461 (AZD), rucaparib (RUCA), AG-14351 (AG) and talazoparib (TALA) enhanced cell proliferation while at the same time reducing IL-1β secretion. These effects were enhanced by dexamethasone.
- Figure 23:: shows results of a fibroblast - macrophage coculture experiment with wound exudate #78 and veliparib 10 µM (VELI) or talazoparib 0.1 µM (TALA) in the absence or presence of dexamethasone 10 nM (DEXA). A) The percentage of live cells in the FACS CD45-gate (corresponding to macrophages), which is reduced upon incubation with wound exudate, is increased by veliparib and further increased by the combination of veliparib with dexamethasone, while dexamethasone has only a marginal effect. B) The proinflammatory cytokine IL-1α, induced by wound exudate, is reduced by veliparib and talazoparib, and this effect is enhanced by dexamethasone.

### Examples

### Example 1: Assays used in the invention

### Abbreviations

| Abbreviation | Description |
|---|---|
| α-SMA | Alpha smooth muscle actin |
| bFGF | Basic fibroblast growth factor |
| DMSO | Dimethylsulfoxide |
| EC | Endothelial cells |
| FCS | Fetal calf serum |
| FDM | Fibroblast-derived matrices |
| FGF10 | Fibroblast growth factor 10 (KGF2) |
| HaCaT | Human keratinocyte cell line |
| HBSS | Hank's balanced salt solution |
| HDF | Human dermal fibroblasts |
| hEGF | Human epidermal growth factor |
| HGF | Hepatocyte growth factor |
| hIGF-1 | Human insulin-like growth factor-1 |
| hVEGF | Human vascular endothelial growth factor |
| KGF2 | Keratinocyte growth factor 2 (FGF10) |
| M-CSF | Macrophage colony stimulating factor |
| PBS | Phosphate buffered saline |
| PDGF-BB | Platelet-derived growth factor |
| RPMI | Roswell Park Memorial Institute medium |
| SRB | Sulforhodamine B |
| TGFbeta | Transforming growth factor beta (TGF-β) |
| WE | Wound exudate |

The assays described in Examples 1.1 and 1.2 represent predictive models for skin wound healing. Most of the non-healing wound exudates (WE) obtained from a variety of patients inhibit proliferation of primary human fibroblasts (HDF) in the assay as described in Example 1.1 and also inhibit the formation of fibroblast-derived matrices (FDM) in 3D, as described in Example 1.2. Approximately one third of the WE enhance FDM formation; most of these WE are from 2 patients.

### Example 1.1: Primary human dermal fibroblast (HDF) proliferation assay: measuring the proliferation of primary fibroblast cells and the secretion of IL-1β in the presence of a wound exudate sample obtained from a skin wound of an individual

Primary human dermal fibroblasts (HDF) were purchased from CELLnTEC, Bern. They were routinely grown in Dulbecco's modified Eagle's medium (DMEM) containing 10% FCS, 2mM glutamine, and 100 U/ml penicillin/100 µg/ml streptomycin. Media, antibiotics, and glutamine were bought from Lonza. The cells were used at passage 5-15. Cells were trypsinized and seeded at 5000 cells/well in 200µl into the inner wells of 96-well plates. The outer wells were loaded with sterile water. The cells were allowed to adhere overnight and then incubated for 72 hours at 37°C under the following conditions: graded compound concentrations or 20ng/ml PDGF-BB (Tonbo Biosciences) in the absence or presence of different dilutions of sterile-filtered WE in medium. For control samples, 200µl medium was added instead of specific stimuli. Alternatively, the cells were seeded into 384-well plates at 2500 cells/well directly together with test compounds or growth factors and WE or medium in a total volume of 50µl.

At the end of the 72-hour incubation period, supernatants were removed for the determination of IL-1β, and the cells were fixed with 4% paraformaldehyde (Morphisto) for 15 minutes at room temperature and washed 3 times with PBS. A control plate was fixed after the overnight adherence of the cells (day 1) to determine the starting cell number.

Total cellular protein was determined as a measure of cell number by staining the fixed cells with sulforhodamine B (SRB, Sigma). A 0.4% SRB solution in 1% acetic acid was added to the wells for 30 minutes. The wells were then washed with 1% acetic acid until the wash solution remained colorless. After drying, the dye was eluted with 10mM Tris-HCl, pH8.5, and absorbance was measured either at 550 or 492nm for lower and higher cell densities, respectively. The average absorbance of the sample representing the day 1 starting cell number (for 96-well plates) was subtracted from the absorbance values of the WE-treated cells.

IL-1β levels were determined with a commercial ELISA kit. The amount of IL-1β contained in the wound exudate added to the cells was subtracted from the total IL-β in the supernatants in order to determine the cytokine secreted by the cells.

All experiments were carried out in triplicate for each sample and concentration, and means ± standard deviation (SD) were used for the evaluation of the experiment. Results are expressed as percentage of control values for unstimulated cells.

### Example 1.2: Measuring the fibroblast-derived matrix formation (FDM) by primary fibroblast cells: measuring the fibroblast-derived matrix formation by primary fibroblast cells in the presence of a wound exudate sample obtained from a skin wound

HDF cells were seeded at 5000 cells/well on day -3 into 96-well tissue culture plates (1250 cells/well for 384-well plates), which had been pre-coated for 1 hour at 37°C with 0.2% gelatin solution (Sigma). When the cells reached confluence (= day 0), a matrix promoting supplement (vitamin C: 2-phospho-L-ascorbic acid trisodium salt, 100µg/ml; Sigma) was added together with test samples containing PDGF-BB, TGF-β1 or graded concentrations of compounds -/+ WE as described for the HDF proliferation assay. After 4 days, medium was replaced by fresh vitamin C- and stimulus- as well as compound-containing medium, maintaining the conditions initiated on day 0. TGF-β1 and PDGF-BB were included as positive controls to promote FDM formation and cell growth, respectively. After a total incubation time of 8 days, FDM production was measured in fixed cultures via SRB staining and evaluated as described above. In some cases, the experiment was stopped and evaluated already on day 4.

### Example 1.3:Keratinocyte proliferation assay: measuring the proliferation of keratinocyte cells in the presence of a wound exudate sample obtained from a skin wound

The HaCaT keratinocyte cell line was routinely cultured in DMEM containing 10% FCS, 2mM glutamine, and 100 U/ml penicillin/100 µg/ml streptomycin. The proliferation assay was carried out as described for HDF cells. Primary human keratinocytes were grown in KBM medium (Lonza) containing 0.06mM calcium and supplemented with growth factors (Lonza) on plastic coated with rat tail collagen (40µg/ml; Gibco) or gelatin (0.2%; Sigma). No antibiotics were used. The proliferation assay was carried out as described for HDF cells.

### Example 1.4: Primary human dermal microvascular endothelial cell proliferation assay: measuring the proliferation of endothelial cells in the presence of a wound exudate sample obtained from a skin wound

The primary human endothelial cells HMVEC-d- (Lonza) were cultured in EGM-2-MV BulletKit medium (Lonza). The proliferation assay was carried out as described for HDF cells.

### Example 1.5:Primary human macrophage stimulation assay

Primary human macrophages were differentiated from monocytes, which had been isolated from peripheral blood mononuclear cells (PBMC). PBMC were isolated from buffy coats obtained from the Red Cross, Vienna, using LymphoPrep (Technoclone). 30 ml of buffy concentrate was diluted 1:2 with PBS, gently underlayered with 15ml Lymphoprep in a 50ml falcon tube and centrifuged for 25 minutes at 1800 rpm at 21°C. The interphase was carefully transferred to a new falcon tube and filled up to 50ml with ice cold PBS. After another centrifugation step (10 minutes, 1200 rpm, 4°C), the cell pellet was washed 3 times with PBS, resuspended in RPMI medium containing 20% FCS and 10% DMSO and frozen in liquid nitrogen. Monocytes were generated from frozen aliquots using positive selection with the CD14 Beads-Kit (Miltenyi) on an autoMACS-Sorter (Miltenyi) according to the manufacturer's instructions.

For culture and differentiation into macrophages, monocytes were seeded at 3-5 x 10⁶ monocytes/well in 6-well-plates (Nunc) and incubated with 20 ng/ml M-CSF (R&D Systems) in RPMI supplemented with 10% FCS, 2mM glutamine, and 100 U/ml penicillin / 100 µg/ml streptomycin in a total volume of 5ml per well. After 2 days, 2ml of the supernatant were removed and replaced by 2.5ml/well of fresh medium containing 20ng/ml M-CSF. On the third day, microscopic examination revealed differentiation into adherent, frequently elongated cells.

The macrophages were harvested and re-seeded in 200µl or 50µl serum-free medium on 96-well or 384-well plates, respectively, combining cells with graded concentrations of test compounds in the absence or presence of various dilutions of sterile-filtered WE.

A combination of 100ng/ml LPS (Sigma) and 50ng/ml IFN-y (PeproTech) served as positive control for the induction of cytokine secretion. For negative control samples, medium was added instead of specific stimuli.

After 24 hours, the supernatants were transferred to fresh plates and frozen at -20°C for future cytokine analysis (IL-1α, IL-1β, IL-6, TNF-α). The cytokine concentration of the input WE was subtracted from the supernatant levels in order to calculate WE-induced cytokine stimulation.

Example 1.6: Human monocyte-dermal fibroblast co-cultures as in vitro models that reflect macrophage behavior in human skin:
measuring (a) the amount(s) of one or more M1 marker(s) and one or more M2 marker(s) in the supernatant of macrophages incubated with a wound exudate sample obtained from a skin wound, wherein the macrophages are in co-culture with fibroblasts, and (b) measuring the amount(s) and/or frequency distribution(s) of one or more M1 cell surface marker(s) and one or more M2 cell surface marker(s) on macrophages incubated with a wound exudate sample obtained from a skin wound, wherein the macrophages are in co-culture with fibroblasts, (c) measuring the expression level(s) of one or more M1 marker mRNA(s) and one or more M2 marker mRNA(s) in macrophages incubated with a wound exudate sample obtained from a skin wound, wherein the macrophages are in co-culture with fibroblasts, and (d) the amount(s) of one or more cytokine markers selected from IL-1alpha, IL-1beta and TNF-alpha in the supernatant of macrophages incubated with a wound exudate sample obtained from a skin wound, wherein the macrophages are in co-culture with fibroblasts
CD14⁺ monocytes, isolated from PBMC of healthy donors by magnetic bead separation were incubated either alone or in the presence of primary human dermal fibroblasts (CellNTec) or fibroblast-derived matrices (FDM). FDM had been generated from primary human dermal fibroblasts by a 3-week incubation with the growth supplements vitamin C or insulin and EGF (vitamin C: 2-phospho-L-ascorbic acid trisodium salt, 100 µg/ml; human EGF, 5ng/ml; human insulin, 5 µg/ml). Alternatively, fibroblast monolayer cultures can be used as well. After 4 days to a week to allow for macrophage differentiation in the presence or absence of M-CSF (25 ng/ml), the cultures were stimulated overnight with graded concentrations of test compounds in the absence or presence of various dilutions of sterile-filtered WE. IFN-g (50ng/ml), LPS (100 ng/ml) and IL-4 (25 ng/ml) or combinations thereof served as controls for M1 and M2 macrophage induction. For negative control samples, medium was added instead of specific stimuli. WE were added to the culture medium for overnight stimulation at dilutions ranging from 1:25 to 1:100.

Supernatants were harvested and frozen for cytokine determination by ELISA, and cells were harvested and subjected to FACS analysis, gating on the monocyte population. Geometric means or mean fluorescence intensities (MFI) were used to quantify surface marker expression.

Specific mRNA levels are determined as ratios compared to a housekeeping gene; the values obtained are "expression relative to housekeeping gene".

There are 2 possibilities for evaluation: a) the % of cells positive for a given marker within a population, which is the most commonly used readout in FACS analysis, or b) the quantity of cell surface expression (as surrogate for the number of labelled molecules on the cell surface per individual cell), as measured by the mean fluorescence intensity.

The following readouts were used:
FACS: CD38, CD64 and CD197 for M1 macrophages, CD200 receptor (CD200R), CD206 and CD209 for M2 macrophages, CD163 as a marker of macrophage differentiation. Ratios of M1/M2 cell surface marker expression were calculated.

ELISA: CXCL10 and IL-23p19 for M1 macrophages and CCL22 and CCL18 as M2 macrophage markers, IL-1alpha, IL-1beta and TNF-alpha as pro-inflammatory markers indicative of an M1 phenotype.

mRNA: CD38, CD64 CD38, CD64 and CD197 for M1 macrophages, CD200 receptor (CD200R), CD206 and CD209 for M2 macrophages, CD163 as a marker of macrophage differentiation.

### Example 1.7:Determination of CCL18

CCL18 in WE and in macrophage supernatants was determined in F96 Maxisorp Nunc Immuno plates (Nunc, #439454) using the hCCL18/PARC DuoSet ELISA Kit from R&D Systems (# DY394) according to the manufacturer's instructions. Enzyme reaction and measurement were performed as described for IL-1α.

### Example 1.8:Analysis of macrophage surface markers by flow cytometry

Cells were harvested and resuspended in FACS buffer (PBS containing 2% FCS). Unspecific antibody binding was prevented by incubation with human Trustain FCR blocking solution (Biolegend, #422302) on ice for 10 minutes. The following fluorchrome-conjugated antibodies from eBioscience (now ThermoFisher Scientific) were used to detect specific surface markers by staining on ice for 30 minutes: CD38-PerCPeFluor710 (#46-0388-42), CD197-APC (#17-1979-42), CD206-AF488 (#53-2069-42), CD209-PerCP Cy5.5 (#45-2099-42). Co-staining with CD45 eFluor (#506 69-0459-42) was used to distinguish macrophages from primary human fibroblasts when analyzed from co-cultures. After washing cells with FACS buffer, they were fixed with 1% paraformaldehyde in PBS and stored at 4°C in the dark until data were acquired on a Gallios flow cytometer from Beckman Coulter and analyzed with the Kaluza analysis software 1.3.

### Example 1.9: Immunolocalization of alpha-smooth muscle actin

After fixation with paraformaldehyde, the cells were incubated for 1 hour at room temperature with a monoclonal mouse-anti-human antibody against alpha-smooth muscle actin (a-SMA) from e-Bioscience, washed 3 times with PBS and developed with an Alexa Fluor-labelled donkey-anti-mouse IgG (Molecular Probes). The cells were washed 3 times with PBS and examined in a Zeiss ZEISS Observer.Z1 microscope, using the software AxioVision 48.

### Example 1.10: Test Compounds

Low molecular weight compounds (see list in Table 1) were dissolved in DMSO (Bioreagent for cell culture, Sigma) at 10mM or 100mM and diluted at least 1:1000 in medium for cellular assays (final DMSO concentration ≤0.1%). Compounds were typically tested in half-logarithmic dilution series (1:3.33), starting at 10µM or 100µM as the highest compound concentration.
Mifepristone, a glucocorticoid and progesterone receptor antagonist, was titrated to reveal potential toxic effects on cells and then used at the well tolerated concentration of 1 µM to counteract glucocorticoid- and progesterone-type compounds (dexamethasone, prednisolone, medroxyprogesterone, etc.). Protein growth factors (see list in Table 1) were dissolved according to the manufacturers' recommendations and used at final concentrations ranging from 0.02 to 1000 ng/ml.

Cells were incubated with compounds for 72 hours in proliferation assays and up to 8 days for FDM assay (refreshed after 4 days). When compounds were tested for their effect on WE stimulation, the incubation of cells with compounds was started and ended simultaneously with WE-incubation.

**Table 1: List of low molecular weight compounds and protein growth factors for cellular assays**

| **Compound** | **Source** |
|---|---|
| | |
| Crenolanib | MedChem Express |
| Dexamethasone 21-acetate | Sigma-Aldrich |
| Prednisolone | Sigma-Aldrich |
| Hydrocortisone | Lonza |
| Medroxyprogesterone | MedChem Express |
| Progesterone | MedChem Express |
| Mifepristone | MedChem Express |
| Veliparib dihydrochloride | MedChem Express |
| Olaparib | MedChem Express |
| AZD-2461 | MedChem Express |
| Niraparib | MedChem Express |
| Rucaparib phosphate | MedChem Express |
| Talazoparib | MedChem Express |
| PJ-34 hydrochloride | Cayman Chemical |
| 1,5-Isoquinolinediol | Cayman Chemical |
| 3-Aminobenzamide | Cayman Chemical |
| BGP-15 | Cayman Chemical |
| PDGF | Tonbo Biosciences |
| bFGF | eBioscience |
| TGFbeta | eBioscience |
| HGF | R&D Systems |
| hEGF | Gibco |
| hIGF-1 | Gibco |
| FGF10 (KGF2) | R&D Systems |
| hVEGF | Gibco |

## Claims

1. A poly-ADP-ribose polymerase (PARP) inhibitor for use in the prevention and/or treatment of impaired skin wound healing in a subject,
wherein the subject:
(i) is a subject treated with at least one glucocorticoid, and/or
(ii) is a subject to which a pharmaceutical, nutritional supplement or dietary supplement comprising ascorbic acid or a pharmaceutically acceptable salt thereof is administered, and/or
(iii) is a subject treated with at least one protein growth factor.

2. A poly-ADP-ribose polymerase (PARP) inhibitor in combination with one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
for use in the treatment of impaired skin wound healing in a subject.

3. The poly-ADP-ribose polymerase (PARP) inhibitor for use of claim 1 or 2, wherein the subject suffers from at least one comorbidity associated with impaired skin wound healing, and/or wherein the subject is treated with at least one glucocorticoid for treating and/or preventing at least one comorbidity associated with impaired skin wound healing and/or wherein the subject is treated with at least one protein growth factor for treating or preventing impaired wound healing.

4. The poly-ADP-ribose polymerase (PARP) inhibitor for use of any of claims 1 to 3, wherein the skin wound is selected from a wound of a diabetic patient, a skin wound which is infected by at least one microorganism, an ischemic wound, a wound in a patient suffering from deficient blood supply or venous stasis, an ulcer, such as a diabetic ulcer, venous ulcer, arterial ulcer, such as ulcus cruris arteriosum, mixed ulcer, or pressure ulcer, a neuropathic wound, ulcus cruris, surgical wound, burn, dehiscence, neoplastic ulcer, a bullous skin disease, such as epidermolysis bullosa, and rare ulcer.

5. The poly-ADP-ribose polymerase (PARP) inhibitor for use of any of claims 1 to 4, wherein:
(i) the skin wound is selected from a wound of a diabetic patient and/or a diabetic ulcer, and/or
(ii) the subject is treated with at least one glucocorticoid by systemic or cutaneous administration, and/or
(iii) the subject is treated with at least one protein growth factor by systemic or cutaneous administration.

6. The poly-ADP-ribose polymerase (PARP) inhibitor for use of any of claims 1 to 5, wherein the subject:
(i) has undergone transplantation of a graft, and/or
(ii) obtains immunosuppressive therapy, and/or
(iii) is treated with at least one immunosuppressive drug, such as a glucocorticoid or a calcineurin inhibitor,
and optionally suffers from diabetes.

7. A poly-ADP-ribose polymerase (PARP) inhibitor in combination with one, two or three of the following (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor,
for use as a medicament.

8. A kit, or kit-of-parts, comprising:
(a) a pharmaceutical composition comprising a poly-ADP-ribose polymerase (PARP) inhibitor,
and one, two or three of the following (b) to (d):
(b) a pharmaceutical composition comprising a glucocorticoid,
(c) ascorbic acid or a pharmaceutically acceptable salt thereof,
(d) a pharmaceutical composition comprising a protein growth factor.

9. The poly-ADP-ribose polymerase (PARP) inhibitor for use of any of claims 1 to 7, or the kit or kit-of-parts of claim 8, wherein:
(i) the (PARP) inhibitor is formulated for systemic, preferably oral or intravenous administration, or wherein the (PARP) inhibitor is formulated for local administration, in particular for topical, mucosal or subcutaneous administration, and/or
(ii) the glucocorticoid is formulated for systemic, preferably oral or intravenous administration, or wherein the glucocorticoid is formulated for local administration, in particular for topical, mucosal or subcutaneous administration, and/or
(iii) ascorbic acid or pharmaceutically acceptable salt thereof is formulated for systemic, preferably oral or intravenous administration, or wherein the ascorbic acid or pharmaceutically acceptable salt thereof is formulated for local administration, in particular for topical, mucosal or subcutaneous administration, and/or
(iv) the protein growth factor is formulated for systemic, preferably oral or intravenous administration, or wherein the protein growth factor is formulated for local administration, in particular for perilesional and/or intralesional, topical, mucosal or subcutaneous administration.

10. The PARP inhibitor for use of any of claims 1 to 7 or 9, or the kit or kit-of-parts of claim 8 or 9, wherein the PARP inhibitor inhibits PARP 1 and optionally PARP2, and/or wherein the PARP inhibitor is selected from, Veliparib, Talazoparib, Olaparib (AZD-2281), Rucaparib, AZD-2461, Iniparib, and PJ-34, or a pharmaceutically acceptable salt thereof.

11. The poly-ADP-ribose polymerase (PARP) inhibitor for use of any of claims 1 to 7, or 9 to 10, or the kit or kit-of-parts of any of claims 8 to 10, wherein
- the glucocorticoid is selected from the group consisting of cortisol, cortisone acetate, prednisone, prednisolone, methylprednisolone, chloroprednisone, cloprednol, difluprednate, fludrocortisone acetate, fluocinolone, fluperolone, fluprednisolone, loteprednol, prednicarbate, tixocortol, triamcinolone, triamcinolone acetonide, dexamethasone, betamethasone, beclometasone, deoxycorticosterone acetate, alclometasone, clobetasol, clobetasone, clocortolone, desoximetasone, diflorasone, difluocortolone, fluclorolone, flumetasone, fluocortin, fluocortolone, fluprednidene, fluticasone, fluticasone furoate, halometasone, meprednisone, mometasone, mometasone furoate, paramethasone, prednylidene, rimexolone, ulobetasol, amcinonide, budesonide, ciclesonide, deflazacort, desonide, formocortal, fluclorolone acetonide, fludroxycortide, flunisolide, fluocinolone acetonide, fluocinonide, halcinonide, hydroxymethylprogesterone, and medroxyprogesterone or a pharmaceutically acceptable salt thereof, and/or
- the protein growth factor is a human protein growth factor, preferably wherein the protein growth factor is selected from a platelet derived growth factor (PDGF), transforming growth factor beta (TGF-β), basic fibroblast growth factor (bFGF), keratinocyte growth factor (KGF), epidermal growth factor (EGF), Insulin-like growth factor 1 (IGF-1), vascular endothelial growth factor (VEGF) and hepatocyte growth factor (HGF).

12. The poly-ADP-ribose polymerase (PARP) inhibitor for use of any of claims 1 to 7, or 9 to 11, wherein the subject is identified to be responsive to the treatment of impaired skin wound healing by performing steps i) and/or ii):
i) measuring the proliferation of primary fibroblast cells in the presence of:
(1) a wound exudate sample or wound biofilm sample, obtained from the skin wound of said subject, and
(2) the following compounds: a PARP inhibitor and one, two or three of (i) to (iii):
(i) a glucocorticoid,
(ii) ascorbic acid or a pharmaceutically acceptable salt thereof,
(iii) a protein growth factor;
ii) measuring the fibroblast-derived matrix formation by primary fibroblast cells in the presence of:
(1) a wound exudate sample, or wound biofilm sample, obtained from the skin wound of said subject, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor.

13. The poly-ADP-ribose polymerase (PARP) inhibitor for use of claim 12, wherein the subject is identified to be responsive to the treatment of impaired skin wound healing in case the value of proliferation of primary fibroblast cells measured in step i) and/or the value of the fibroblast-derived matrix formation by primary fibroblast cells measured in step ii) is at least 20% above a control value established in the absence of the compounds of (2).

14. An *in vitro* method of identifying a subject suffering from impaired skin wound healing to be responsive to the treatment with a poly-ADP-ribose polymerase (PARP) inhibitor in combination with one, two or three of (i) to (iii):
(i) a pharmaceutical composition comprising a glucocorticoid,
(ii) ascorbic acid or a pharmaceutically acceptable salt thereof,
(iii) a pharmaceutical composition comprising a protein growth factor;
comprising performing steps i) and/or ii):
i) measuring the proliferation of primary fibroblast cells in the presence of:
(1) a wound exudate sample, or wound biofilm sample, obtained from the skin wound of said subject, and
(2) the following compounds: a PARP inhibitor and one, two or three of (i) to (iii):
(i) a glucocorticoid,
(ii) ascorbic acid or a pharmaceutically acceptable salt thereof,
(iii) a protein growth factor;
ii) measuring the fibroblast-derived matrix formation by primary fibroblast cells in the presence of:
(1) a wound exudate sample, or wound biofilm sample, obtained from the skin wound of said subject, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor;
wherein the subject is identified to be responsive to the treatment of impaired skin wound healing in case the value of proliferation of primary fibroblast cells measured in step i) and/or the value of the fibroblast-derived matrix formation by primary fibroblast cells measured in step ii) is at least 20% above a control value established in the absence of the compounds of (2).

15. The poly-ADP-ribose polymerase (PARP) inhibitor for use of claim 12, or the *in vitro* method of claim 14, wherein in addition step iiia) and/or one, two, three or four of the following steps iiib) to iiie) are performed:
iiia) measuring the proliferation of keratinocyte cells in the presence of:
(1) a wound exudate sample, or wound biofilm sample, obtained from the skin wound of said subject, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor,
iiib) measuring the amount(s) of one or more M1 marker(s) and one or more M2 marker(s) in the supernatant of macrophages incubated with
(1) a wound exudate sample or wound biofilm sample obtained from said skin wound, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor,
wherein the macrophages are in co-culture with fibroblasts, and
wherein the one or more M1 markers are selected from CXCL10 and IL-23p19, and the one or more M2 markers are selected from CCL22 and CCL18,
iiic) measuring the amount(s) and/or frequency distribution(s) of one or more M1 cell surface marker(s) and one or more M2 cell surface marker(s) on macrophages incubated with
(1) a wound exudate sample or wound biofilm sample obtained from said skin wound, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor,
wherein the macrophages are in co-culture with fibroblasts, and
wherein the one or more M1 cell surface markers are selected from CD38, CD64 and CD197, and wherein the one or more M2 cell surface markers are selected from CD200 receptor, CD206 and CD209,
iiid) measuring the expression level(s) of one or more M1 marker mRNA(s) and one or more M2 marker mRNA(s) in macrophages incubated with
(1) a wound exudate sample or wound biofilm sample obtained from said skin wound, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor,
wherein the macrophages are in co-culture with fibroblasts, and
wherein the one or more M1 marker mRNA(s) are selected from CD38, CD64, CD197, CXCL10 and IL-23p19, and the one or more M2 marker mRNA(s) are selected from CD200 receptor (CD200R), CD206, CD209, CCL22 and CCL18,
iiie) measuring the amount(s) of one or more cytokine markers in the supernatant of macrophages incubated
(1) with a wound exudate sample or wound biofilm sample obtained from said skin wound, and
(2) the following compounds: a PARP inhibitor and one, two or three of (i) to (iii):
(i) a glucocorticoid
(ii) ascorbic acid or a pharmaceutically acceptable salt thereof,
(iii) a protein growth factor,
wherein the macrophages are in co-culture with fibroblasts, and wherein the one or more cytokine markers are selected from IL-1alpha, IL-1beta and TNF-alpha,
and wherein the subject is identified to be responsive to the treatment of impaired skin wound healing, in case the value of proliferation of primary fibroblast cells measured in step i) and/or the value of the fibroblast-derived matrix formation by primary fibroblast cells measured in step ii) and/or the value of the proliferation of keratinocyte cells in step iiia) is at least 20% above a control value established in the absence of the compounds of (2), and/or in case one or more of the following applies:
- the ratio of amount(s) of one or more M1 marker(s) to the amount(s) of one or more M2 marker(s) obtained in iiib) is/are below a control value established in the absence of the compounds of (2),
- the ratio of amount(s) and/or frequency distribution(s) of one or more M1 cell surface marker(s) to the amount(s) and/or frequency distribution(s) of one or more M2 cell surface marker(s) obtained in iiic) is/are below a control value established in the absence of the compounds of (2),
- the ratio of expression level(s) of one or more M1 marker mRNA(s) to the expression level(s) of one or more M2 marker mRNA(s) obtained in iiid) is/are below a control value established in the absence of the compounds of (2),
- the value obtained in iiie) is below a control value established in the absence of the compounds of (2).

## Patentansprüche

1. Poly-ADP-Ribose-Polymerase (PARP)-Inhibitor zur Verwendung bei der Prävention und/oder Behandlung von beeinträchtigter Hautwundheilung in einem Subjekt, wobei das Subjekt:
(i) ein Subjekt ist, das mit mindestens einem Glukokortikoid behandelt wird, und/oder
(ii) ein Subjekt ist, dem ein pharmazeutisches, Nahrungsergänzungs- oder diätisches Ergänzungsmittel umfassend Ascorbinsäure oder einem pharmazeutisch akzeptablen Salz davon, verabreicht wird, und/oder
(iii) ein Subjekt ist, das mit mindestens einem Proteinwachstumsfaktor behandelt wird.

2. Poly-ADP-Ribose-Polymerase (PARP)-Inhibitor in Kombination mit einem, zwei oder drei der folgenden (i) bis (iii):
(i) eine pharmazeutische Zusammensetzung, umfassend ein Glukokortikoid,
(ii) Ascorbinsäure oder ein pharmazeutisch akzeptables Salz davon,
(iii) eine pharmazeutische Zusammensetzung umfassend einen Proteinwachstumsfaktor, zur Verwendung bei der Behandlung beeinträchtigter Hautwundheilung in einem Subjekt.

3. Poly-ADP-Ribose-Polymerase (PARP)-Inhibitor zur Verwendung nach Anspruch 1 oder 2, wobei das Subjekt an mindestens einer Komorbidität leidet, die mit beeinträchtigter Hautwundheilung assoziiert ist, und/oder wobei das Subjekt mit mindestens einem Glukokortikoid zum Behandeln und/oder Vorbeugen mindestens einer Komorbidität behandelt wird, die mit beeinträchtigter Hautwundheilung assoziiert ist, und/oder wobei das Subjekt mit mindestens einem Proteinwachstumsfaktor zum Behandeln oder Vorbeugen beeinträchtigter Wundheilung behandelt wird.

4. Poly-ADP-Ribose-Polymerase (PARP)-Inhibitor zur Verwendung nach einem beliebigen der Ansprüche 1 bis 3, wobei die Hautwunde ausgewählt ist aus einer Wunde eines diabetischen Patienten, einer Hautwunde, die mit mindestens einem Mikroorganismus infiziert ist, einer ischämischen Wunde, einer Wunde bei einem Patienten, der an mangelnder Blutversorgung oder venöser Stase leidet, einem Ulkus, wie z.B. einem diabetischen Ulkus, einem venösen Ulkus, einem arteriellen Ulkus, wie z.B. ein Ulcus cruris arteriosum, gemischtem Ulkus oder einem Druckulkus, einer neuropathischen Wunde, Ulcus cruris, Operationswunde, Verbrennung, Dehiszenz, neoplastischem Ulkus, einer bullösen Hauterkrankung, wie z.B. epidermolysis bullosa, und seltenem Ulkus.

5. Poly-ADP-Ribose-Polymerase (PARP)-Inhibitor zur Verwendung nach einem beliebigen der Ansprüche 1 bis 4, wobei:
(i) die Hautwunde ausgewählt ist aus einer Wunde eines diabetischen Patienten und/oder einem diabetischen Ulkus, und/oder
(ii) das Subjekt mit mindestens einem Glukokortikoid durch systemische oder kutane Verabreichung behandelt wird, und/oder
(iii) das Subjekt mit mindestens einem Proteinwachstumsfaktor durch systemische oder kutane Verabreichung behandelt wird.

6. Poly-ADP-Ribose-Polymerase (PARP)-Inhibitor zur Verwendung nach einem beliebigen der Ansprüche 1 bis 5, wobei das Subjekt:
(i) sich einer Transplantation eines Transplantats unterzogen hat, und/oder
(ii) eine immunsuppressive Therapie erhält, und/oder
(iii) mit mindestens einem immunsuppressiven Medikament, wie z.B. einem Glukokortikoid oder einem Calcineurin-Inhibitor, behandelt wird,
und optional an Diabetes leidet.

7. Poly-ADP-Ribose-Polymerase (PARP)-Inhibitor in Kombination mit einem, zwei oder drei der folgenden (i) bis (iii):
(i) eine pharmazeutische Zusammensetzung, umfassend ein Glukokortikoid,
(ii) Ascorbinsäure oder ein pharmazeutisch akzeptables Salz davon,
(iii) eine pharmazeutische Zusammensetzung, umfassend einen Proteinwachstumsfaktor,
zur Verwendung als ein Medikament.

8. Kit oder kit-of-parts, umfassend:
(a) eine pharmazeutische Zusammensetzung, umfassend einen Poly-ADP-Ribose-Polymerase (PARP)-Inhibitor,
und einen, zwei oder drei der folgenden (b) bis (d):
(b) eine pharmazeutische Zusammensetzung, umfassend ein Glukokortikoid,
(c) Ascorbinsäure oder ein pharmazeutisch akzeptables Salz davon,
(d) eine pharmazeutische Zusammensetzung, umfassend einen Proteinwachstumsfaktor.

9. Poly-ADP-Ribose-Polymerase (PARP)-Inhibitor zur Verwendung nach einem beliebigen der Ansprüche 1 bis 7 oder Kit oder Kit-of-parts nach Anspruch 8, wobei:
(i) der (PARP)-Inhibitor formuliert ist zur systemischen, vorzugsweise oralen oder intravenösen Verabreichung, oder wobei der (PARP)-Inhibitor formuliert ist zur lokalen Verabreichung, insbesondere zur topischen, mukosalen oder subkutanen Verabreichung, und/oder
(ii) das Glukokortikoid formuliert ist zur systemischen, vorzugsweise oralen oder intravenösen Verabreichung, oder wobei das Glukokortikoid zur lokalen Verabreichung, insbesondere zur topischen, mukosalen oder subkutanen Verabreichung, und/oder
(iii) Ascorbinsäure oder ein pharmazeutisch akzeptables Salz davon formuliert ist zur systemischen, vorzugsweise oralen oder intravenösen Verabreichung, oder wobei die Ascorbinsäure oder das pharmazeutisch akzeptable Salz davon formuliert ist zur lokalen Verabreichung, insbesondere zur topischen, mukosalen oder subkutanen Verabreichung, und/oder
(iv) der Proteinwachstumsfaktor formuliert ist zur systemischen, vorzugsweise oralen oder intravenösen Verabreichung, oder wobei der Proteinwachstumsfaktor formuliert ist zur lokalen Verabreichung, insbesondere zur perilesionalen und/oder intralesionalen, topischen, mukosalen oder subkutanen Verabreichung.

10. PARP-Inhibitor zur Verwendung nach einem beliebigen der Ansprüche 1 bis 7 oder 9 oder Kit oder Kit-of-parts nach Anspruch 8 oder 9, wobei der PARP-Inhibitor PARP 1 inhibiert und optional PARP2, und/oder wobei der PARP-Inhibitor ausgewählt ist aus Veliparib, Talazoparib, Olaparib (AZD-2281), Rucaparib, AZD-2461, Iniparib und PJ-34, oder einem pharmazeutisch akzeptablen Salz davon.

11. Poly-ADP-Ribose-Polymerase (PARP)-Inhibitor zur Verwendung nach einem beliebigen der Ansprüche 1 bis 7 oder 9 bis 10 oder dem Kit oder Kit-of-parts nach einem beliebigen der Ansprüche 8 bis 10, wobei
- das Glukokortikoid ausgewählt ist aus der Gruppe bestehend aus Kortisol, Kortisonacetat, Prednison, Prednisolon, Prednisolon, Methylprednisolon, Chlorprednison, Cloprednol, Difluprednat, Fludrocortisonacetat, Fluocinolon, Fluperolon, Fluprednisolon, Loteprednol, Prednicarbat, Tixocortol, Triamcinolon, Triamcinolonacetonid, Dexamethason, Betamethason, Beclometason, Deoxycorticosteronacetat, Alclometason, Clobetasol, Clobetason, Clocortolon, Desoximetason, Diflorason, Difluocortolon, Fluclorolon, Flumetason, Fluocortin, Fluocortolon, Flupredniden, Fluticason, Fluticasonfuroat, Halometason, Meprednison, Mometason, Mometasonfuroat, Paramethason, Prednyliden, Rimexolon, Ulobetasol, Amcinonid, Budesonid, Ciclesonid, Deflazacort, Desonid, Formocortal, Fluclorolonacetonid, Fludroxycortid, Flunisolid, Fluocinolonacetonid, Fluocinonid, Halcinonid, Hydroxymethylprogesteron und Medroxyprogesteron oder einem pharmazeutisch akzeptablen Salz davon, und/oder
- der Proteinwachstumsfaktor ein humaner Proteinwachstumsfaktor ist, vorzugsweise wobei der Proteinwachstumsfaktor ausgewählt ist aus einem von Blutplättchen abgeleiteten Wachstumsfaktor (PDGF), dem transformierenden Wachstumsfaktor beta (TGF-β), dem basischen Fibroblasten-Wachstumsfaktor (bFGF), dem Keratinozyten-Wachstumsfaktor (KGF), dem epidermalen Wachstumsfaktor (EGF), dem insulinähnlichen Wachstumsfaktor 1 (IGF-1), dem vaskulären endothelialen Wachstumsfaktor (VEGF) und dem Hepatozyten-Wachstumsfaktor (HGF).

12. Poly-ADP-Ribose-Polymerase (PARP)-Inhibitor zur Verwendung nach einem beliebigen der Ansprüche 1 bis 7 oder 9 bis 11, wobei das Subjekt identifiziert wurde auf die Behandlung beeinträchtigter Hautwundheilung anzusprechen, durch Durchführung der Schritte i) und/oder ii):
i) Messen der Proliferation von primären Fibroblastenzellen in Gegenwart von:
(1) einer Wundexsudatprobe oder Wundbiofilmprobe, erhalten aus der Hautwunde des Subjekts, und
(2) die folgenden Verbindungen: ein PARP-Inhibitor und eine, zwei oder drei von (i) bis (iii):
(i) ein Glukokortikoid,
(ii) Ascorbinsäure oder ein pharmazeutisch akzeptables Salz davon,
(iii) ein Proteinwachstumsfaktor;
ii) Messen der von Fibroblasten abgeleiteten Matrixbildung durch primäre Fibroblastenzellen in Gegenwart von:
(1) einer Wundexsudatprobe oder Wundbiofilmprobe, erhalten aus der Hautwunde des Subjekts, und
(2) die folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor.

13. Poly-ADP-Ribose-Polymerase (PARP)-Inhibitor zur Verwendung nach Anspruch 12, wobei das Subjekt identifiziert wurde ansprechend auf die Behandlung beeinträchtigter Hautwundheilung zu sein, falls der in Schritt i) gemessene Wert der Proliferation primärer Fibroblastenzellen und/oder der in Schritt ii) gemessene Wert der von Fibroblasten abgeleiteten Matrixbildung durch primäre Fibroblastenzellen mindestens 20% über einem Kontrollwert liegt, der in Abwesenheit der Verbindungen von (2) etabliert wurde.

14. In-vitro-Verfahren zum Identifizieren eines Subjekts, das an beeinträchtigter Hautwundheilung leidet, als ansprechend auf die Behandlung mit einem Poly-ADP-Ribose-Polymerase (PARP)-Inhibitor in Kombination mit einer, zwei oder drei der von (i) bis (iii):
(i) eine pharmazeutische Zusammensetzung, umfassend ein Glukokortikoid,
(ii) Ascorbinsäure oder ein pharmazeutisch akzeptables Salz davon,
(iii) eine pharmazeutische Zusammensetzung, umfassend einen Proteinwachstumsfaktor;
umfassend die Durchführung der Schritte i) und/oder ii):
i) Messen der Proliferation von primären Fibroblastenzellen in Gegenwart von:
(1) einer Wundexsudatprobe oder Wundbiofilmprobe, erhalten aus der Hautwunde des Subjekts, und
(2) die folgenden Verbindungen: ein PARP-Inhibitor und eine, zwei oder drei von (i) bis (iii):
(i) ein Glukokortikoid,
(ii) Ascorbinsäure oder ein pharmazeutisch akzeptables Salz davon,
(iii) ein Proteinwachstumsfaktor;
ii) Messen der von Fibroblasten abgeleiteten Matrixbildung durch primäre Fibroblastenzellen in Gegenwart von:
(1) einer Wundexsudatprobe oder Wundbiofilmprobe, erhalten aus der Hautwunde des Subjekts, und
(2) die folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor;
wobei das Subjekt als auf die Behandlung beeinträchtigter Hautwundheilung ansprechend identifiziert wird, falls der in Schritt i) gemessene Wert der Proliferation von primären Fibroblastenzellen und/oder der in Schritt ii) gemessene Wert der von Fibroblasten abgeleiteten Matrixbildung durch primäre Fibroblastenzellen mindestens 20% über einem Kontrollwert liegt, der in Abwesenheit der Verbindungen von (2) etabliert wurde.

15. Poly-ADP-Ribose-Polymerase (PARP)-Inhibitor zur Verwendung nach Anspruch 12 oder in-vitro-Verfahren nach Anspruch 14, wobei zusätzlich Schritt iiia) und/oder einer, zwei, drei oder vier der folgenden Schritte iiib) bis iiie) durchgeführt werden:
iiia) Messen der Proliferation von Keratinozytenzellen in Gegenwart von:
(1) einer Wundexsudatprobe oder einer Wundbiofilmprobe, erhalten aus der Hautwunde des Subjekts, und
(2) die folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor,
iiib) Messen der Menge(n) eines oder mehrerer M1-Marker(s) und eines oder mehrere M2-Marker(s) im Überstand von Makrophagen, die inkubiert wurden mit
(1) einer Wundexsudatprobe oder Wundbiofilmprobe, erhalten aus der Hautwunde, und
(2) den folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor,
wobei die Makrophagen sich in Co-Kultur mit Fibroblasten befinden, und
wobei der eine oder die mehreren M1-Marker ausgewählt ist/sind aus CXCL10 und IL-23p19 und der eine oder die mehreren M2-Marker ausgewählt ist/sind aus CCL22 und CCL18,
iiic) Messen der Menge(n) und/oder Häufigkeitsverteilung(en) eines oder mehrerer M1-Zelloberflächenmarker(s) und eines oder mehrerer M2-Zelloberflächenmarker(s) auf Makrophagen, die inkubiert werden mit
(1) einer Wundexsudatprobe oder Wundbiofilmprobe, erhalten aus der Hautwunde, und
(2) den folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor,
wobei die Makrophagen sich in Co-Kultur mit Fibroblasten befinden, und
wobei der eine oder mehrere M1-Zelloberflächenmarker ausgewählt ist/sind aus CD38, CD64 und CD197, und wobei der eine oder mehrere M2-Zelloberflächenmarker ausgewählt ist/sind aus CD200-Rezeptor, CD206 und CD209,
iiid) Messen des/der Expressionslevel(s) eines oder mehrerer M1-Marker-mRNA(s) und eines oder mehrerer M2-Marker-mRNA(s) in Makrophagen, die inkubiert werden mit
(1) einer Wundexsudatprobe oder Wundbiofilmprobe, erhalten aus der Hautwunde, und
(2) den folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor,
wobei die Makrophagen sich in Co-Kultur mit Fibroblasten befinden, und
wobei der/die eine oder mehrere M1-Marker-mRNA(s) ausgewählt sind aus CD38, CD64, CD197, CXCL10 und IL-23p19, und der/die eine oder mehrere M2-Marker-mRNA(s) ausgewählt sind aus CD200-Rezeptor (CD200R), CD206, CD209, CCL22 und CCL18,
iiie) Messen der Menge(n) eines oder mehrerer Zytokinmarker(s) im Überstand von Makrophagen, die inkubiert werden mit
(1) einer Wundexsudatprobe oder Wundbiofilmprobe, erhalten aus der Hautwunde, und
(2) den folgenden Verbindungen: ein PARP-Inhibitor und eine, zwei oder drei von (i) bis (iii):
(i) ein Glukokortikoid
(ii) Ascorbinsäure oder ein pharmazeutisch akzeptables Salz davon,
(iii) ein Proteinwachstumsfaktor,
wobei die Makrophagen sich in Co-Kultur mit Fibroblasten befinden, und
wobei der/die eine oder die mehreren Zytokinmarker ausgewählt ist/sind aus
IL-1alpha, IL-1beta und TNF-alpha,
und
wobei das Subjekt als auf die Behandlung beeinträchtigter Hautwundheilung ansprechend identifiziert wird, falls der in Schritt i) gemessene Wert der Proliferation von primären Fibroblastenzellen und/oder der in Schritt ii) gemessene Wert der von Fibroblasten abgeleiteten Matrixbildung durch primäre Fibroblastenzellen und/oder der Wert der Proliferation von Keratinozytenzellen in Schritt iiia) mindestens 20% über einem Kontrollwert liegt, der in Abwesenheit der Verbindungen von (2) etabliert wurde, und/oder falls eine oder mehrere der folgenden zutrifft:
- das Verhältnis der Menge(n) des einen oder der mehreren M1-Marker(s) zu der/den Menge(n) des einen oder der mehreren M2-Marker(s), der/die in iiib) erhalten wurde(n), liegt unter einem Kontrollwert, der in Abwesenheit der Verbindungen von (2) etabliert wurde,
- das Verhältnis der Menge(n) und/oder Häufigkeitsverteilung(en) des einen oder der mehreren M1-Zelloberflächenmarker(s) zu der Menge(n) und/oder Häufigkeitsverteilung(en) des einen oder der mehreren M2-Zelloberflächenmarker(s), die in iiic) erhalten wurde, liegt unter einem Kontrollwert, der in Abwesenheit der Verbindungen von (2) etabliert wurde,
- das Verhältnis des/der Expressionslevel(s) des einen oder der mehreren M1-Marker-mRNA(s) zu dem/den Expressionslevel(s) des einen oder der mehreren M2-Marker-mRNA(s), die in iiid) erhalten wurde, liegt unter einem Kontrollwert, der in Abwesenheit der Verbindungen von (2) etabliert wurde,
- der in iiie) erhaltene Wert liegt unter einem Kontrollwert, der in Abwesenheit der Verbindungen von (2) etabliert wurde.

## Revendications

1. Inhibiteur de la poly(ADP-ribose) polymérase (PARP) pour une utilisation dans la prévention et/ou le traitement d'une mauvaise cicatrisation de plaies cutanées chez un sujet,
dans lequel le sujet :
(i) est un sujet traité avec au moins un glucocorticoïde, et/ou
(ii) est un sujet auquel est administré un produit pharmaceutique, un complément nutritionnel ou un complément alimentaire comprenant l'acide ascorbique ou un sel pharmaceutiquement acceptable de celui-ci, et/ou
(iii) est un sujet traité avec au moins un facteur de croissance protéique.

2. Inhibiteur de la poly(ADP-ribose) polymérase (PARP) en combinaison avec un, deux ou trois des éléments (i) à (iii) suivants :
(i) une composition pharmaceutique comprenant un glucocorticoïde,
(ii) l'acide ascorbique ou un sel pharmaceutiquement acceptable de celui-ci,
(iii) une composition pharmaceutique comprenant un facteur de croissance protéique,
pour une utilisation dans le traitement d'une mauvaise cicatrisation de plaies cutanées chez un sujet.

3. Inhibiteur de la poly(ADP-ribose) polymérase (PARP) pour une utilisation de la revendication 1 ou 2, dans lequel le sujet souffre d'au moins une comorbidité associée à une mauvaise cicatrisation de plaies cutanées, et/ou dans lequel le sujet est traité avec au moins un glucocorticoïde pour traiter et/ou prévenir au moins une comorbidité associée à une mauvaise cicatrisation de plaies cutanées et/ou dans lequel le sujet est traité avec au moins un facteur de croissance protéique pour traiter ou prévenir une mauvaise cicatrisation de plaies.

4. Inhibiteur de la poly(ADP-ribose) polymérase (PARP) pour une utilisation de l'une des revendications 1 à 3, dans lequel la plaie cutanée est choisie parmi une plaie d'un patient diabétique, une plaie cutanée qui est infectée par au moins un microorganisme, une plaie ischémique, une plaie chez un patient souffrant d'un apport sanguin déficient ou d'une stase veineuse, un ulcère, tel qu'un ulcère diabétique, un ulcère veineux, un ulcère artériel, tel qu'un ulcère artériel de la jambe, un ulcère mixte ou une escarre de décubitus, une plaie neuropathique, un ulcère de la jambe, une plaie chirurgicale, une brûlure, une déhiscence, un ulcère néoplasique, une maladie de la peau bulleuse, telle que l'épidermolyse bulleuse, et un ulcère rare.

5. Inhibiteur de la poly(ADP-ribose) polymérase (PARP) pour une utilisation de l'une des revendications 1 à 4, dans lequel :
(i) la plaie cutanée est choisie parmi une plaie d'un patient diabétique et/ou un ulcère diabétique, et/ou
(ii) le sujet est traité avec au moins un glucocorticoïde par administration systémique ou cutanée, et/ou
(iii) le sujet est traité avec au moins un facteur de croissance protéique par administration systémique ou cutanée.

6. Inhibiteur de la poly(ADP-ribose) polymérase (PARP) pour une utilisation de l'une des revendications 1 à 5, dans lequel le sujet :
(i) a subi une transplantation d'un greffon, et/ou
(ii) obtient une thérapie immunosuppressive, et/ou
(iii) est traité avec au moins un médicament immunosuppresseur, tel qu'un glucocorticoïde ou un inhibiteur de la calcineurine,
et souffre éventuellement du diabète.

7. Inhibiteur de la poly(ADP-ribose) polymérase (PARP) en combinaison avec un, deux ou trois des éléments (i) à (iii) suivants :
(i) une composition pharmaceutique comprenant un glucocorticoïde,
(ii) l'acide ascorbique ou un sel pharmaceutiquement acceptable de celui-ci,
(iii) une composition pharmaceutique comprenant un facteur de croissance protéique, pour une utilisation comme médicament.

8. Kit, ou kit d'éléments, comprenant :
(a) une composition pharmaceutique comprenant un inhibiteur de la poly(ADP-ribose) polymérase (PARP),
et un, deux ou trois des éléments (b) à (d) suivants :
(b) une composition pharmaceutique comprenant un glucocorticoïde,
(c) l'acide ascorbique ou un sel pharmaceutiquement acceptable de celui-ci,
(d) une composition pharmaceutique comprenant un facteur de croissance protéique.

9. Inhibiteur de la poly(ADP-ribose) polymérase (PARP) pour une utilisation de l'une des revendications 1 à 7, ou kit ou kit d'éléments de la revendication 8, dans lequel :
(i) l'inhibiteur (de PARP) est formulé pour une administration systémique, de préférence orale ou intraveineuse, ou dans lequel l'inhibiteur (de PARP) est formulé pour une administration locale, en particulier pour une administration topique, muqueuse ou sous-cutanée, et/ou
(ii) le glucocorticoïde est formulé pour une administration systémique, de préférence orale ou intraveineuse, ou dans lequel le glucocorticoïde est formulé pour une administration locale, en particulier pour une administration topique, muqueuse ou sous-cutanée, et/ou
(iii) l'acide ascorbique ou un sel pharmaceutiquement acceptable de celui-ci est formulé pour une administration systémique, de préférence orale ou intraveineuse, ou dans lequel l'acide ascorbique ou un sel pharmaceutiquement acceptable de celui-ci est formulé pour une administration locale, en particulier pour une administration topique, muqueuse ou sous-cutanée, et/ou
(iv) le facteur de croissance protéique est formulé pour une administration systémique, de préférence orale ou intraveineuse, ou dans lequel le facteur de croissance protéique est formulé pour une administration locale, en particulier pour une administration périlésionnelle et/ou intralésionnelle, topique, muqueuse ou sous-cutanée.

10. Inhibiteur de PARP pour une utilisation de l'une des revendications 1 à 7 ou 9, ou kit ou kit d'éléments de la revendication 8 ou 9, dans lequel l'inhibiteur de PARP inhibe PARP 1 et éventuellement PARP2, et/ou dans lequel l'inhibiteur de PARP est choisi parmi le Véliparib, le Talazoparib, l'Olaparib (AZD-2281), le Rucaparib, AZD-2461, l'Iniparib et PJ-34, ou un sel pharmaceutiquement acceptable de ceux-ci.

11. Inhibiteur de la poly(ADP-ribose) polymérase (PARP) pour une utilisation de l'une des revendications 1 à 7, ou 9 et 10, ou kit ou kit d'éléments de l'une des revendications 8 à 10, dans lequel
- le glucocorticoïde est choisi dans le groupe constitué par le cortisol, l'acétate de cortisone, la prednisone, la prednisolone, la méthylprednisolone, la chloroprednisone, le cloprednol, le difluprednate, l'acétate de fludrocortisone, la fluocinolone, la flupérolone, la fluprednisolone, le lotéprednol, le prednicarbate, le tixocortol, la triamcinolone, l'acétonide de triamcinolone, la dexaméthasone, la bétaméthasone, la béclométasone, l'acétate de désoxycorticostérone, l'alclométasone, le clobétasol, la clobétasone, la clocortolone, la désoximétasone, la diflorasone, la difluocortolone, la fluclorolone, la flumétasone, la fluocortine, la fluocortolone, le fluprednidène, la fluticasone, le furoate de fluticasone, l'halométasone, la méprednisone, la mométasone, le furoate de mométasone, la paraméthasone, le prednylidène, la rimexolone, l'ulobétasol, l'amcinonide, le budésonide, le ciclésonide, le déflazacort, le désonide, le formocortal, l'acétonide de fluclorolone, le fludroxycortide, le flunisolide, l'acétonide de fluocinolone, le fluocinonide, l'halcinonide, l'hydroxyméthylprogestérone et la médroxyprogestérone ou un sel pharmaceutiquement acceptable de ceux-ci, et/ou
- le facteur de croissance protéique est un facteur de croissance protéique humain, de préférence dans lequel le facteur de croissance protéique est choisi parmi le facteur de croissance dérivé des plaquettes (PDGF), le facteur de croissance transformant bêta (TGF-β), le facteur de croissance basique des fibroblastes (bFGF), le facteur de croissance des kératinocytes (KGF), le facteur de croissance épidermique (EGF), le facteur de croissance 1 analogue à l'insuline (IGF-1), le facteur de croissance de l'endothélium vasculaire (VEGF) et le facteur de croissance des hépatocytes (HGF).

12. Inhibiteur de la poly(ADP-ribose) polymérase (PARP) pour une utilisation de l'une des revendications 1 à 7 ou 9 à 11, dans lequel le sujet est identifié comme répondant au traitement d'une mauvaise cicatrisation de plaies cutanées en réalisant les étapes i) et/ou ii) :
i) la mesure de la prolifération de cellules fibroblastiques primaires en présence :
(1) d'un échantillon d'exsudat de plaie ou d'un échantillon de biofilm de plaie, obtenu à partir de la plaie cutanée dudit sujet, et
(2) des composés suivants : un inhibiteur de PARP et un, deux ou trois des éléments (i) à (iii) :
(i) un glucocorticoïde,
(ii) l'acide ascorbique ou un sel pharmaceutiquement acceptable de celui-ci,
(iii) un facteur de croissance protéique ;
ii) la mesure de la formation de matrice dérivée de fibroblastes par des cellules fibroblastiques primaires en présence :
(1) d'un échantillon d'exsudat de plaie, ou d'un échantillon de biofilm de plaie, obtenu à partir de la plaie cutanée dudit sujet, et
(2) des composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique.

13. Inhibiteur de la poly(ADP-ribose) polymérase (PARP) pour une utilisation de la revendication 12, dans lequel le sujet est identifié comme répondant au traitement d'une mauvaise cicatrisation de plaies cutanées dans le cas où la valeur de la prolifération de cellules fibroblastiques primaires mesurée à l'étape i) et/ou la valeur de la formation de matrice dérivée de fibroblastes par des cellules fibroblastiques primaires mesurée à l'étape ii) est/sont supérieure(s) d'au moins 20% à une valeur témoin établie en l'absence des composés de (2).

14. Procédé *in vitro* d'identification d'un sujet souffrant d'une mauvaise cicatrisation de plaies cutanées comme répondant au traitement avec un inhibiteur de la poly(ADP-ribose) polymérase (PARP) en combinaison avec un, deux ou trois des éléments (i) à (iii) :
(i) une composition pharmaceutique comprenant un glucocorticoïde,
(ii) l'acide ascorbique ou un sel pharmaceutiquement acceptable de celui-ci,
(iii) une composition pharmaceutique comprenant un facteur de croissance protéique ;
comprenant la réalisation des étapes i) et/ou ii) :
i) la mesure de la prolifération de cellules fibroblastiques primaires en présence :
(1) d'un échantillon d'exsudat de plaie, ou d'un échantillon de biofilm de plaie, obtenu à partir de la plaie cutanée dudit sujet, et
(2) des composés suivants : un inhibiteur de PARP et un, deux ou trois des éléments (i) à (iii) :
(i) un glucocorticoïde,
(ii) l'acide ascorbique ou un sel pharmaceutiquement acceptable de celui-ci,
(iii) un facteur de croissance protéique ;
ii) la mesure de la formation de matrice dérivée de fibroblastes par des cellules fibroblastiques primaires en présence :
(1) d'un échantillon d'exsudat de plaie, ou d'un échantillon de biofilm de plaie, obtenu à partir de la plaie cutanée dudit sujet, et
(2) des composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique ;
dans lequel le sujet est identifié comme répondant au traitement d'une mauvaise cicatrisation de plaies cutanées dans le cas où la valeur de la prolifération de cellules fibroblastiques primaires mesurée à l'étape i) et/ou la valeur de la formation de matrice dérivée de fibroblastes par des cellules fibroblastiques primaires mesurée à l'étape ii) est/sont supérieure(s) d'au moins 20% à une valeur témoin établie en l'absence des composés de (2).

15. Inhibiteur de la poly(ADP-ribose) polymérase (PARP) pour une utilisation de la revendication 12, ou procédé *in vitro* de la revendication 14, dans lequel en outre l'étape iiia) et/ou une, deux, trois ou quatre des étapes iiib) à iiie) suivantes sont réalisées :
iiia) la mesure de la prolifération de cellules kératinocytaires en présence:
(1) d'un échantillon d'exsudat de plaie, ou d'un échantillon de biofilm de plaie, obtenu à partir de la plaie cutanée dudit sujet, et
(2) des composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique,
iiib) la mesure de la/des quantité(s) d'un ou de plusieurs marqueur(s) M1 et d'un ou de plusieurs marqueur(s) M2 dans le surnageant de macrophages incubés avec
(1) un échantillon d'exsudat de plaie ou un échantillon de biofilm de plaie obtenu à partir de ladite plaie cutanée, et
(2) les composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique,
dans lequel les macrophages sont en co-culture avec des fibroblastes, et dans lequel le ou les plusieurs marqueur(s) M1 est/sont choisi(s) parmi CXCL10 et IL-23p19, et le ou les plusieurs marqueur(s) M2 est/sont choisi(s) parmi CCL22 et CCL18,
iiic) la mesure de la/des quantité(s) et/ou de la/des distribution(s) de fréquence d'un ou de plusieurs marqueur(s) de surface cellulaire M1 et d'un ou de plusieurs marqueur(s) de surface cellulaire M2 sur des macrophages incubés avec
(1) un échantillon d'exsudat de plaie ou un échantillon de biofilm de plaie obtenu à partir de ladite plaie cutanée, et
(2) les composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique,
dans lequel les macrophages sont en co-culture avec des fibroblastes, et
dans lequel le ou les plusieurs marqueur(s) de surface cellulaire M1 est/sont choisi(s) parmi CD38, CD64 et CD197, et dans lequel le ou les plusieurs marqueur(s) de surface cellulaire M2 est/sont choisi(s) parmi le récepteur CD200, CD206 et CD209,
iiid) la mesure du/des niveau(x) d'expression d'un ou de plusieurs ARNm de marqueur(s) M1 et d'un ou de plusieurs ARNm de marqueur(s) M2 dans des macrophages incubés avec
(1) un échantillon d'exsudat de plaie ou un échantillon de biofilm de plaie obtenu à partir de ladite plaie cutanée, et
(2) les composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique,
dans lequel les macrophages sont en co-culture avec des fibroblastes, et
dans lequel le ou les plusieurs ARNm de marqueur(s) M1 est/sont choisi(s) parmi CD38, CD64, CD197, CXCL10 et IL-23p19, et le ou les plusieurs ARNm de marqueur(s) M2 est/sont choisi(s) parmi le récepteur CD200 (CD200R), CD206, CD209, CCL22 et CCL18,
iiie) la mesure de la/des quantité(s) d'un ou de plusieurs marqueur(s) de type cytokine dans le surnageant de macrophages incubés avec
(1) un échantillon d'exsudat de plaie ou un échantillon de biofilm de plaie obtenu à partir de ladite plaie cutanée, et
(2) les composés suivants : un inhibiteur de PARP et un, deux ou trois des éléments (i) à (iii) :
(i) un glucocorticoïde
(ii) l'acide ascorbique ou un sel pharmaceutiquement acceptable de celui-ci,
(iii) un facteur de croissance protéique,
dans lequel les macrophages sont en co-culture avec des fibroblastes, et
dans lequel le ou les plusieurs marqueur(s) de type cytokine est/sont choisi(s) parmi IL-1 alpha, IL-1 bêta et TNF-alpha,
et
dans lequel le sujet est identifié comme répondant au traitement d'une mauvaise cicatrisation de plaies cutanées, dans le cas où la valeur de la prolifération de cellules fibroblastiques primaires mesurée à l'étape i) et/ou la valeur de la formation de matrice dérivée de fibroblastes par des cellules fibroblastiques primaires mesurée à l'étape ii) et/ou la valeur de la prolifération de cellules kératinocytaires à l'étape iiia) est/sont supérieure(s) d'au moins 20% à une valeur témoin établie en l'absence des composés de (2), et/ou dans le cas où une ou plusieurs des situations suivantes s'applique/s'appliquent :
- le rapport de la/des quantité(s) d'un ou de plusieurs marqueur(s) M1 sur la/les quantité(s) d'un ou de plusieurs marqueur(s) M2 obtenues dans iiib) est inférieur à une valeur témoin établie en l'absence des composés de (2),
- le rapport de la/des quantité(s) et/ou de la/des distribution(s) de fréquence d'un ou de plusieurs marqueur(s) de surface cellulaire M1 sur la/les quantité(s) et/ou la/les distribution(s) de fréquence d'un ou de plusieurs marqueur(s) de surface cellulaire M2 obtenues dans iiic) est inférieur à une valeur témoin établie en l'absence des composés de (2),
- le rapport du/des niveau(x) d'expression d'un ou de plusieurs ARNm de marqueur(s) M1 sur le/les niveau(x) d'expression d'un ou de plusieurs ARNm de marqueur(s) M2 obtenus dans iiid) est inférieur à une valeur témoin établie en l'absence des composés de (2),
- la valeur obtenue dans iiie) est inférieure à une valeur témoin établie en l'absence des composés de (2).
